# EUROPEAN PATENT SPECIFICATION

(11) **EP 3 656 847 B1**
(45) Date of publication and mention of the grant of the patent: **17.08.2022**
(21) Application number: 18837937.4
(22) Date of filing: 27.07.2018
(51) Int. Cl.: C12N 5/00, C07C 251/86

(54) **ADDITIVE COMPOSITION FOR CULTURE MEDIUM, ADDITIVE COMPOUND FOR CULTURE MEDIUM, AND METHOD FOR CULTURE OF CELLS OR TISSUE USING SAME**
ADDITIVZUSAMMENSETZUNG FÜR KULTURMEDIUM, ADDITIVVERBINDUNG FÜR KULTURMEDIUM UND VERFAHREN ZUR KULTIVIERUNG VON ZELLEN ODER GEWEBE UNTER VERWENDUNG DAVON
COMPOSITION ADDITIVE POUR MILIEU DE CULTURE, COMPOSÉ ADDITIF POUR MILIEU DE CULTURE, ET PROCÉDÉ DE CULTURE DE CELLULES OU DE TISSU L'UTILISANT

(30) Priority: 28.07.2017 JP 2017147071; 13.12.2017 JP 2017239102
(43) Date of publication of application: 27.05.2020
(73) Proprietor: Nissan Chemical Corporation, Tokyo 103-6119 (JP)
(72) Inventor: NISHINO, Taito, Shiraoka-shi Saitama 349-0294 (JP); AIHARA, Ayako, Shiraoka-shi Saitama 349-0294 (JP); OTSUKA, Keiichiro, Shiraoka-shi Saitama 349-0294 (JP); SARUHASHI, Koichiro, Funabashi-shi Chiba 274-0052 (JP); MIKASHIMA, Takumi, Tokyo 101-0054 (JP); KAMAURA, Masahiro, Funabashi-shi Chiba 274-8507 (JP)
(74) Representative: J A Kemp LLP
(86) International application number: PCT/JP2018/028205
(87) International publication number: WO 2019/022222

(56) References cited:
- WO-A1-95/22599
- WO-A1-2014/017513
- WO-A1-2014/075807
- WO-A1-2019/235569
- HWANG SUNG HEE ET AL: "Synthesis and biological evaluation of sorafenib- and regorafenib-like sEH inhibitors", BIOORGANIC & MEDICINAL CHEMISTRY LETTERS, PERGAMON, AMSTERDAM, NL, vol. 23, no. 13, 15 May 2013 (2013-05-15), pages 3732-3737, XP028564919, ISSN: 0960-894X, DOI: 10.1016/J.BMCL.2013.05.011
- JASON G. KETTLE ET AL: "Inhibitors of JAK-family kinases: an update on the patent literature 2013-2015, part 2", EXPERT OPINION ON THERAPEUTIC PATENTS, vol. 27, no. 2, 4 November 2016 (2016-11-04), pages 145-161, XP055564155, ISSN: 1354-3776, DOI: 10.1080/13543776.2017.1252754
- NOA FURTH ET AL: "The LATS1 and LATS2 tumor suppressors: beyond the Hippo pathway", CELL DEATH AND DIFFERENTIATION., vol. 24, no. 9, 23 June 2017 (2017-06-23), pages 1488-1501, XP055695078, GB ISSN: 1350-9047, DOI: 10.1038/cdd.2017.99

## Description

### [Technical Field]

The present invention relates to a medium additive composition and the like. In more detail, it relates to a medium additive composition for promoting cell proliferation and the like.

### [Background Art]

In recent years, experiments using cells have been extremely frequently performed in many fields including the life science field for the purpose of elucidating the action mechanism of life phenomena and establishing treatment methods for diseases and the like. For example, as one example in the field of drug discovery, there is a method of having a candidate compound act on cancer cells to be a treatment target and screening for a compound capable of suppressing proliferation of the cancer cells. In such screening, tens of thousands of candidate compounds may be screened, and in such embodiment, it is necessary to prepare a large amount of homogeneous cells. However, cells of higher organisms such as human and the like require a period of about one day even for cells that divide relatively quickly. In addition, some cancer cells and stem cells require more than a few months for a single cell division. This is the factor preventing rapid cell procurement. From such background, construction of means capable of promoting proliferation of slow-dividing cells has been demanded. For example, there are reports that a thiol compound having a particular structure promotes proliferation of hematopoietic progenitor cells, and that polyprenyl compounds promote proliferation of hepatocytes and the like (patent documents 1, 2).

On the other hand, in the field of drug discovery screening, three-dimensional culture of cells is attracting attention in recent years. Three-dimensional culture is a cell culture technique that is between in vitro and in vivo. In three-dimensional culture, cells can form a steric structure such as a sphere (also referred to as spheroid) or the like, and therefore, an assay that is closer to a living body compared with general two-dimensional culture can be available. Hence, three-dimensional culture may be able to identify a compound for treating diseases that could not be identified by drug discovery screening using two-dimensional culture (non-patent document 1).

Non-patent document 2 describes the synthesis and biological evaluation of sorafenib- and regorafenib-like she inhibitors. Non-patent document 3 describes inhibitors of JAK-family kinases from 2013-2015 patent literature.

Patent document 3 describes a kinase inhibitor that can inhibit multiple kinases including LATS that is a major kinase involved in the Hippo signal transduction pathway.

### [Document List]

### [Patent documents]

patent document 1: National Publication of International Patent Application No. H11-504000
patent document 2: WO 2008/155920
patent document 3: WO 2019/235569 A1

### [non-patent document]

non-patent document 1: Susan Breslin, "Drug Discovery Today", 2013, vol. 18, No. 5, p.240-249
non-patent document 2: HWANG SUNG HEE ET AL: "Synthesis and biological evaluation of sorafenib- and regorafenib-like she inhibitors", BIOORGANIC & MEDICINAL CHEMISTRY LETTERS, vol.23, no. 13, 15 May 2021, pages 3732-3737
non-patent document 3: JASON G. KETTLE ET AL, "Inhibitors of JAK-family kinases: an update on the patent literature 2013-2015, part 2", EXPERT OPINION ON THERAPEUTIC PATENTS, vol. 27, no. 2, 4 November 2016, pages 145-161

### [SUMMARY OF THE INVENTION]

### [Problems to be Solved by the Invention]

The present invention aims to provide novel compounds capable of promoting cell proliferation in cell culture (particularly three-dimensional cell culture).

### [Means of Solving the Problems]

The present inventors have conducted intensive studies of the aforementioned problems and found that the compounds newly synthesized at this time can promote proliferation of various cells under three-dimensional culture extremely well. Based on such finding, they have conducted further studies and completed the present invention. Therefore, the present invention provides the following.

[1] A medium additive composition comprising a compound represented by the following formula (I), or a salt thereof:

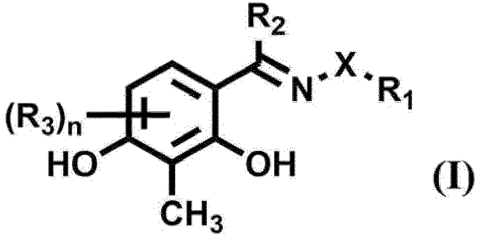

   {wherein, X is a single bond, -CH₂COO-, -CONH-, or -NHCO-, R₁ is an alkyl group having 1 - 10 carbon atoms, an aryl group, or -Y-W-Z-Ar wherein Y and Z are each a single bond or an alkylene group having 1 - 6 carbon atoms, W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms, R₃ is a hydroxyl group, and n is 0, 1 or 2}, wherein when Ar has substituent(s) the substituent(s) are selected from halogeno groups and hydroxyl groups.
[2] The invention also provides the composition of [1], wherein X is -NHCO-.
[3] The invention also provides the composition of [1] or [2], wherein n is 0.
[4] The invention also provides the composition of [2], wherein R₁ is -Y-W-Z-Ar, Y is an alkylene group having 1 - 6 carbon atoms, W is N(R₄), Z is a single bond, and n=0, preferably wherein the aryl group is a phenyl group.
[5] The invention also provides the composition of any of [1] to [3], wherein R₁ is -Y-W-Z-Ar, Y is a single bond, W is N(R₄) and R₄ is a hydrogen atom, preferably wherein the aryl group of Ar is a phenyl group, and/or Z is a methylene group.
[6] The invention also provides the composition of any of [1] to [3], wherein R₁ is -Y-W-Z-Ar, Y is a single bond, W is an oxygen atom, and Z is a methylene group, preferably wherein the aryl group of Ar is a phenyl group.
[7] The invention also provides the composition of any of [1] to [4], wherein the compound is a compound selected from the group consisting of the following, or a salt thereof:

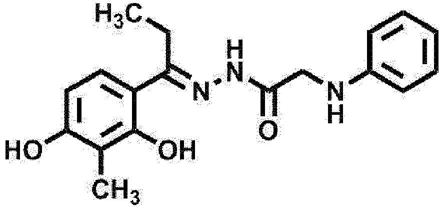

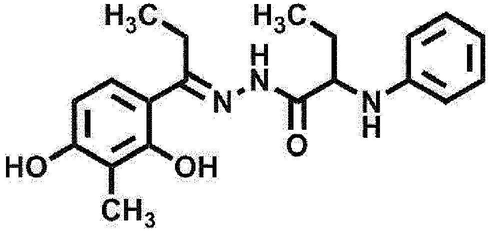

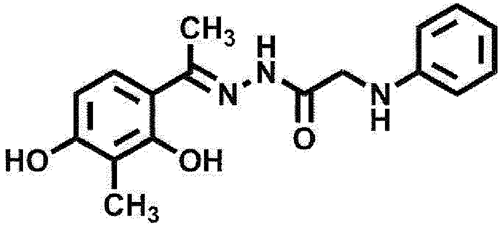

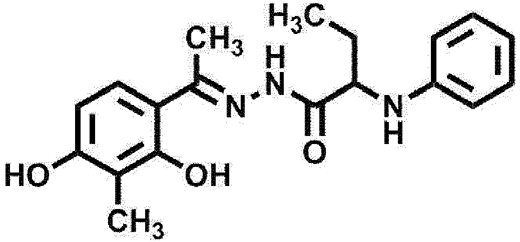

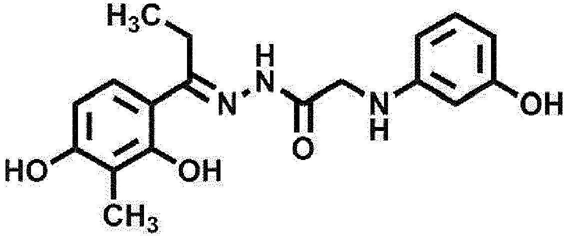

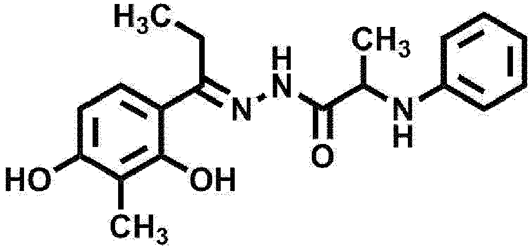

   and, 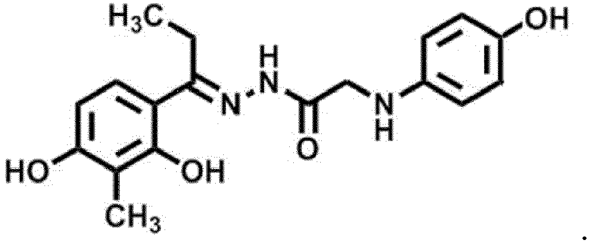
[8] The invention also provides the composition of any of [1] - [3] and [5], wherein the compound is a compound selected from the group consisting of the following, or a salt thereof:

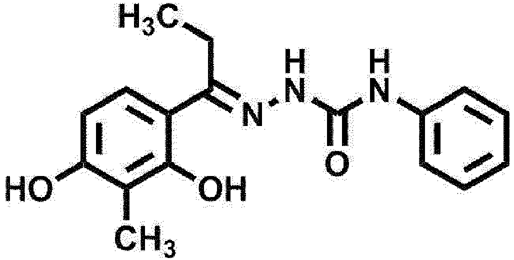

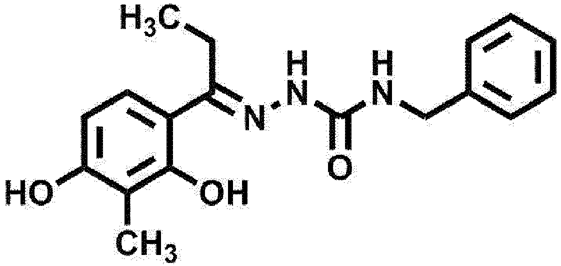

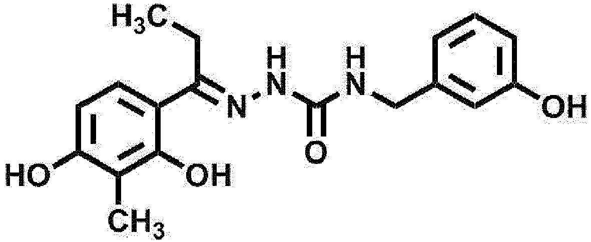

   and, 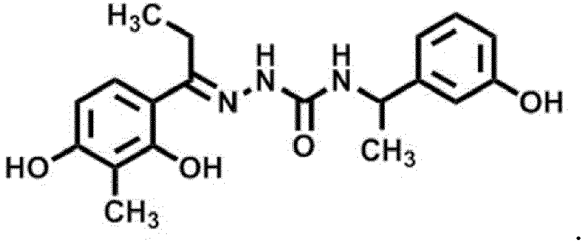
[9] The invention also provides the composition of any of [1] - [3] and [6], wherein the compound is a compound represented by: 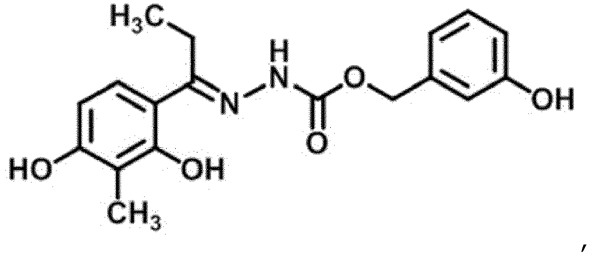 or a salt thereof.
[10] The invention also provides a medium additive composition comprising a compound selected from the following group of compounds, or a salt thereof: 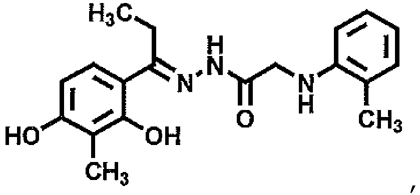 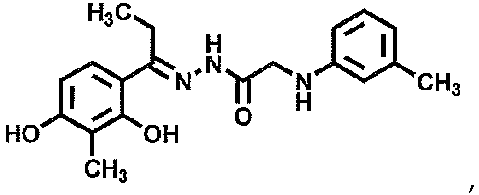 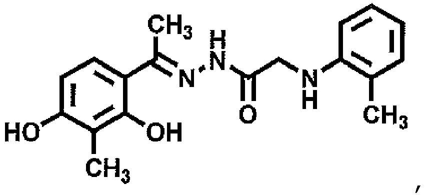 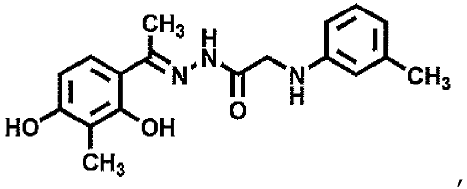 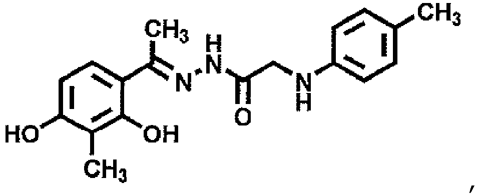 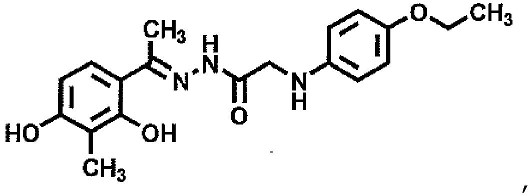 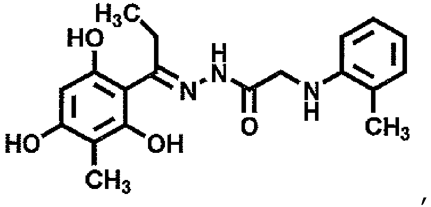 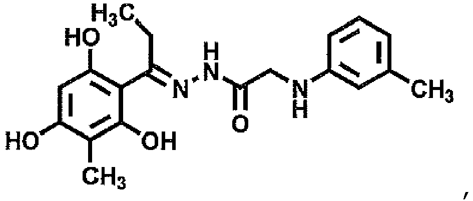 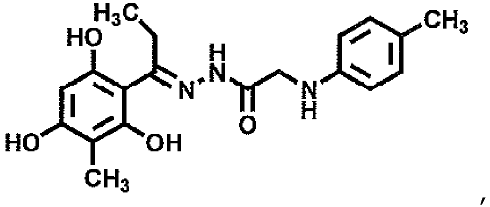 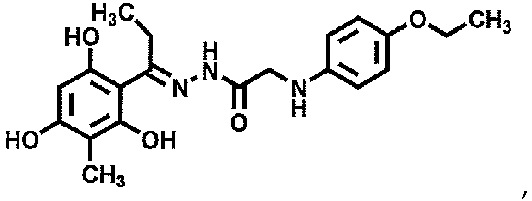 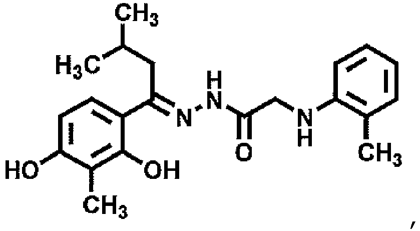 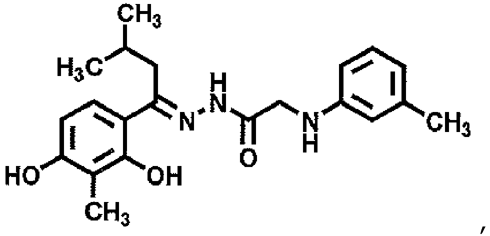 and, 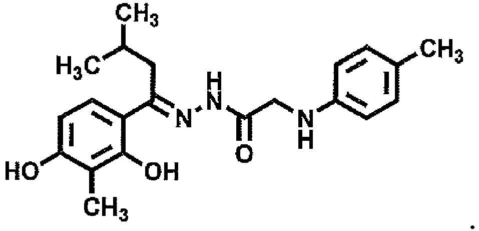
[11] The invention also provides the composition of any of [1] to [10], wherein the composition is for promoting cell proliferation, preferably wherein the cell is selected from the group consisting of a normal cell line, a cancer cell line and a stem cell.
[12] The invention also provides the composition of any of [1] to [10], wherein the composition is for promoting sphere formation, organoid formation, or Cyst formation.
   The sphere may be composed of cancer cell lines, human induced pluripotent stem cells (iPS cells) or human mesenchymal stem cells (MSCs).
[13] The invention also provides a medium comprising the medium additive composition of any of [1] to [10].
[14] The invention also provides a method for promoting cell proliferation comprising adding the medium additive composition of any of [1] to [10] to a medium, preferably wherein the cell is selected from the group consisting of a normal cell line, a cancer cell line and a stem cell.
[15] The invention also provides a method for promoting sphere formation, organoid formation or Cyst formation, comprising adding the medium additive composition of any of [1] to [10] to a medium.
   The sphere may be composed of cancer cell lines, human induced pluripotent stem cells (iPS cells) or human mesenchymal stem cells (MSCs).
[16] The invention also provides a compound represented by the following formula (I), or a salt thereof:

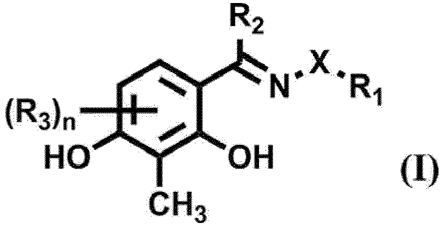

   {wherein, X, R₁, R₂, R₃, and n are as defined in any one of [1] to [10] (provided that when X is -NHCO-, R₂ is an ethyl group, and n is 0, then R₁ is not -CH₂-NH-C₆H₅) }.
[17] The invention also provides a compound selected from the following group of compounds, or a salt thereof: 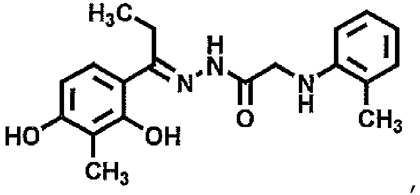 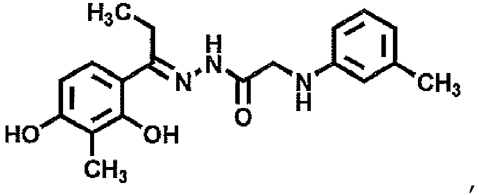 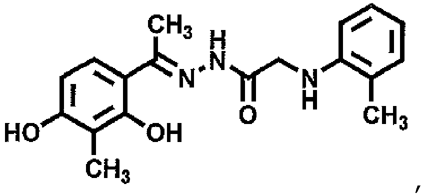 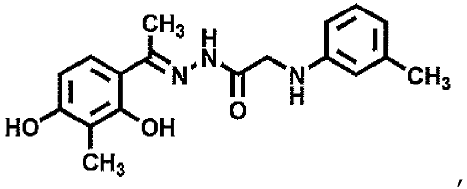 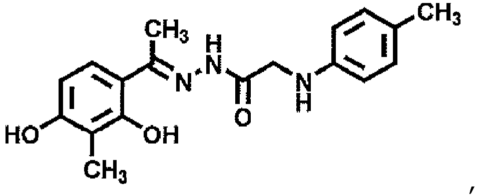 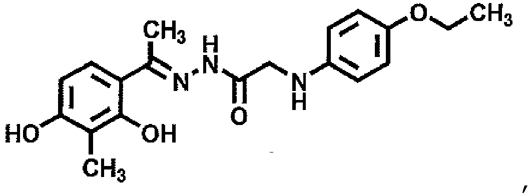 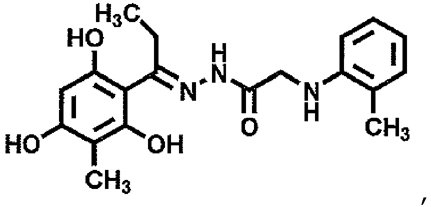 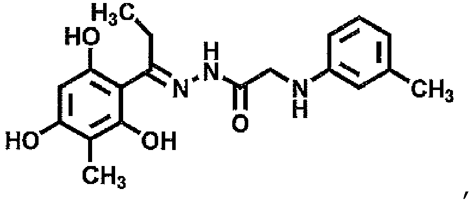 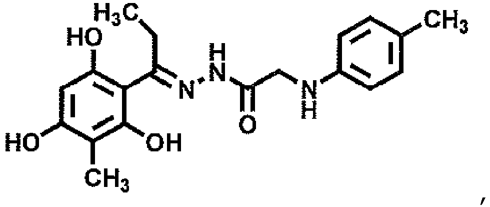 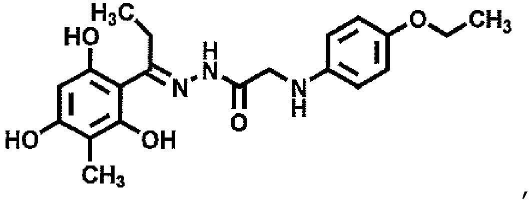 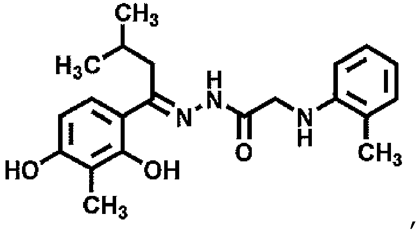 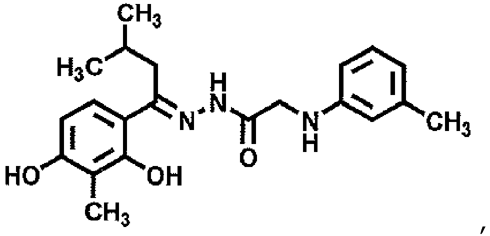 and,

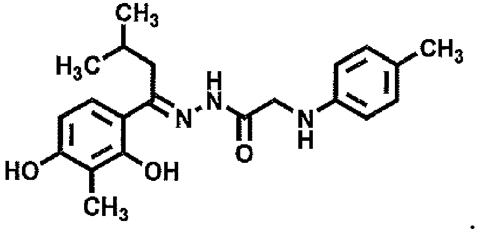

### [Effect of the Invention]

The compound represented by the formula (I) or a salt thereof has a cell proliferation promoting activity under three-dimensional culture. Therefore, it can remarkably promote cell proliferation, sphere formation, organoid formation, and/or Cyst formation.

### [Brief Description of the Drawings]

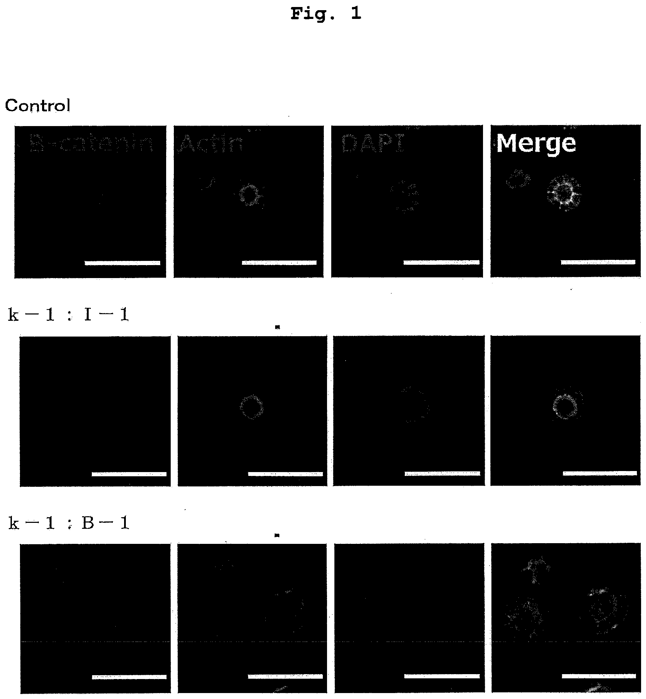

Fig. 1 shows diagram in which Cyst formation of MDCK cells cultured in a medium added with the composition of the present invention is observed using a confocal fluorescence microscope.

### [Description of Embodiments]

The terms used in the present specification are defined in the following.

In the present specification, n- means normal, i- means iso, sec- means secondary and tert- means tertiary. In addition, in the present specification, o- means ortho, m-means meta and p- means para.

The "halogen atom" is a fluorine atom, a chlorine atom, a bromine atom, or an iodine atom. The "halogeno group" is fluoro, chloro, bromo, or iodo.

The "alkyl group" and "alkyl group having 1 - 10 carbon atoms" means a straight chain or branched alkyl group, and specifically, methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl, n-heptyl, n-octyl, n-nonyl, n-decyl and the like group can be mentioned. The "an alkyl group having 1 - 6 carbon atoms" means a straight chain or branched alkyl group, and specifically, groups such as methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned.

The "aryl group" is, for example, monocyclic, bicyclic, tricyclic or tetracyclic carbon cyclic group in which at least one ring is aromatic and each ring has 5 to 8 ring atoms. Specifically, phenyl, indenyl, naphthyl, fluorenyl and the like can be mentioned. Particularly, the aryl group may be a ring having a carbon number of 6 to 10 such as phenyl, indenyl or naphthyl.

The "alkylene group" and "alkylene group having 1 - 6 carbon atoms" mean straight chain or branched alkylene groups. Specifically, groups such as methylene, ethylene, propylene, butylene, pentylene, hexylene and the like can be mentioned.

The "alkyl group", "aryl group" and "alkylene group" may have a substituent. Examples of such substituent include the following. For "alkyl group", the following (1) to (40) can be mentioned, and the following (1) to (41) can be mentioned for "aryl group" and "alkylene group".

(1) halogeno group,
(2) hydroxyl group,
(3) cyano group,
(4) nitro group,
(5) carboxyl group,
(6) alkenyl group (C₂₋₁₀ alkenyl group; e.g., vinyl, allyl, propenyl, butenyl, pentenyl, hexenyl, heptenyl, butadienyl, hexatrienyl, and each isomer thereof),
(7) alkynyl group (C₂₋₁₀ alkynyl group; e.g., ethynyl, propynyl, butynyl, pentynyl, hexynyl, and each isomer thereof),
(8) halogenoalkyl group (e.g., monofluoromethyl, difluoromethyl, trifluoromethyl, monofluoroethyl, difluoroethyl, trifluoroethyl, chloromethyl, chloroethyl, dichloroethyl, and each isomer thereof),
(9) cyclic alkyl group (optionally having hetero atom in the ring) (e.g., cyclopropyl, cyclobutyl, cyclopentyl, cyclohexyl, tetrahydrofuranyl, tetrahydropyranyl, aziridinyl, azetidinyl, pyrrolidinyl, piperidinyl, morpholinyl),
(10) aryl group (e.g., phenyl, naphthyl),
(11) heteroaryl group (e.g., pyridyl, pyridazinyl, pyrimidinyl, pyrazinyl, furyl, thiophenyl, pyrrolyl, pyrazolyl, imidazolyl, triazolyl (e.g., 1,2,3-triazolyl, 1,2,4-triazolyl), tetrazolyl, oxazolyl, isoxazolyl, thiazolyl, isothiazolyl, oxadiazolyl (e.g., 1,2,3-oxadiazolyl, 1,2,4-oxadiazolyl, 1,3,4-oxadiazolyl), thiadiazolyl (e.g., 1,2,3-thiadiazolyl, 1,2,4-thiadiazolyl, 1,3,4-thiadiazolyl), benzofuryl, benzothiophenyl, indolyl, isoindolyl, benzoxazolyl, benzothiazolyl, benzimidazolyl, indazolyl, benzisoxazolyl, benzisothiazolyl, benzooxadiazolyl, benzothiadiazolyl, purinyl, quinolinyl, isoquinolinyl, cinnolinyl, phthalazinyl, quinazolinyl, quinoxalinyl, pteridinyl, imidazooxazolyl, imidazothiazolyl, imidazoimidazolyl),
(12) alkoxy group (e.g., methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy),
(13) alkylthio group (e.g., methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio),
(14) alkoxy group (same as the above-mentioned (12)) substituted by aryl group (same as the above-mentioned (10)),
(15) alkylthio group (same as the above-mentioned (13)) substituted by aryl group (same as the above-mentioned (10)),
(16) alkoxy group (same as the above-mentioned (12)) substituted by heteroaryl group (same as the above-mentioned (11)),
(17) alkylthio group (same as the above-mentioned (13)) substituted by heteroaryl group (same as the above-mentioned (11)),
(18) cyclic alkyl(optionally having hetero atom in the ring)oxy group (e.g., cyclopropyloxy, cyclobutyloxy, cyclopentyloxy, cyclohexyloxy, tetrahydrofuranyloxy, tetrahydropyranyloxy, aziridinyloxy, azetidinyloxy, pyrrolidinyloxy, piperidinyloxy, morpholinyloxy),
(19) aryloxy group (e.g., group with aryl group (same as the above-mentioned (10)) bonded to oxygen atom),
(20) heteroaryloxy group (e.g., group with heteroaryl group (same as the above-mentioned (11)) bonded to oxygen atom),
(21) halogenoalkoxy group (e.g., group with halogenoalkyl group (same as the above-mentioned (8)) bonded to oxygen atom),
(22) halogenoalkylthio group (e.g., group with halogenoalkyl group (same as the above-mentioned (8)) bonded to sulfur atom),
(23) alkoxy group (same as the above-mentioned (12)) substituted by hydroxyl group,
(24) alkoxy group (same as the above-mentioned (12)) substituted by alkoxy group (same as the above-mentioned (12)),
(25) amino group,
(26) amino group mono- or di-substituted by alkyl group,

As used herein, the "alkyl group" is, for example, C₁₋₆ alkyl group. Specifically, methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned.
(27) carbamoyl group,
(28) carbamoyl group mono- or di-substituted by alkyl group (same as "alkyl group" in the above-mentioned (26)) (e.g., methylcarbamoyl, ethylcarbamoyl, dimethylcarbamoyl, diethylcarbamoyl, ethylmethylcarbamoyl),
(29) sulfamoyl group,
(30) sulfamoyl group mono- or di-substituted by alkyl group (same as "alkyl group" in the above-mentioned (26)) (e.g., methylsulfamoyl, ethylsulfamoyl, dimethylsulfamoyl, diethylsulfamoyl, ethylmethylsulfamoyl),
(31) alkanoyl group (e.g., carbonyl group with hydrogen atom or alkyl group (same as "alkyl group" in the above-mentioned (26)) bonded to carbon atom),
(32) aroyl group (e.g., carbonyl group with aryl group (same as the above-mentioned (10)) bonded to carbon atom),
(33) alkylsulfonylamino group (e.g., sulfonylamino group substituted by alkyl group (same as "alkyl group" in the above-mentioned (26)))
(34) arylsulfonylamino group (e.g., sulfonylamino group substituted by aryl group (same as the above-mentioned (10))),
(35) heteroarylsulfonylamino group (e.g., sulfonylamino group substituted by heteroaryl group (same as the above-mentioned (11))),
(36) acylamino group (e.g., amino group substituted by acyl group),

As used herein, the "acyl group" is an acyl group having a C₁₋₆ alkyl group, or a C₆₋₁₀ aryl group. As used herein, the "C₁₋₆ alkyl group" is the above-mentioned "alkyl group" having 1 - 6 carbon number, and "C₆₋₁₀ aryl group" is the above-mentioned "aryl group" having 6 - 10 carbon number. Specific examples of the acyl group include acetyl group, propionyl group, butyroyl group, isobutyroyl group, valeroyl group, isovaleroyl group, pivaloyl group, hexanoyl group, acryloyl group, methacryloyl group, crotonoyl group, isocrotonoyl group, benzoyl group, naphthoyl group and the like,
(37) alkoxycarbonylamino group (e.g., carbonylamino group substituted by alkoxy group (same as the above-mentioned (12))),
(38) alkylsulfonyl group (e.g., sulfonyl group substituted by alkyl group (same as "alkyl group" in the above-mentioned (26))),
(39) alkylsulfinyl group (e.g., sulfinyl group substituted by alkyl group (same as "alkyl group" in the above-mentioned (26))),
(40) alkoxycarbonyl group (e.g., methoxycarbonyl group, ethoxycarbonyl group),
(41) alkyl group (C₁₋₁₀ alkyl group; e.g., methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl etc.) and the like.

When two or more substituents are present, they may be the same or different.

The compound of the formula (I) may be in the form of a salt. Examples of the salt of the aforementioned compound represented by the formula (I) include salts with inorganic acids such as hydrochloric acid and hydrobromic acid, and salts with organic acids such as acetic acid, propionic acid, tartaric acid, fumaric acid, maleic acid, malic acid, oxalic acid, succinic acid, citric acid and benzoic acid.

The compound represented by the formula (I) may contain geometric isomers of an E-form having an E-steric configuration and Z-form having a Z-steric configuration depending on the type of the substituent. The present invention includes E-form, Z-form or a mixture containing E-form and Z-form in any ratio.

### [Synthesis method 1] Synthesis of compound represented by the formula (I)

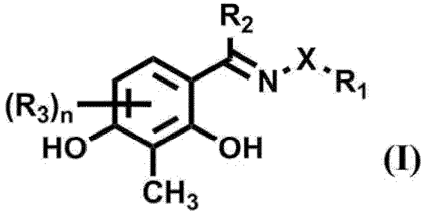

{wherein, X is a single bond, -CH₂COO-, -CONH-, or -NHCO-, R₁ is an alkyl group having 1 - 10 carbon atoms, an aryl group, or -Y-W-Z-Ar wherein Y and Z are each a single bond or an alkylene group having 1 - 6 carbon atoms, W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms, R₃ is a hydroxyl group, and n is 0, 1 or 2}, wherein when Ar has substituent(s) the substituent(s) are selected from halogeno groups and hydroxyl groups.

The compound represented by the above-mentioned formula (I) can be synthesized by reacting, as shown in the following reaction scheme, ketone compound (k) with H₂N-X-R₁ wherein X and R₁ are as defined above, for example, hydrazide compound and the like. It is preferable to use 1 equivalent each of the aforementioned starting materials, and perform the reaction in a solvent such as toluene, 1,4-dioxane, N,N-dimethylformamide, dimethyl sulfoxide and the like at not less than 100°C for 1 hr to 3 days.

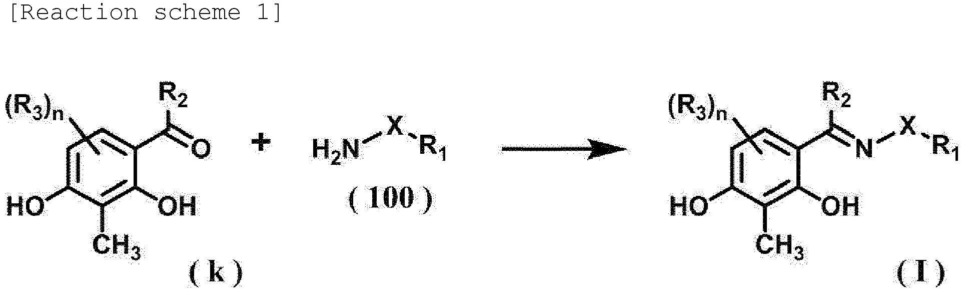

wherein R₁, R₂, R₃, X and n are as defined above.

Some of the above-mentioned k and 100 are commercially available, and others can also be synthesized according to known synthesis methods.

### [Synthesis method 2] Synthesis of compound composed of combination of ketone compound and hydrazide compound

Among the compounds represented by the formula (I), a compound composed of a combination of a ketone compound and a hydrazide compound can be synthesized by a method analogous to the aforementioned synthesis method 1 by using a hydrazide compound in which X of H₂N-X-R₁ is -NHCO-, R₁ is -Y-W-Z-Ar, W is N(R₄), Y is an alkylene group having 1 - 6 carbon atoms, and R₂, R₃, R₄, Z, Ar and n are as defined above.

Some of the above-mentioned hydrazide compounds are commercially available, and others can also be synthesized according to known synthesis methods.

### [Synthesis method 3] Synthesis of compound composed of combination of ketone compound and amine compound

Among the compounds represented by the formula (I), a compound composed of a combination of a ketone compound and an amine compound can be synthesized using the aforementioned ketone compound (k) [for example, 2',4'-dihydroxy-3'-methylpropiophenone (k-1) etc.] and a desired primary amine in which X of H₂N-X-R₁ is a single bond or -CH₂COO-, and R₁ is as defined above [for example, n-octylamine (A-3) etc.] or a salt thereof [for example, glycine ethyl ester hydrochloride (A-1) etc.]. It is preferable to use 1 equivalent each of the aforementioned starting materials, and perform the reaction in a solvent such as toluene, 1,4-dioxane, N,N-dimethylformamide, dimethyl sulfoxide and the like at not less than 100°C for 1 hr to 24 hr. As a salt with amine, hydrochloride, p-toluenesulfonate, trifluoroacetate and the like can be used.

Some of the above-mentioned primary amines are commercially available, and others can also be synthesized according to known synthesis methods.

### [Synthesis method 4] Synthesis of urea compound

Among the compounds represented by the formula (I), a urea compound wherein X is -CONH-, and R₁, R₂, R₃ and n are as defined above can be synthesized by dissolving the aforementioned ketone compound (k) [for example, 2',4'-dihydroxy-3'-methylpropiophenone (k-1) etc.] in a methanol solution of ammonia according to a known synthesis method, agitating the mixture while injecting an ammonia gas to synthesis an imine compound (k-1') and thereafter reacting same with the corresponding isocyanate [for example, phenylisocyanate (A-5)].

Some of the above-mentioned isocyanates are commercially available, and others can also be synthesized according to known synthesis methods.

In the Synthesis method 1 to Synthesis method 4, the reaction mixture after completion of the reaction is precipitated by adding distilled water, or when no precipitation occurs, a general post-treatment such as concentration after extraction with an organic solvent is performed to obtain the target compound to be used in the present invention. When purification is necessary, the compound can be separated and purified by any purification method such as recrystallization, column chromatography, thin-layer chromatography, liquid chromatography and the like.

The three-dimensional cell culture (3D cell culture) in the present specification means, for example, culturing cells in a three-dimensional environment using an embedded culture method, a microcarrier culture method, a sphere culture method and the like. Embedded culture is a method of cultivating cells by embedding and fixing the cells in a solid or semisolid gel substrate such as Matrigel (registered trade mark), Geltrex(registered trade mark), agar, methylcellulose, collagen, gelatin, fibrin, agarose, alginates and the like. Microcarrier culture method is a method of cultivating cells in a suspended state by proliferating cells in a single layer on the surface of a fine particle slightly heavier than water (hereinafter to be also referred to as a microcarrier), and stirring the fine particles in a culture container such as a flask and the like. Sphere culture is a culture method including forming an aggregate composed of several dozen - several hundred object cells (hereinafter to be also referred to as a sphere or spheroid), and culturing the aggregates with standing or shaking in a medium. As the three-dimensional cell culture (3D cell culture) in the present invention, a method of culturing cells in a three-dimensional state closer to that in the living body can also be used by dispersing polysaccharides such as hyaluronic acid, deacylated gellan gum, xanthan gum and the like or a derivative of these in a medium to form an atypical three-dimensional network, and maintaining adherent cells suspended in the medium by using the network as a scaffold. At this time, the cells in the three-dimensional cell culture are trapped in the three-dimensional network and do not precipitate. Therefore, the cells can be cultured without a shaking or rotation operation or the like. The three-dimensional cell culture can be performed by a method known per se (e.g., WO 2014/017513).

### 1. Compound

The compound used for the composition, medium, method of the present invention is the formula (I):

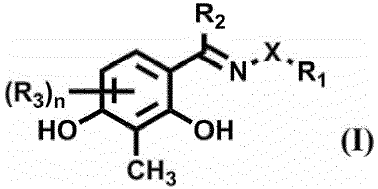

{wherein, X is a single bond, -CH₂COO-, -CONH-, or -NHCO-, R₁ is an alkyl group having 1 - 10 carbon atoms, an aryl group, or -Y-W-Z-Ar wherein Y and Z are each a single bond or an alkylene group having 1 - 6 carbon atoms, W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms, R₃ is a hydroxyl group, and n is 0, 1 or 2}, wherein when Ar has substituent(s) the substituent(s) are selected from halogeno groups and hydroxyl groups.

Alternatively, the compound used for the composition, medium, method of the present invention is selected from the following group of compounds, or a salt thereof: 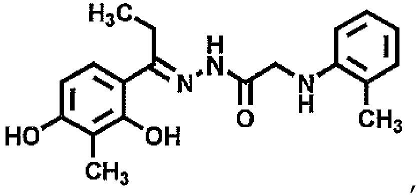 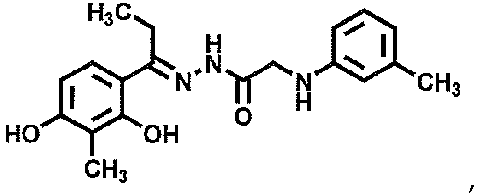 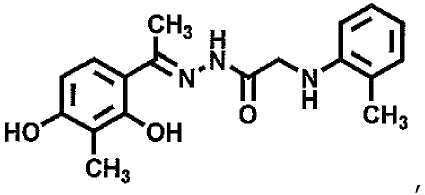 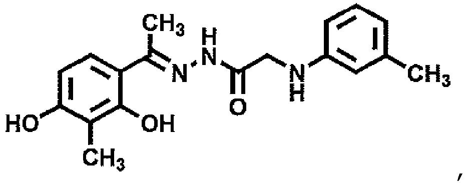 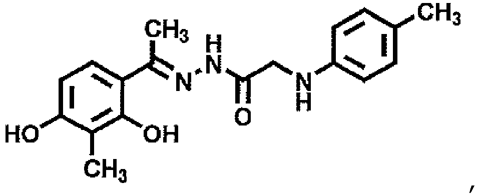 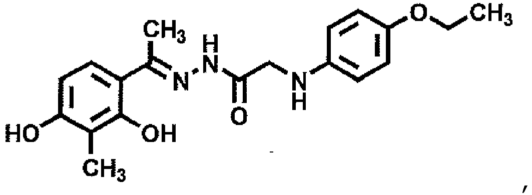 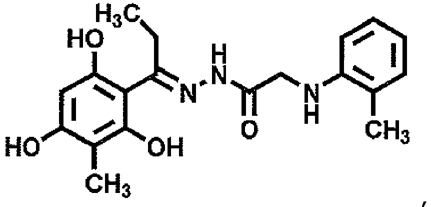 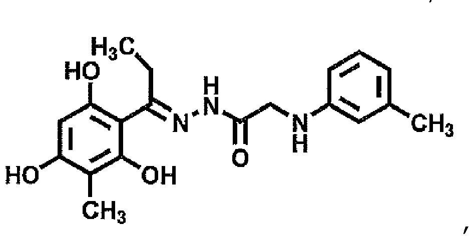 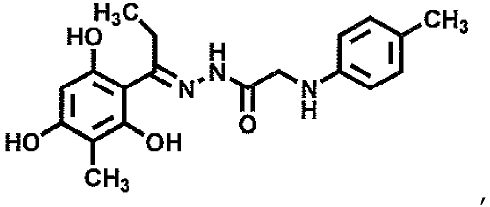 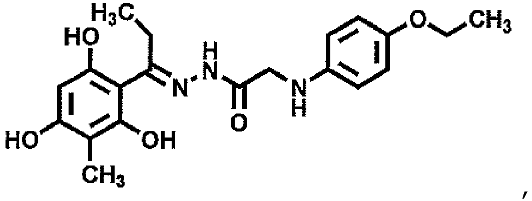 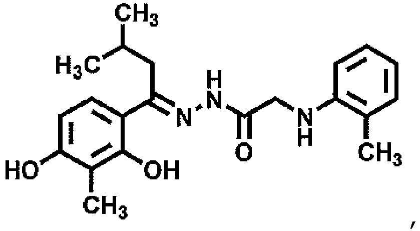 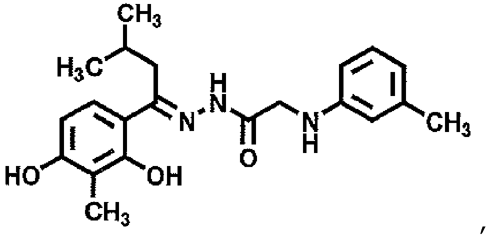 and,

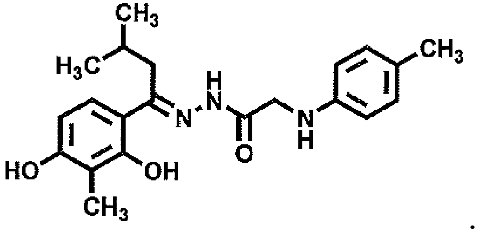

Hereinafter the compound and a salt thereof to be used for the composition, medium and method of the present invention are sometimes to be also generically referred to as "compound to be used in the present invention".

In one embodiment, when X in the aforementioned formula (I) is a single bond, R₁ is an alkyl group having 1 - 10 carbon atoms (particularly preferably, octyl group), R₂ is an alkyl group having 1 - 6 carbon atoms (particularly preferably, an ethyl group), and n=0.

In addition, when X is -CH₂COO-, R₁ is an alkyl group having 1 - 10 carbon atoms , R₂ is an alkyl group having 1 - 6 carbon atoms (particularly preferably, an ethyl group), and n=0.

In addition, when X is -CONH-, R₁ is an aryl group (particularly preferably, a phenyl group), R₂ is an alkyl group having 1 - 6 carbon atoms (particularly preferably, an ethyl group), and n=0.

In addition, when X is -NHCO-, and R₁ is -Y-W-Z-Ar wherein Y is a single bond, then W is N(R₄) (more preferably, N(R₄), R₄ is a hydrogen atom or an alkyl group having 1 - 6 carbon atoms, particularly preferably, N(R₄), R₄ is a hydrogen atom), or an oxygen atom, Z is a single bond or an alkylene group (particularly preferably, a single bond or a methylene group), Ar is an aryl group optionally having a halogen atom, or a hydroxyl group (more preferably, an aryl group having a hydroxyl group or an aryl group not having a substituent, particularly preferably, a phenyl group having a hydroxyl group or a phenyl group not having a hydroxyl group), R₂ is an alkyl group having 1 - 6 carbon atoms (particularly preferably, an ethyl group), and n=0.

In addition, when X is -NHCO-, R₁ is -Y-W-Z-Ar, and Y is an alkylene group having 1 - 6 carbon atoms (particularly preferably a methylene group), then W is N(R₄) (more preferably, N(R₄) wherein R₄ is a hydrogen atom or an alkyl group having 1 - 6 carbon atoms, particularly preferably, N(R₄) wherein R₄ is a hydrogen atom), Z is a single bond, Ar is an aryl group optionally having substituent(s) selected from halogeno groups and hydroxyl groups (more preferably, an aryl group having a halogeno group, or a hydroxyl group, particularly preferably, a phenyl group having a halogeno group, or a hydroxyl group), R₂ is an alkyl group having 1 - 6 carbon atoms (particularly preferably, a methyl group, an ethyl group, or an isopropyl group), and n=0.

Also, the present invention provides the following novel compound or a salt thereof (hereinafter sometimes referred to as "the compound of the present invention").

The compound of the present invention is a compound represented by the following formula (I):

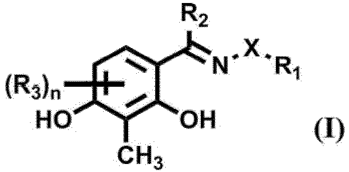

{wherein, X is a single bond, -CH₂COO-, -CONH-, or -NHCO-, R₁ is an alkyl group having 1 - 10 carbon atoms, an aryl group, or -Y-W-Z-Ar wherein Y and Z are each a single bond or an alkylene group having 1 - 6 carbon atoms, W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms, R₃ is a hydroxyl group, and n is 0, 1 or 2, wherein when Ar has substituent(s) the substituent(s) are selected from halogeno groups and hydroxyl groups (provided that when X is -NHCO-, R₂ is an ethyl group, and n is 0, then R₁ is not -CH₂-NH-C₆H₅)}, or a salt thereof.

In one embodiment of the compound of the present invention, when X in the aforementioned formula (I) is a single bond, then R₁ is an alkyl group having 1 - 10 carbon atoms (particularly preferably, an octyl group), R₂ is an alkyl group having 1 - 6 carbon atoms (particularly preferably, an ethyl group), and n=0.

In addition, when X is -CH₂COO-, R₁ is an alkyl group having 1 - 10 carbon atoms , R₂ is an alkyl group having 1 - 6 carbon atoms (particularly preferably, an ethyl group), and n=0.

In addition, when X is -CONH-, then R₁ is an aryl group (particularly preferably, a phenyl group), R₂ is an alkyl group having 1 - 6 carbon atoms (particularly preferably, an ethyl group), and n=0.

In addition, when X is -NHCO-, and R₁ is -Y-W-Z-Ar wherein Y is a single bond, then W is N(R₄) (more preferably, N(R₄) wherein R₄ is a hydrogen atom or an alkyl group having 1 - 6 carbon atoms, particularly preferably, N(R₄), R₄ is a hydrogen atom), or an oxygen atom, Z is a single bond or an alkylene group (particularly preferably, a single bond or a methylene group), Ar is an aryl group optionally having substituent(s) selected from halogeno groups and hydroxyl groups (more preferably, an aryl group having a hydroxyl group or an aryl group not having a substituent, particularly preferably, a phenyl group having a hydroxyl group or a phenyl group not having a substituent), R₂ is an alkyl group having 1 - 6 carbon atoms (particularly preferably, an ethyl group), and n=0.

In addition, when X is -NHCO-, R₁ is -Y-W-Z-Ar, and Y is an alkylene group having 1 - 6 carbon atoms (particularly preferably a methylene group), then W is N(R₄) (more preferably, N(R₄) wherein R₄ is a hydrogen atom or an alkyl group having 1 - 6 carbon atoms, particularly preferably, N(R₄) wherein R₄ is a hydrogen atom), Z is a single bond, Ar is an aryl group optionally having substituent(s) selected from halogeno groups and hydroxyl groups (particularly preferably, a phenyl group having a halogeno group, or a hydroxyl group), R₂ is an alkyl group having 1 - 6 carbon atoms (particularly preferably, a methyl group, an ethyl group, or an isopropyl group), and n=0.

In one preferable embodiment, the compound to be used in the present invention is as follows.

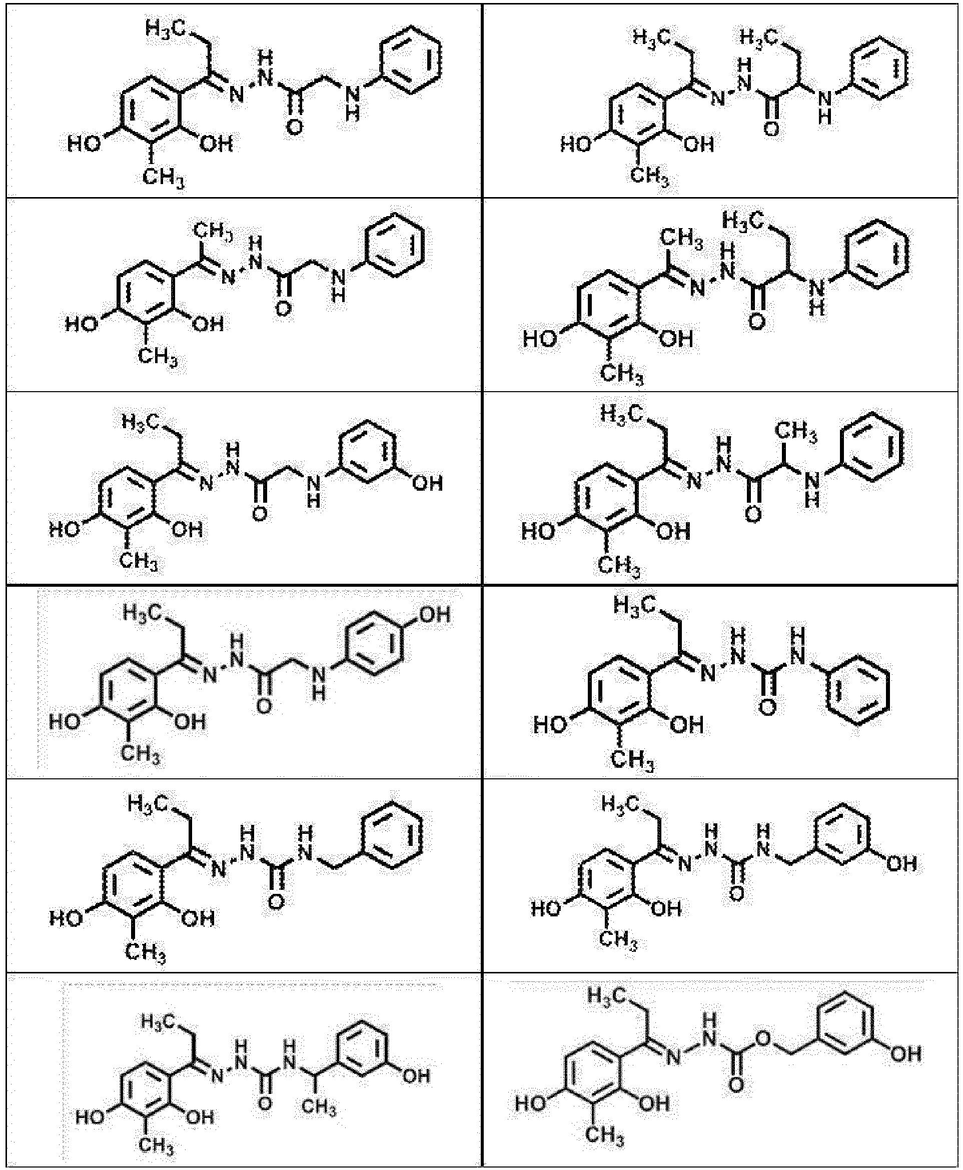

### 2. Medium additive composition

The present invention provides a medium additive composition containing a compound to be used in the present invention as an active ingredient (hereinafter sometimes referred to as "the composition of the present invention"). The composition of the present invention can achieve any or any combination of promoting cell proliferation, promoting sphere formation, promoting organoid formation, and promoting Cyst formation when added to a cell medium, particularly a three-dimensional cell culture medium.

That is, the use of the composition of the present invention is specifically exemplified by the following:
(1) promoting cell proliferation;
(2) promoting sphere formation;
(3) promoting organoid formation;
(4) promoting Cyst formation;
(5) promoting cell proliferation and promoting sphere formation;
(6) promoting cell proliferation and promoting organoid formation;
(7) promoting cell proliferation and promoting Cyst formation;
(8) promoting sphere formation and promoting organoid formation;
(9) promoting sphere formation and promoting Cyst formation;
(10) promoting organoid formation and promoting Cyst formation;
(11) promoting cell proliferation, promoting sphere formation and promoting organoid formation;
(12) promoting cell proliferation, promoting sphere formation and promoting Cyst formation;
(13) promoting cell proliferation, promoting organoid formation and promoting Cyst formation;
(14) promoting sphere formation, promoting organoid formation and promoting Cyst formation; or
(15) promoting cell proliferation, promoting sphere formation, promoting organoid formation and promoting Cyst formation.

The composition of the present invention may contain one kind or a combination of two or more kinds of the compound to be used in the present invention as an active ingredient.

In addition, the composition of the present invention optionally contains components other than the compound to be used in the present invention. Such component is not particularly limited as long as the desired effect of the present invention is obtained, and includes, for example, water, saline, dimethyl sulfoxide (DMSO), glycerol, propylene glycol, butyleneglycol, and various alcohols such as methanol, ethanol, butanol, propanol and the like, and the like. The composition of the present invention may be sterilized as necessary. The sterilization method is not particularly limited, and, for example, radiation sterilization, ethylene oxide gas sterilization, autoclave sterilization, filter sterilization and the like can be mentioned. When filter sterilization (hereinafter sometimes to be referred to as filtration sterilization) is to be performed, the material of the filter part is not particularly limited and, for example, glass fiber, nylon, PES (polyethersulfone), hydrophilic PVDF (polyvinylidene fluoride), cellulose mixed ester, celluloseacetate, polytetrafluoroethylene and the like can be mentioned. While the size of the pore in the filter is not particularly limited, it is preferably 0.1 µm to 10 µm, more preferably 0.1 µm to 1 µm, most preferably 0.1 µm to 0.5 µm. The sterilization treatment may be applied when the composition is in a solid state or a solution state.

The amount of the compound to be used in the present invention as an active ingredient in the composition of the present invention is not particularly limited as long as a medium (particularly, a three-dimensional cell culture medium) added with the composition of the present invention has a concentration that can exert the desired effect of the present invention. As the concentration at which the desired effect of the present invention can be exerted, for example, the lower limit of the concentration of the compound to be used in the present invention in the medium (particularly, three-dimensional cell culture medium) is generally not less than 0.001 µM, preferably not less than 0.01 µM, more preferably not less than 0.1 µM, further preferably not less than 1 µM, particularly preferably not less than 10 µM. The upper limit of the concentration is generally not more than 100 µM, preferably not more than 50 µM, particularly preferably not more than 10 µM.

The composition of the present invention can have any shape during provision or preservation. The composition may be in the form of a formulated solid such as tablet, pill, capsule, granule, or a liquid such as a solution obtained by dissolving in an appropriate solvent using a solubilizer or a suspension, or may be bonded to a substrate or a carrier. Examples of the additive used formulating include preservatives such as p-oxybenzoic acid esters and the like; excipients such as lactose, glucose, sucrose, mannit and the like; lubricants such as magnesium stearate, talc and the like; binders such as poly(vinyl alcohol), hydroxypropylcellulose, gelatin and the like; surfactants such as fatty acid ester and the like; plasticizers such as glycerol and the like; and the like. These additives are not limited to those mentioned above, and can be selected freely as long as they are utilizable for those of ordinary skill in the art.

The cell type whose cell proliferation and the like are promoted by adding the composition of the present invention to a medium (particularly, three-dimensional cell culture medium) is not particularly limited as long as the desired effect is obtained. Examples thereof include cell types such as reproductive cells such as spermatozoon, oocyte and the like, somatic cells constituting the living body, normal cell line, cancer cell line, progenitor cells, stem cell, cells separated from the living body and applied with artificial genetic modification, cells separated from the living body wherein the nucleus is artificially exchanged and the like. While the derivation of these cells is not particularly limited, the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like are preferable. The tissue or organ from which the cells are derived is not particularly limited as long as the desired effect of the present invention can be obtained. Examples of the aforementioned tissue include tissues such as skin, kidney, spleen, adrenal gland, liver, lung, ovary, pancreas, uterus, stomach, colon, small intestine, large intestine, spleen, bladder, prostate, testis, thymus, muscle, bond tissue, bone, joints, blood vessel tissue, blood, heart, eye, brain, nerve tissue and the like. Examples of the aforementioned organ include, but are not limited to, organs such as liver, lung, kidney, heart, pancreas, stomach, spleen, small intestine, large intestine, reproductive organ and the like. When the purpose is to promote organoid formation, the organoid may be preferably composed of cells derived from the small intestine. When the purpose is to promote Cyst formation, the Cyst may be preferably composed of cells derived from the kidney.

Examples of the normal cell lines include C3H10T1/2 (mouse embryonic fibroblast), HEK293 (human embryonic kidney cell), MDBK (bovine kidney-derived cell), MDCK (Canine kidney renal tubule epithelial cell), CHO-K1 (Chinese hamster ovary-derived cell), Vero cell (Cercopithecus aethiops kidney epithelium-derived cell), NIH3T3 (mouse fetal fibroblast), HepaRG (hepatocyte, registered trade mark), HUVEC (human umbilical vein endothelial cell), human primary culture hepatocyte and the like. Among these, particularly MDCK, HUVEC, CHO-K1 and Vero cell are preferable. Examples of the cancer cell line include, but are not limited to, HBC-4, BSY-1, BSY-2, MCF-7, MCF-7/ADR RES, HS578T, MDA-MB-231, MDA-MB-435, MDA-N, BT-549, T47D as human breast cancer cell lines, HeLa as human cervical carcinoama cell line, A549, EKVX, HOP-62, HOP-92, NCI-H23, NCI-H226, NCI-H322M, NCI-H460, NCI-H522, DMS273, DMS114 as human lung cancer cell line, Caco-2, COLO-205, HCC-2998, HCT-15, HCT-116, HT-29, KM-12, SW-620, WiDr as human colon cancer cell line, DU-145, PC-3, LNCaP as human prostate cancer cell line, U251, SF-295, SF-539, SF-268, SNB-75, SNB-78, SNB-19 as human central nervous system cancer cell line, OVCAR-3, OVCAR-4, OVCAR-5, OVCAR-8, SK-OV-3, IGROV-1 as human ovarian cancer cell line, RXF-631L, ACHN, UO-31, SN-12C, A498, CAKI-1, RXF-393L, 786-0, TK-10 as human kidney cancer cell line, MKN45, MKN28, St-4, MKN-1, MKN-7, MKN-74 as human stomach cancer cell line, LOX-IMVI, LOX, MALME-3M, SK-MEL-2, SK-MEL-5, SK-MEL-28, UACC-62, UACC-257, M14 as skin cancer cell line, CCRF-CRM, K562, MOLT-4, HL-60TB, RPMI8226, SR, UT7/TPO, Jurkat as leukemia cell line. Among these, human ovarian cancer cell line SKOV3, human uterus cervix cancer cell line HeLa, human malignant melanoma derived from cell line A375, human epithelium-like cell cancer-derived cell line A431, human stomach adenocarcinoma-derived cell line AGS, human prostate cancer-derived cell line LNCap clone FGC, human colon adenocarcinoma-derived cell line HCT116, human alveolar basal epithelial adenocarcinoma-derived cell line A549, and human prostate cancer-derived cell DU145 are particularly preferable. Furthermore, stem cells are cells concurrently having an ability to replicate itself, and an ability to differentiate into other plural lineages. Examples thereof include, but are not limited to, embryonic stem cells (ES cells), embryonic tumor cells, embryonic germ stem cells, artificial pluripotent stem cells (iPS cells), neural stem cells, hematopoietic stem cells, mesenchymal stem cells, liver stem cells, pancreas stem cells, muscle stem cells, germ stem cells, intestinal stem cells, cancer stem cells, hair follicle stem cells and the like. Examples of the pluripotent stem cells include ES cells, embryonic germ stem cells and iPS cells, from among the aforementioned stem cells. Progenitor cells are cells on the way to differentiate from the aforementioned stem cells into particular somatic cells or reproductive cells. As the stem cells, iPS cells and mesenchymal stem cells (MSCs) are particularly preferable.

In one embodiment, when stem cells such as MSCs and the like are cultured using a three-dimensional cell culture medium added with the composition of the present invention, the cell proliferation thereof can be promoted while maintaining the characteristics (e.g., undifferentiated state) of the cells. Maintenance of the undifferentiated state of MSCs can be confirmed by analyzing expression of a cell surface marker by flow cytometry (FCM) (e.g., WO 2016/136986). Examples of the cell surface marker of MSC include CD29, CD73, CD90, CD105 and the like being positive. Therefore, the present invention can also be preferably used for a large-scale production of stem cells such as MSCs and the like.

In the present specification, the term "the composition of the present invention" can be replaced with the term "the agent of the present invention" or "the medium additive agent of the present invention".

### 3. Medium

The present invention provides a medium containing the compound to be used in the present invention or the composition of the present invention (hereinafter sometimes referred to as "the medium of the present invention"). Using the medium of the present invention, any or any combination of promoting cell proliferation, promoting sphere formation, promoting organoid formation, and promoting Cyst formation can be achieved. The medium of the present invention is particularly preferably a three-dimensional cell culture medium.

The concentration of the compound to be used in the present invention which is contained in the medium of the present invention as an active ingredient is not particularly limited as long as the desired effect of the present invention is obtained. For example, the lower limit of the concentration of the compound to be used in the present invention in the medium (particularly, three-dimensional cell culture medium) is generally not less than 0.001 µM, preferably not less than 0.01 µM, more preferably not less than 0.1 µM, further preferably not less than 1 µM, particularly preferably not less than 10 µM. The upper limit of the concentration is generally not more than 100 µM, preferably not more than 50 µM, particularly preferably not more than 10 µM.

The medium of the present invention can have the same composition as that of a known medium, except that the compound to be used in the present invention or the composition of the present invention is blended.

In one embodiment, the medium of the present invention can be prepared by adding the compound or composition to be used in the present invention to a commercially available medium (particularly three-dimensional cell culture medium). A commercially available medium that can be made into the medium of the present invention by adding the compound to be used in the present invention or the composition of the present invention is not particularly limited as long as the desired effect is obtained. Examples of the medium include Dulbecco's Modified Eagle's Medium (DMEM), HamF12 medium (Ham's Nutrient Mixture F12), DMEM/F12 medium, McCoy's 5A medium, Eagle MEM (Eagle's Minimum Essential Medium; EMEM), αMEM (alpha Modified Eagle's Minimum Essential Medium; αMEM), MEM (Minimum Essential Medium), RPMI1640 medium, Iscove's Modified Dulbecco's Medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma Aldrich), QBSF-60 (manufactured by Qualitybiological), StemPro hESC SFM (manufactured by Invitrogen), Essential8 (registered trade mark) medium (manufactured by Gibco), mTeSR1 medium (manufactured by STEMCELL Technologies), mTeSR2 medium (manufactured by STEMCELL Technologies), ReproFF (manufactured by ReproCELL), ReproFF2 (manufactured by ReproCELL), StemFit (registered trade mark) AK02N (manufactured by Ajinomoto Co., Inc.), StemFit (registered trade mark) AK03N (manufactured by Ajinomoto Co., Inc.), PSGro hESC/iPSC medium (manufactured by System Biosciences), NutriStem (registered trade mark) medium (manufactured by Biological Industries), CSTI-7 medium (manufactured by Cell Science & Technology Institute, Inc.), MesenPRO RS medium (manufactured by Gibco), MF-Medium (registered trade mark) mesenchymal stem cell proliferation medium (manufactured by TOYOBO CO., LTD.), medium for mesenchymal stem cell (manufactured by PromoCell), Sf-900II (manufactured by Invitrogen), Opti-Pro (manufactured by Invitrogen), and the like. In addition, a three-dimensional cell culture medium obtained by adding polysaccharides such as deacylated gellan gum and the like to these media can be used. Examples of such three-dimensional cell culture medium include, but are not limited to, FCeM (registered trade mark) (manufactured by Wako Pure Chemical Industries, Ltd.).

In addition, it is possible to add, according to the object, sodium, potassium, calcium, magnesium, phosphorus, chlorine, various amino acids, various vitamins, antibiotics, serum, fatty acids, sugars, cell growth factors, differentiation inducing factors, cell adhesion factors, antibodies, enzymes, cytokines, hormones, lectins, extracellular matrices, bioactive substances, and the like to the above-mentioned medium.

When cells are cultivated in the medium of the present invention (particularly three-dimensional cell culture), culture vessels generally used for cell culture such as schales, flasks, plastic bags, Teflon (registered trade mark) bags, dishes, schales, dishes for tissue culture, multidishes, microplates, microwell plates, multiplates, multiwell plates, chamber slides, tubes, trays, culture bags, roller bottles and the like can be used for cultivation. These culture containers are desirably low cell -adhesive so that the (adherent) cells to be cultured will not adhere to the culture container. As a cell-nonadhesive culture vessel, a culture vessel having a surface not artificially treated to improve adhesiveness to cells (e.g., coating treatment with extracellular matrix and the like), or a culture vessel having a surface artificially treated to reduce adhesiveness to cells can be used. Examples of such container include, but are not limited to, Sumilon cell-tight plate (manufactured by SUMITOMO BAKELITE CO., LTD.), PrimeSurface (registered trade mark) plate (manufactured by SUMITOMO BAKELITE CO., LTD.), Ultra-low Attachment surface plate (manufactured by Corning Incorporated), Nunclon Spheraplate (manufactured by Thermo Fisher Scientific) and the like.

### 4. Cell proliferation promoting method, sphere formation promoting method, organoid formation promoting method, and Cyst formation promoting method

The present invention provides a method for promoting cell proliferation, a method for promoting sphere formation, a method for promoting organoid formation, or a method for promoting Cyst formation method (hereinafter these are sometimes collectively referred to as "the method of the present invention"), each including adding the compound to be used in the present invention or the composition of the present invention to a medium.

The medium to be used in the method of the present invention is not particularly limited as long as the desired effect is obtained. Preferred is a three-dimensional cell culture medium. The cell culture conditions (e.g., temperature, carbon dioxide concentration, culture period etc.) used in the method of the present invention may be those for a method known per se, or may be appropriately modified according to the purpose. For example, the temperature for culturing cells in the case of animal cells is generally 25°C - 39°C, preferably 33°C - 39°C (e.g., 37°C). The carbon dioxide concentration is generally 4% by volume - 10% by volume, preferably 4% by volume - 6% by volume, in the atmosphere of culture. The culture period is generally 1 to 35 days, which can be appropriately set according to the purpose of the culture.

A method for forming a cell aggregate (sphere) is not particularly limited, and can be appropriately selected by those of ordinary skill in the art. Examples thereof include a method using a container having a cell non-adhesive surface, hanging drop method, gyratory culture method, three-dimensional scaffold method, centrifugation method, a method using coagulation by an electric field or magnetic field and the like. For example, using a method using a container having a cell non-adhesive surface, the target cells are cultured in a culture container such as schale and the like applied with a surface treatment to inhibit cell adhesion, whereby a sphere can be formed. Such cell non-adhesive culture container is used, the target cells are first collected, a cell suspension thereof is prepared and plated in the culture container to perform culture. When culture is continued for about 1 week, the cells spontaneously form a sphere. As a cell non-adhesive surface used here, a surface of a culture container generally used such as schale and the like, which is coated with a substance inhibiting cell adhesion and the like can be used. Examples of such substance include agarose, agar, copolymer of poly-HEMA(poly-(2-hydroxl-ethylmethacrylate)2-methacryloyloxyethylphosphoryl choline and other monomer (e.g., butylmethacrylate etc.), poly(2-methoxymethylacrylate), poly-N-isopropylacrylamide, mebiol gel (registered trade mark) and the like. When cytotoxicity is absent, the substance is not limited thereto.

As a method for forming a cell aggregate (sphere), the methods described in NATURE BIOTECHNOLOGY, VOL. 28, NO. 4, APRIL 2010, 361-366, NATURE PROTOCOLS, VOL. 6, NO. 5, 2011, 689-700, NATURE PROTOCOLS, VOL. 6, NO. 5, 2011, 572-579, Stem Cell Research, 7, 2011, 97-111, Stem Cell Rev and Rep, 6, 2010, 248-259 and the like can also be used.

In addition, a medium used for culture for forming a sphere can also contain a component that promotes formation of a sphere or promotes maintenance thereof. Examples of the component having such effect include dimethyl sulfoxide, superoxide dismutase, caeruloplasmin, catalase, peroxidase, L-ascorbic acid, L-ascorbic acid phosphate, tocopherol, flavonoid, uric acid, bilirubin, selenium-containing compound, transferrin, unsaturated fatty acid, albumin, theophylline, forskolin, glucagon, dibutyryl cAMP and the like. As the selenium-containing compound, ROCK inhibitors such as sodium selenite, sodium selenate, dimethyl selenide, hydrogen selenide, Selenomethionine, Se-Methylselenocysteine, Selenocystathionine, Selenocysteine, Selenohomocysteine, adenosine-5'-triphosphoric acid, Se-Adenosylselenomethionine, Y27632, Fasudil (HA1077), H-1152, Wf-536 and the like can be mentioned. To obtain the object cell aggregate having a uniform size, plural concaves having the same diameter as the object cell aggregate can also be introduced onto a cell non-adhesive culture container to be used. When these concaves are in contact with each other or within the range of the diameter of the object cell aggregate, and cells are plated, the plated cells do not form a cell aggregate between concaves but certainly form a cell aggregate with a size corresponding to the volume thereof in the concave, thus affording a cell aggregate population having a uniform size. As the shape of the concave in this case is preferably a hemisphere or cone.

Alternatively, a sphere can also be formed based on a support showing cell adhesiveness. Examples of such support include collagen, polyrotaxane, polylactic acid (PLA), polylactic acid glycolic acid (PLGA) copolymer, hydrogel and the like.

In addition, a sphere can also be formed by co-cultivating with a feeder cell. As a feeder cell to promote sphere formation, any adhesive cell can be used. Preferably, a feeder cell for each kind of cell is desirable. Although not limited, for example, when a sphere of cells derived from the liver or cartilage is formed, examples of the feeder cells include COS-1 cells and vascular endothelial cells as preferable cell types.

Alternatively, a hanging drop method can also be selected as a method for forming a sphere. As the hanging drop method, for example, a method including spotting a droplet (about 10 - 50 µL in volume) of a cell suspension on the ceiling side such as a lid of a culture vessel, and culturing in an inverted state such that the placed droplet hangs can be mentioned. By culturing in this manner, the cells are minimally influenced by a contact with the flat surface and form a sphere at the bottom of the droplet. Such droplet can also be prepared using a special culture vessel such as GravityPLUS Plate (manufactured by PerkinElmer). Specifically, a sphere can be prepared using a droplet containing 100 - 100000 cells, preferably 200 - 10000 cells, more preferably 500 - 10000 cells. To form spheres, it is preferable to culture for 6 -48 hr.

The size of the sphere varies depending on the cell type and culture period and is not particularly limited. When it has a spherical shape or ellipse spherical shape, the diameter thereof is 20 µm to 1000 µm, preferably 40 µm to 500 µm, more preferably 50 µm to 300 µm, most preferably 80 µm to 200 µm.

Such sphere can maintain proliferative capacity for not less than 10 days, preferably not less than 13 days, more preferably not less than 30 days, by continuing the standing culture. By regularly further performing, during the standing culture, mechanical division, or a single cell -forming treatment and coagulation, the proliferative capacity can be maintained substantially infinitely.

The culture container to be used for culturing sphere is not particularly limited as long as it generally permits animal cell culture. For example, flasks, dishes, schales, tissue culture dishes, multidishes, microplates, microwell plates, multiplates, multiwall plates, chamber slides, cell culture flasks, spinner flasks, schales, tubes, trays, culture bags, roller bottles, EZ SPHERE (manufactured by AGC TECHNO GLASS CO., LTD.), Sumilon celltight plates (manufactured by SUMITOMO BAKELITE CO., LTD.) and the like can be mentioned.

Of these culture containers, microplates, microwell plates, multiplates and multiwall plates are preferably used when evaluation of many anticancer drugs, pharmaceutical product candidate compounds or pharmaceutical products is performed. While the well bottom shape of these plates is not particularly limited, flat bottom, U-shaped bottom and V-shaped bottom can be used, and U-shaped bottom is preferably used. While the materials of these culture tools are not particularly limited, for example, glass, plastics such as polyvinyl chloride, cellulosic polymers, polystyrene, polymethylmethacrylate, polycarbonate, polysulfone, polyurethane, polyester, polyamide, polystyrene, polypropylene and the like, and the like can be mentioned.

The medium used for embedding culture can contain a cell adhesion factor, and examples thereof include Matrigel (registered trade mark), Geltrex (registered trade mark), collagen, gelatin, poly- L-lysine, poly- D-lysine, laminin, fibronectin, vitronectin, tenascin, selectin, hyaluronic acid, fibrin and the like. Two or more kinds of these cell adhesion factors can also be added in combination. Furthermore, the medium to be used for embedding culture can be mixed with a thickener such as agar, guar gum, tamarind gum, alginic acid propylene glycol, locust bean gum, gum arabic, tara gum, tamarind gum, methylcellulose, carboxymethylcellulose, agarose, tamarind seed gum, pullulan and the like. Two or more kinds of these thickeners can also be added in combination.

A method for forming an organoid (mini-organ formed by culturing stem cells or progenitor cells in vitro in a three-dimensional environment) or Cyst (luminal structure formed by epithelial cells) is not particularly limited, and can be appropriately selected by those of ordinary skill in the art. As an example, a method using the above-mentioned embedding culture can be mentioned. Specifically, an organoid or Cyst can be formed by culturing target cells or tissues in medium for embedding culture containing the above-mentioned cell adhesion factor. For example, after target cells or tissues is collected, a suspension thereof is prepared, and the suspension is seeded in a medium for embedding culture and cultured. After culturing for 3 to 14 days, the cells spontaneously form an organoid or Cyst.

The medium used in the method of the present invention (particularly three-dimensional cell culture medium) may be the medium of the present invention.

The concentration, cell type, and the like of the compound to be used in the present invention or the composition of the present invention in the method of the present invention are the same as those described in "2. Medium additive composition".

While the present invention is explained in more detail in the following by referring to Examples, the present invention is not limited by the Examples. Unless particularly indicated, the reagents and the like to be used are commercially available.

### [Example]

The synthesis methods and structural formulas of the compounds to be used in the present invention are shown below. ¹H-NMR shows proton nuclear magnetic resonance spectrum which was measured at 270 MHz or 400 MHz in deuterodimethyl sulfoxide. As the chemical shift value, the value of deuterodimethyl sulfoxide is shown as 2.49 ppm. In addition, s shows singlet, and similarly, brs shows broad singlet, d shows doublet, dd shows double doublet, t shows triplet, q shows quartet, and m shows multiplet.

### [Synthetic Example 1] Synthesis of the compound of the present invention using ketone and hydrazide as starting materials

### (Materials and methods)

Compounds were synthesized from 4 kinds of ketones [1-(2,4-dihydroxy-3-methylphenyl)propan-1-one (hereinafter abbreviated as k-1), 1-(2,4-dihydroxy-3-methylphenyl)ethan-1-one (hereinafter abbreviated as k-2), 1-(2,4,6-trihydroxy-3-methylphenyl)propan-1-one (hereinafter abbreviated as k-3), 1-(2,4-dihydroxy-3-methylphenyl)-3-methylbutan-1-one (hereinafter abbreviated as k-5)] and 10 kinds of hydrazides [2-(phenylamino)acetohydrazide (hereinafter abbreviated as H-1), 2-(o-tolylamino)acetohydrazide (hereinafter abbreviated as H-2), 2-(m-tolylamino)acetohydrazide (hereinafter abbreviated as H-3), 2-(p-tolylamino)acetohydrazide (hereinafter abbreviated as H-4), 2-[(4-fluorophenyl)amino]acetohydrazide (hereinafter abbreviated as H-5), 2-(naphthalen-1-ylamino)acetohydrazide (hereinafter abbreviated as H-6), 2-(phenylamino)butanehydrazide (hereinafter abbreviated as H-7), 2-[(4-ethoxyphenyl)amino]acetohydrazide (hereinafter abbreviated as H-9), 2-[(4-hydroxyphenyl)amino]acetohydrazide (hereinafter abbreviated as D-2), 2-[(2-hydroxyphenyl)amino]acetohydrazide (hereinafter abbreviated as D-4)] shown in the following.

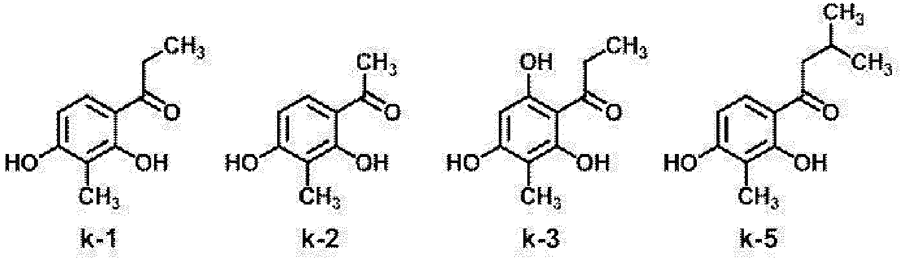

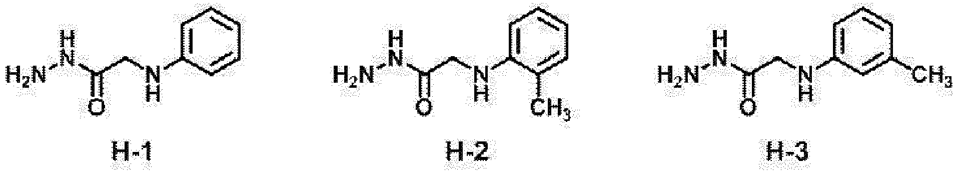

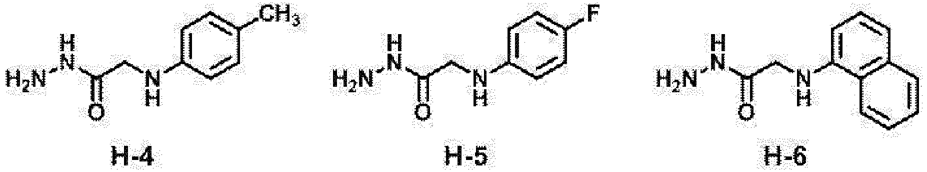

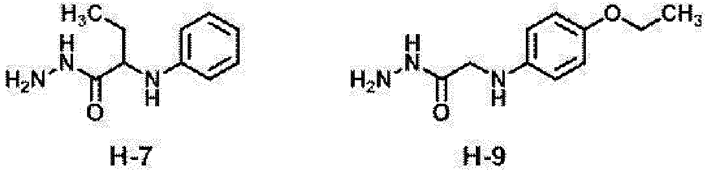

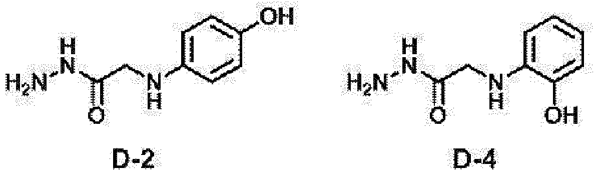

The synthesized 34 compounds are described in the first table. In the table, Me is methyl, and similarly, Et is ethyl, n-Pr is normal propyl, i-Bu is isobutyl, Ph is phenyl, and Naph is naphthyl. In (R₃)ₙ, "-" means unsubstituted, and the numbers indicated in the structural formulas show the substitutable position of (R₃)ₙ.

### [the first table]

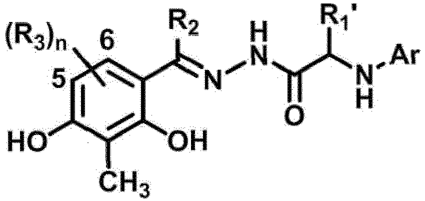

**[Table 1]**

| compound No. | R₂ | (R₃)ₙ | R₁' | Ar |
|---|---|---|---|---|
| k-1:H-1 | Et | - | H | Ph |
| k-1:H-2 | Et | - | H | 2-Me-Ph |
| k-1:H-3 | Et | - | H | 3-Me-Ph |
| k-1:H-4 | Et | - | H | 4-Me-Ph* |
| k-1:H-5 | Et | - | H | 4-F-Ph |
| k-1:H-6 | Et | - | H | 1-Naph |
| k-1:H-7 | Et | - | Et | Ph |
| k-1:H-9 | Et | - | H | 4-EtO-Ph* |
| k-1:D-2 | Et | - | H | 4-OH-Ph |
| k-1:D-4 | Et | - | H | 2-OH-Ph |
| k-2:H-1 | Me | - | H | Ph |
| k-2:H-2 | Me | - | H | 2-Me-Ph |
| k-2:H-3 | Me | - | H | 3-Me-Ph |
| k-2:H-4 | Me | - | H | 4-Me-Ph |
| k-2:H-5 | Me | - | H | 4-F-Ph |
| k-2:H-6 | Me | - | H | 1-Naph |
| k-2:H-7 | Me | - | Et | Ph |
| k-2:H-9 | Me | - | H | 4-EtO-Ph |
| k-3:H-1 | Et | 6-OH | H | Ph |
| k-3:H-2 | Et | 6-OH | H | 2-Me-Ph |
| k-3:H-3 | Et | 6-OH | H | 3-Me-Ph |
| k-3:H-4 | Et | 6-OH | H | 4-Me-Ph |
| k-3:H-5 | Et | 6-OH | H | 4-F-Ph |
| k-3:H-6 | Et | 6-OH | H | 1-Naph |
| k-3:H-7 | Et | 6-OH | Et | Ph |
| k-3:H-9 | Et | 6-OH | H | 4-EtO-Ph |
| k-5:H-1 | i-Bu | - | H | Ph |
| k-5:H-2 | i-Bu | - | H | 2-Me-Ph |
| k-5:H-3 | i-Bu | - | H | 3-Me-Ph |
| k-5:H-4 | i-Bu | - | H | 4-Me-Ph |
| k-5:H-5 | i-Bu | - | H | 4-F-Ph |
| k-5:H-6 | i-Bu | - | H | 1-Naph |
| k-5:H-7 | i-Bu | - | Et | Ph |
| k-5:H-9 | i-Bu | - | H | 4-EtO-Ph* |

| | | | | |
|---|---|---|---|---|
| *Reference Example | | | | |

The measurement results of ¹H-NMR of the compounds described in the first table are shown in the second table.

### [the second table]

**[Table 2-1]**

| compound No. | ¹H-NMR (270 MHz) |
|---|---|
| k-1:H-1 | δ13.98(s, 1H), 11.13(s, 1H), 10.01(s, 1H), 7.58(d, J=8.1Hz, 1H), 7.41(t, J=8.1Hz, 2H), 6.95(d, J=8.1Hz, 2H), 6.89(t, J=8.1Hz, 1H), 6.72(d, J=8.1Hz, 1H), 6.29(t, J=8.1Hz, NH), 4.27(d, J=8.1Hz, 2H), 3.11(q, J=8.1Hz, 2H), 2.29(s, 3H), 1.37(t, J=8.1Hz, 3H). |
| k-1:H-2 | δ13.63(s, 1H), 10.80(s, 1H), 9.67(s, 1H), 7.24(d, J=8.1Hz, 1H), 7.00(t, J=8.1Hz, 1H), 6.99(d, J=8.1Hz, 1H), 6.54(t, J=8.1Hz, 1H), 6.46(d, J=8.1Hz, 1H), 6.38(d, J=8.1Hz, 1H), 5.27(t, J=8.1Hz, NH), 3.98(d, J=5.4Hz, 2H), 2.76(q, J=5.4Hz, 2H), 2.14(s, 3H), 1.95(s, 3H), 1.02(t, J=8.1Hz, 3H). |
| k-1:H-3 | δ13.64(s, 1H), 10.77(brs, 1H), 9.68(brs, 1H), 7.24(d, J=8.1Hz, 1H), 6.96(t, J=8.1Hz, 1H), 6.50-6.30(m, 4H), 5.86(t, J=5.4Hz, NH), 3.91(d, J=5.4Hz, 2H), 2.78(q, J=8.1Hz, 2H), 2.17(s, 3H), 1.95(s, 3H), 1.04(t, J=8.1Hz, 3H). |
| k-1:H-4* | δ13.65(s, 1H), 10.76(brs, 1H), 9.68(brs, 1H), 7.25(d, J=8.1Hz, 1H), 6.91(d, J=8.1Hz, 2H), 6.54(d, J=8.1Hz, 2H), 6.39(d, J=8.1Hz, 1H), 5.76(t, J=5.4Hz, NH), 3.90(d, J=5.4Hz, 2H), 2.78(q, J=8.1Hz, 2H), 2.14(s, 3H), 1.96(s, 3H), 1.04(t, J=8.1Hz, 3H). |
| k-1:H-5 | δ13.64(s, 1H), 10.80(brs, 1H), 9.67(brs, 1H), 7.24(d, J=10.8Hz, 1H), 6.93(m, 2H), 6.60(m, 2H), 6.38(d, J=10.8Hz, 1H), 5.93(t, J=8.1Hz, NH), 3.91(d, J=5.4Hz, 2H), 2.78(q, J=8.1Hz, 2H), 1.95(s, 3H), 1.04(t, J=8.1Hz, 3H). |
| k-1:H-6 | δ13.65(s, 1H), 10.91(brs, 1H), 9.67(brs, 1H), 8.16(d, J=8.1Hz, 1H), 7.77(d, J=8.1Hz, 1H), 7.50-7.20(m, 4H), 7.15(d, J=8.1Hz, 1H), 6.61(t, J=8.1Hz, NH), 6.45(d, J=5.4Hz, 1H), 6.38(d, J=10.8Hz, 1H), 4.14(d, J=5.4Hz, 2H), 2.78(q, J=8.1Hz, 2H), 1.95(s, 3H), 1.02(t, J=8.1Hz, 3H). |
| k-1:H-7 | δ13.64(s, 1H), 10.79(s, 1H), 9.67(s, 1H), 7.24(d, J=8.1Hz, 1H), 7.05(t, J=8.1Hz, 2H), 6.66(d, J=8.1Hz, 2H), 6.53(t, J=8.1Hz, 1H), 6.38(d, J=8.1Hz, 1H), 5.83(d, J=8.1Hz, NH), 4.13(m, J=8.1Hz, 1H), 2.80(q, J=8.1Hz, 2H), 1.94(s, 3H), 1.76(m, 2H), 1.02(t, J=8.1Hz, 3H), 0.98(t, J=8.1Hz, 3H). |
| k-1:H-9* | δ13.64(s, 1H), 10.73(s, 1H), 9.67(brs, 1H), 7.23(d, J=8.1Hz, 1H), 6.72(d, J=8.1Hz, 2H), 6.56(d, J=8.1Hz, 2H), 6.38(d, J=8.1Hz, 1H), 5.58(t, J=8.1Hz, NH), 3.87(q, J=8.1Hz, 2H), 3.86(d, J=8.1Hz, 2H), 2.76(q, J=8.1Hz, 2H), 1.95(s, 3H), 1.24(t, J=8.1Hz, 3H), 1.02(t, J=8.1Hz, 3H). |

| | |
|---|---|
| *Reference Example | |

**[Table 2-2]**

| compound No. | ¹H-NMR (270 MHz) |
|---|---|
| k-1:D-2 | δ13.65(s, 1H), 10.71(brs, 1H), 9.69(brs, 1H), 8.48(s, 1H), 7.25(d, J=8.1Hz, 1H), 6.57(d, J=8.1Hz, 2H), 6.49(d, J=8.1Hz, 2H), 6.39(d, J=10.8Hz, 1H), 5.40(t, J=8.1Hz, NH), 3.84(d, J=5.4Hz, 2H), 2.76(q, J=8.1Hz, 2H), 1.96(s, 3H), 1.02(t, J=8.1Hz, 3H). |
| k-1:D-4 | δ13.65(s, 1H), 10.88(brs, 1H), 9.70(s, 1H), 9.37(s, 1H), 7.26(d, J=8.1Hz, 1H), 6.69(d, J=8.1Hz, 1H), 6.65(t, J=8.1Hz, 1H), 6.50-6.35(m, 3H), 5.15(t, J=8.1Hz, NH), 3.95(d, J=5.4Hz, 2H), 2.78(q, J=8.1Hz, 2H), 1.97(s, 3H), 1.03(t, J=8.1Hz, 3H). |
| k-2:H-1 | 13.59(s, 1H), 10.82(brs, 1H), 9.67(brs, 1H), 7.25(d, J=8.1Hz, 1H), 7.08(t, J=8.1Hz, 2H), 6.62(d, J=8.1Hz, 2H), 6.56(t, J=8.1Hz, 1H), 6.38(d, J=8.1Hz, 1H), 5.93(t, J=8.1Hz, NH), 3.91(d, J=5.4Hz, 2H), 2.28(s, 3H), 1.96(s, 3H). |
| k-2:H-2 | δ13.58(s, 1H), 10.85(brs, 1H), 9.67(brs, 1H), 7.25(d, J=8.1Hz, 1H), 7.10-6.90(m, 2H), 6.54(t, J=8.1Hz, 1H), 6.45(d, J=8.1Hz, 1H), 6.38(d, J=8.1Hz, 1H), 5.25(t, J=8.1Hz, NH), 3.97(d, J=8.1Hz, 2H), 2.27(s, 3H), 2.14(s, 3H), 1.96(s, 3H). |
| k-2:H-3 | δ13.59(s, 1H), 10.78(brs, 1H), 9.67(brs, 1H), 7.25(d, J=8.1Hz, 1H), 6.96(t, J=8.1Hz, 1H), 6.45-6.30(m, 4H), 5.82(t, J=8.1Hz, NH), 3.89(d, J=5.4Hz, 2H), 2.27(s, 3H), 2.18(s, 3H), 1.96(s, 3H): |
| k-2:H-4 | δ13.59(s, 1H), 10.78(brs, 1H), 9.67(brs, 1H), 7.25(d, J=8.1Hz, 1H), 6.90(d, J=8.1Hz, 2H), 6.52(d, J=8.1Hz, 2H), 6.38(d, J=8.1Hz, 1H), 5.72(t, J=8.1Hz, NH), 3.87(d, J=5.4Hz, 2H), 2.27(s, 3H), 2.14(s, 3H), 1.96(s, 3H). |
| k-2:H-5 | δ13.58(s, 1H), 10.80(brs, 1H), 9.67(brs, 1H), 7.25(d, J=8.1Hz, 1H), 6.95-6.80(m, 2H), 6.65-6.50(m, 2H), 6.38(d, J=8.1Hz, 1H), 5.90(t, J=8.1Hz, NH), 3.89(d, J=5.4Hz, 2H), 2.28(s, 3H), 1.96(s, 3H). |
| k-2:H-6 | δ13.60(s, 1H), 10.95(brs, 1H), 9.67(brs, 1H), 8.16(d, J=8.1Hz, 1H), 7.77(d, J=8.1Hz, 1H), 7.50-7.35(m, 2H), 7.35-7.20(m, 2H), 7.15(d, J=8.1Hz, 1H), 6.61(t, J=8.1Hz, NH), 6.44(d, J=8.1Hz, 1H), 6.38(d, J=8.1Hz, 1H), 4.12(d, J=5.4Hz, 2H), 2.30(s, 3H), 1.95(s, 3H). |
| k-2:H-7 | δ13.59(s, 1H), 10.83(brs, 1H), 9.67(brs, 1H), 7.24(d, J=8.1Hz, 1H), 7.06(t, J=8.1Hz, 2H), 6.65(d, J=8.1Hz, 2H), 6.53(t, J=8.1Hz, 1H), 6.37(d, J=8.1Hz, 1H), 5.80(d, J=8.1Hz, NH), 4.05(m, 1H), 2.28(s, 3H), 1.95(s, 3H), 1.75(m, 2H), 0.98(d, J=8.1Hz, 3H). |
| k-2:H-9 | δ13.59(s, 1H), 10.75(brs, 1H), 9.67(brs, 1H), 7.25(d, J=8.1Hz, 1H), 6.72(d, J=8.1Hz, 2H), 6.56(d, J=8.1Hz, 2H), 6.37(d, J=8.1Hz, 1H), 5.54(t, J=8.1Hz, NH), 3.87(q, J=8.1Hz, 2H), 3.85(d, J=8.1Hz, 2H), 2.26(s, 3H), 1.96(s, 3H), 1.25(t, J=8.1Hz, 3H). |

**[Table 2-3]**

| compound No. | ¹H-NMR (270 MHz) |
|---|---|
| k-3:H-1 | δ13.07(s, 1H), 10.62(s, 1H), 9.67(s, 1H), 9.45(s, 1H), 7.09(t, J=8.1Hz, 2H), 6.62(d, J=8.1Hz, 2H), 6.58(t, J=8.1Hz, 1H), 6.01(t, J=8.1Hz, NH), 5.95(s, 1H), 3.92(d, J=5.4Hz, 2H), 2.88(q, J=8.1Hz, 2H), 1.84(s, 3H), 1.00(t, J=8.1Hz, 3H). |
| k-3:H-2 | δ13.06(s, 1H), 10.62(s, 1H), 9.67(s, 1H), 9.45(s, 1H), 7.01(t, J=8.1Hz, 1H), 7.00(d, J=8.1Hz, 1H), 6.55(t, J=8.1Hz, 1H), 6.47(d, J=8.1Hz, 1H), 5.95(s, 1H), 5.33(t, J=8.1Hz, NH), 3.97(d, J=5.4Hz, 2H), 2.87(q, J=8.1Hz, 2H), 2.15(s, 3H), 1.84(s, 3H), 0.99(t, J=8.1Hz, 3H). |
| k-3:H-3 | δ13.07(s, 1H), 10.60(s, 1H), 9.67(s, 1H), 9.45(s, 1H), 6.97(t, J=8.1Hz, 1H), 6.45-6.30(m, 3H), 5.95(s, 1H), 5.89(t, J=8.1Hz, NH), 3.90(d, J=5.4Hz, 2H), 2.88(q, J=8.1Hz, 2H), 2.18(s, 3H), 1.84(s, 3H), 1.01(t, J=8.1Hz, 3H). |
| k-3:H-4 | δ13.07(s, 1H), 10.58(s, 1H), 9.67(s, 1H), 9.45(s, 1H), 6.92(d, J=8.1Hz, 2H), 6.54(d, J=8.1Hz, 2H), 5.95(s, 1H), 5.79(t, J=5.4Hz, NH), 3.88(d, J=5.4Hz, 2H), 2.87(q, J=8.1Hz, 2H), 2.15(s, 3H), 1.84(s, 3H), 1.00(t, J=8.1Hz, 3H). |
| k-3:H-5 | δ13.07(s, 1H), 10.63(s, 1H), 9.67(s, 1H), 9.45(s, 1H), 7.00-6.80(m, 2H), 6.70-6.65(m, 2H), 5.96(t, J=5.4Hz, NH), 5.95(s, 1H), 3.90(d, J=5.4Hz, 2H), 2.89(q, J=8.1Hz, 2H), 1.85(s, 3H), 1.01(t, J=8.1Hz, 3H). |
| k-3:H-6 | δ13.04(s, 1H), 10.71(s, 1H), 9.66(s, 1H), 9.43(s, 1H), 8.16(d, J=8.1Hz, 1H), 7.78(d, J=8.1Hz, 1H), 7.50-7.35(m, 2H), 7.27(t, J=8.1Hz, 1H), 7.14(t, J=8.1Hz, 1H), 6.62(t, J=5.4Hz, NH), 6.46(d, J=8.1Hz, 1H), 5.94(s, 1H), 4.12(d, J=5.4Hz, 2H), 2.87(q, J=8.1Hz, 2H), 1.83(s, 3H), 0.96(t, J=8.1Hz, 3H). |
| k-3:H-7 | δ13.09(s, 1H), 10.66(s, 1H), 9.67(s, 1H), 9.45(s, 1H), 7.06(t, J=10.8Hz, 2H), 6.67(d, J=8.1Hz, 2H), 6.55(t, J=8.1Hz, 1H), 5.95(s, 1H), 5.83(d, J=10.8Hz, NH), 4.07(m, 1H), 2.92(q, J=8.1Hz, 2H), 1.83(s, 3H), 1.77(m, 2H), 0.99(t, J=8.1Hz, 3H). |
| k-3:H-9 | δ13.08(s, 1H), 10.57(s, 1H), 9.67(s, 1H), 9.45(s, 1H), 6.75(d, J=10.8Hz, 2H), 6.57(d, J=8.1Hz, 2H), 5.95(s, 1H), 5.63(t, J=5.4Hz, NH), 3.88(q, J=8.1Hz, 2H), 3.86(d, J=8.1Hz, 2H), 2.86(q, J=8.1Hz, 2H), 1.84(s, 3H), 1.26(t, J=8.1Hz, 3H), 0.99(t, J=8.1Hz, 3H). |

**[Table 2-4]**

| compound No. | ¹H-NMR (270 MHz) |
|---|---|
| k-5:H-1 | δ13.71(s, 1H), 10.67(brs, 1H), 9.66(brs, 1H), 7.23(d, J=8.1Hz, 1H), 7.07(t, J=8.1Hz, 2H), 6.60(d, J=8.1Hz, 2H), 6.57(t, J=8.1Hz, 1H), 6.36(d, J=8.1Hz, 1H), 6.01(t, J=5.4Hz, NH), 3.91(d, J=5.4Hz, 2H), 2.69(d, J=5.4Hz, 2H), 1.95(s, 3H), 1.82(m.1H), 0.83(d, J=5.4Hz, 6H). |
| k-5:H-2 | δ13.71(s, 1H), 10.66(brs, 1H), 9.66(brs, 1H), 7.23(d, J=8.1Hz, 1H), 7.01(d, J=8.1Hz, 1H), 6.99(t, J=8.1Hz, 1H), 6.55(t, J=8.1Hz, 1H), 6.44(d, J=8.1Hz, 1H), 6.37(d, J=8.1Hz, 1H), 5.37(t, J=8.1Hz, NH), 3.97(d, J=5.4Hz, 2H), 2.68(d, J=5.4Hz, 2H), 2.16(s, 3H), 1.96(s, 3H), 1.79(m.1H), 0.81(d, J=5.4Hz, 6H). |
| k-5:H-3 | δ13.71(s, 1H), 10.66(brs, 1H), 9.65(brs, 1H), 7.23(d, J=8.1Hz, 1H), 6.95(t, J=8.1Hz, 1H), 6.45-6.30(m, 4H), 5.92(t, J=8.1Hz, NH), 3.90(d, J=8.1Hz, 2H), 2.69(d, J=5.4Hz, 2H), 2.16(s, 3H), 1.95(s, 3H), 1.82(m, 1H), 0.83(d, J=5.4Hz, 6H). |
| k-5:H-4 | δ13.71(s, 1H), 10.65(brs, 1H), 9.66(brs, 1H), 7.24(d, J=8.1Hz, 1H), 6.91(d, J=8.1Hz, 2H), 6.53(d, J=8.1Hz, 2H), 6.37(d, J=8.1Hz, 1H), 5.82(t, J=8.1Hz, NH), 3.89(d, J=8.1Hz, 2H), 2.69(d, J=5.4Hz, 2H), 2.14(s, 3H), 1.96(s, 3H), 1.82(m, 1H), 0.84(d, J=5.4Hz, 6H). |
| k-5:H-5 | δ13.71(s, 1H), 10.69(brs, 1H), 9.66(brs, 1H), 7.23(d, J=8.1Hz, 1H), 7.00-6.85(m, 2H), 6.65-6.55(m, 2H), 6.37(d, J=8.1Hz, 1H), 5.98(t, J=8.1Hz, NH), 3.89(d, J=8.1Hz, 2H), 2.70(d, J=8.1Hz, 2H), 1.95(s, 3H), 1.82(m, 1H), 0.84(d, J=5.4Hz, 6H). |
| k-5:H-6 | δ13.71(s, 1H), 10.75(brs, 1H), 9.65(brs, 1H), 8.17(d, J=8.1Hz, 1H), 7.78(d, J=8.1Hz, 1H), 7.50-7.40(m, 2H), 7.30-7.10(m, 3H), 6.69(t, J=8.1Hz, NH), 6.43(d, J=5.4Hz, 1H), 6.36(d, J=8.1Hz, 1H), 4.13(d, J=8.1Hz, 2H), 2.66(d, J=8.1Hz, 2H), 1.95(s, 3H), 1.76(m, 1H), 0.74(d, J=8.1Hz, 6H). |
| k-5:H-7 | δ 13.72(s, 1H), 10.75(brs, 1H), 9.66(brs, 1H), 7.23(d, J=8.1Hz, 1H), 7.05(t, J=8.1Hz, 2H), 6.65(d, J=8.1Hz, 2H), 6.55(t, J=8.1Hz, 1H), 6.37(d, J=8.1Hz, 1H), 5.87(d, J=8.1Hz, NH), 4.09(m, 1H), 2.75(d, J=5.4Hz, 2H), 1.95(s, 3H), 1.90-1.70 (m, 3H), 0.99(t, J=8.1Hz, 3H), 0.89(d, J=5.4Hz, 3H), 0.83(d, J=5.4Hz, 3H). |
| k-5:H-9* | δ13.71(s, 1H), 10.62(brs, 1H), 9.66(brs, 1H), 7.22(d, J=8.1Hz, 1H), 6.71(d, J=8.1Hz, 2H), 6.55(d, J=8.1Hz, 2H), 6.36(d, J=8.1Hz, 1H), 5.65(t, J=8.1Hz, NH), 3.88(q, J=8.1Hz, 2H), 3.85(d, J=8.1Hz, 2H), 2.67(d, J=8.1Hz, 2H), 1.95(s, 3H), 1.82(m, 1H), 1.24(t, J=8.1Hz, 3H), 0.82(d, J=5.4Hz, 6H). |

| | |
|---|---|
| *Reference Example | |

The synthesis methods of the compounds to be used in the present invention and synthesis intermediate compounds are described below.

In the 4 kinds of ketones, k-1, k-2 and k-5 can be synthesized by a known method (Sum TH et al., Tetrahedron. 2015 Jul 1; 71(26-27): 4557-4564.). In the 10 kinds of hydrazides, H-1, H-2, H-3, H-4, H-5, H-6, H-9, D-2 and D-4 can be synthesized by a known method (Samal RP et al., Chem Biol Drug Des. 2013 Jun; 81(6) :715-29. etc.). Therefore, the synthesis methods of k-3 and H-7 are described in detail below.

### [Synthesis of k-3]

2,4,6-Trihydroxybenzaldehyde (2.22 g, 14.4 mmol) was dissolved in THF (40 mL), NaBH₃CN (2.7 g, 43 mmol) and acetic acid (8 mL) were added under ice-cooling and the mixture was stirred at room temperature for 2 hr. The reaction solution was diluted with ethyl acetate (50 mL), and washed successively with water (50 mLx2), saturated aqueous sodium hydrogen carbonate solution (50 mL), and brine (50 mL). The mixture was dried over anhydrous sodium sulfate, filtered, and concentrated under reduced pressure and the obtained residue was purified by moderate-pressure silica gel column chromatography (silica gel 50 g, ethyl acetate/hexane=20/80 - 50/50) to give an intermediate compound (1.20 g, 8.56 mmol, yield 59%) as a white solid.

The intermediate compound (1.04 g, 7.42 mmol) obtained as mentioned above was suspended in propionic acid (7 mL), propionic acid anhydride (1.15 mL, 8.90 mmol) and BF₃-Et₂O (1.12 mL, 8.90 mmol) were added and the mixture was heated under reflux at 130°C for 1 hr. The mixture was allowed to cool, and the reaction solution was diluted with ethyl acetate (100 mL), and washed successively with water (100 mLx3), saturated aqueous sodium hydrogen carbonate solution (100 mLx2), and brine (100 mL). The mixture was dried over anhydrous sodium sulfate, filtered, and concentrated under reduced pressure and the obtained residue was purified by moderate-pressure silica gel column chromatography (silica gel 100 g, ethyl acetate/hexane=10/90 - 45/55). The obtained solid was washed with hexane to give k-3 (0.41 g) as a light orange solid. The filtrate was concentrated under reduced pressure and the obtained residue was purified again by moderate-pressure silica gel column chromatography (silica gel 30 g, ethyl acetate/hexane=10/90 - 50/50) to give k-3 (0.70 g) as a light orange solid. In total, k-3 (1.11 g, 5.66 mmol, yield 76%) was obtained as a light orange solid.

### [Synthesis of H-7]

Methyl 2-bromobutyrate (8.0 g, 44 mmol) and aniline (8.0 mL, 88 mmol) were dissolved in toluene (10 mL), and the mixture was heated under reflux for 5 hr. The mixture was allowed to cool, and the reaction solution was washed successively with water (30 mL), 2 M hydrochloric acid (25 mL), water (30 mL), saturated aqueous sodium hydrogen carbonate solution (30 mL), and brine (30 mL), and dried over anhydrous sodium sulfate.

The mixture was filtered, and concentrated under reduced pressure and the obtained residue was purified by moderate-pressure silica gel column chromatography (silica gel 100 g, ethyl acetate/hexane=1/99 - 10/90) to give an intermediate compound (5.11 g, 26.4 mmol, yield 60%) as a yellow liquid.

The intermediate compound (5.11 g, 26.4 mmol) obtained as mentioned above was dissolved in methanol (26 mL), hydrazine monohydrate (12.8 mL, 264 mmol) was added and the mixture was stirred at room temperature for 4.5 hr. Water (150 mL) was added and the mixture was extracted with methylene chloride (30 mLx5). The organic layer was washed with saturated brine (100 mL), dried over anhydrous magnesium sulfate, filtered, and concentrated under reduced pressure. The obtained solid was washed with IPE to give H-7 (4.60 g, 23.8 mmol, yield 90%) as a white solid.

### [Synthesis of k-1:H-1]

k-1 (100 mg, 0.555 mmol), H-1 (110 mg, 0.666 mmol) were dissolved in DMSO (1.1 mL), and the mixture was stirred at 100°C for 14 hr. The mixture was allowed to cool, distilled water (11 mL) was added, and the mixture was stirred again at 100°C and filtered while hot to give k-1:H-1 (70.1 mg, 0.214 mmol, yield 39%) as a light yellow solid.

### [Synthesis of k-1:H-2]

k-1 (50 mg, 0.28 mmol), H-2 (69 mg, 0.33 mmol) were dissolved in DMSO (0.55 mL), and the mixture was stirred at 100°C for 18 hr. The mixture was allowed to cool, distilled water (6 mL) was added, decantated, and the remaining solid was washed successively with methylene chloride, and ethyl acetate. The obtained residue was dissolved in DMSO (0.2 mL), water was added and the precipitated solid was washed with methanol to give k-1:H-2 (19.6 mg, 0.0574 mmol, yield 21%) as a light orange solid.

### [Synthesis of k-1:H-3]

k-1 (100 mg, 0.555 mmol), H-3 (119 mg, 0.666 mmol) were dissolved in DMSO (1.1 mL), and the mixture was stirred at 100°C for 14 hr. Distilled water (11 mL) was added at the same temperature and the mixture was allowed to cool. The precipitated solid was collected by filtration, and washed with methanol to give k-1:H-3 (98.9 mg, 0.290 mmol, yield 52%) as a white solid.

### [Synthesis of k-1:H-4] (Reference Example)

k-1 (50 mg, 0.28 mmol), H-4 (65 mg, 0.36 mmol) were dissolved in DMSO (0.55 mL), and the mixture was stirred at 100°C for 19 hr. The mixture was allowed to cool, distilled water (6 mL) was added and the precipitated solid was collected by filtration, and washed with ethyl acetate to give k-1:H-4 (28.6 mg, 0.0838 mmol, yield 30%) as a light yellow solid.

### [Synthesis of k-1:H-5]

k-1 (100 mg, 0.555 mmol), H-5 (122 mg, 0.666 mmol) were dissolved in DMSO (1.1 mL), and the mixture was stirred at 100°C for 14 hr. Distilled water (11 mL) was added at the same temperature and the mixture was allowed to cool, and the precipitated solid was collected by filtration, and washed with methanol to give k-1:H-5 (55.6 mg, 0.161 mmol, yield 29%) as a light yellow solid.

### [Synthesis of k-1:H-6]

k-1 (100 mg, 0.555 mmol), H-6 (143 mg, 0.666 mmol) were dissolved in DMSO (1.1 mL), and the mixture was stirred at 100°C for 14 hr. The mixture was allowed to cool, distilled water (11 mL) was added and the precipitated solid was collected by filtration and washed with methylene chloride to give k-1:H-6 (86.5 mg, 0.229 mmol, yield 41%) as a brown solid.

### [Synthesis of k-1:H-7]

k-1 (50 mg, 0.28 mmol), H-7 (54 mg, 0.28 mmol) were dissolved in DMSO (0.55 mL), and the mixture was stirred at 100°C for 17 hr. The mixture was allowed to cool, distilled water (6 mL) was added and the mixture was decantated, and the residue was dissolved in methylene chloride (0.5 mL). Hexane (0.5 mL) was added and the precipitated solid was collected by filtration to give k-1:H-7 (56.8 mg, 0.160 mmol, yield 57%) as a white solid.

### [Synthesis of k-1:H-9] (Reference Example)

k-1 (150 mg, 0.83 mmol), H-9 (226 mg, 1.08 mmol) was suspended in DMSO (0.55 mL) and the mixture was stirred at 100°C for 17 hr. The mixture was allowed to cool, distilled water (20 mL) was added and the mixture was decantated. The obtained residue was purified by moderate-pressure silica gel column chromatography (silica gel 10 g, ethyl acetate/hexane=10/90 - 60/40). The obtained solid was washed with methylene chloride to give k-1:H-9 (40.2 mg, 0.108 mmol, yield 13%) as a white solid.

### [Synthesis of k-2:H-1]

k-2 (80 mg, 0.48 mmol), H-1 (95.4 mg, 0.578 mmol) were dissolved in DMSO (1.0 mL), and the mixture was stirred at 100°C for 15 hr. The mixture was allowed to cool, distilled water (10 mL) was added and the precipitated solid was collected by filtration and washed with methylene chloride to give k-2:H-1 (80.4 mg, 0.257 mmol, yield 53%) as a yellow solid.

### [Synthesis of k-2:H-2]

k-2 (80 mg, 0.48 mmol), H-2 (112 mg, 0.625 mmol) were dissolved in DMSO (1 mL), and the mixture was stirred at 100°C for 19 hr. The mixture was allowed to cool, distilled water (30 mL), ethyl acetate (30 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 80/20) to give k-2:H-2 (34 mg, 0.10 mmol, yield 21%) as a light yellow solid.

### [Synthesis of k-2:H-3]

k-2 (80 mg, 0.48 mmol), H-3 (104 mg, 0.578 mmol) were dissolved in DMSO (1 mL), and the mixture was stirred at 100°C for 15 hr. The mixture was allowed to cool, distilled water (10 mL) was added and the mixture was decantated. The obtained residue was dissolved in methylene chloride (3 mL), hexane (3 mL) was added and the precipitated solid was collected by filtration. The obtained solid was purified by moderate-pressure silica gel column chromatography (silica gel 10 g, ethyl acetate/hexane=50/50 - 80/20) to give k-2:H-3 (46.9 mg, 0.143 mmol, yield 30%) as a light yellow solid.

### [Synthesis of k-2:H-4]

k-2 (80 mg, 0.48 mmol), H-4 (112 mg, 0.625 mmol) were dissolved in DMSO (1 mL), and the mixture was stirred at 100°C for 19 hr. The mixture was allowed to cool, distilled water (10 mL) was added, the mixture was decantated, and the residue was washed with methylene chloride to give k-2:H-4 (58.7 mg, 0.179 mmol, yield 37%) as a light yellow solid.

### [Synthesis of k-2:H-5]

k-2 (80 mg, 0.48 mmol), H-5 (106 mg, 0.578 mmol) were dissolved in DMSO (1 mL), and the mixture was stirred at 100°C for 15 hr. The mixture was allowed to cool, distilled water (10 mL) was added and the mixture was decantated. The obtained residue was dissolved in methylene chloride (3 mL), hexane (1 mL) was added and the precipitated solid was collected by filtration. The obtained solid was purified by moderate-pressure silica gel column chromatography (silica gel 10 g, ethyl acetate/hexane=50/50 - 80/20) to give k-2:H-5 (36.6 mg, 0.110 mmol, yield 23%) as a white solid.

### [Synthesis of k-2:H-6]

k-2 (100 mg, 0.602 mmol), H-6 (155 mg, 0.722 mmol) were dissolved in DMSO (1 mL), and the mixture was stirred at 100°C for 15 hr. The mixture was allowed to cool, distilled water (10 mL) was added and the mixture was decantated. The obtained residue was washed with methylene chloride to give k-2:H-6 (59.3 mg, 0.163 mmol, yield 27%) as a light brown solid.

### [Synthesis of k-2:H-7]

k-2 (80 mg, 0.48 mmol), H-7 (121 mg, 0.626 mmol) were dissolved in DMSO (0.55 mL), and the mixture was stirred at 100°C for 17 hr. The mixture was allowed to cool, distilled water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=5/95 - 50/50) to give k-2:H-7 (109 mg, 0.319 mmol, yield 66%) as a light yellow solid.

### [Synthesis of k-2:H-9]

k-2 (100 mg, 0.60 mmol), H-9 (164 mg, 0.784 mmol) were suspended in DMSO (1.2 mL) and the mixture was stirred at 100°C for 18 hr. The mixture was allowed to cool, distilled water (12 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 60/40), and the obtained solid was washed with methylene chloride to give k-2:H-9 (63.1 mg, 0.177 mmol, yield 30%) as a light yellow solid.

### [Synthesis of k-3:H-1]

k-3 (200 mg, 1.02 mmol), H-1 (253 mg, 1.53 mmol) were dissolved in DMSO (2.0 mL) and the mixture was stirred at 100°C for 3 days. The mixture was allowed to cool, distilled water (15 mL) was added and the mixture was decantated. The obtained residue was purified by moderate-pressure silica gel column chromatography (silica gel 30 g, ethyl acetate/hexane=40/60 - 70/30). The obtained solid was washed with methylene chloride to give k-3:H-1 (25.0 mg, 0.0728 mmol, yield 7.1%) as a light brown solid.

### [Synthesis of k-3:H-2]

k-3 (200 mg, 1.02 mmol), H-2 (237 mg, 1.33 mmol) were dissolved in DMSO (2 mL) and the mixture was stirred at 100°C for 4 days. The mixture was allowed to cool, distilled water (15 mL) was added and the precipitated solid was collected by filtration and washed with methanol. The obtained solid was purified by moderate-pressure silica gel column chromatography (silica gel 30 g, ethyl acetate/hexane=40/60 - 70/30). The obtained solid was washed with methylene chloride to give k-3:H-2 (10.3 mg, 0.0288 mmol, yield 2.8%) as a light brown solid.

### [Synthesis of k-3:H-3]

k-3 (200 mg, 1.02 mmol), H-3 (219 mg, 1.22 mmol) were dissolved in DMSO (2 mL) and the mixture was stirred at 100°C for 5 days. The mixture was allowed to cool, distilled water (15 mL) was added and the precipitated solid was collected by filtration and purified by moderate-pressure silica gel column chromatography (silica gel 30 g, ethyl acetate/hexane=40/60 - 70/30). The obtained solid was washed with methylene chloride to give k-3:H-3 (17.9 mg, 0.0501 mmol, yield 4.9%) as a light brown solid.

### [Synthesis of k-3:H-4]

k-3 (200 mg, 1.02 mmol), H-4 (237 mg, 1.33 mmol) were dissolved in DMSO (2 mL) and the mixture was stirred at 100°C for 4 days. The mixture was allowed to cool, distilled water (15 mL) was added and the precipitated solid was collected by filtration and purified by moderate-pressure silica gel column chromatography (silica gel 30 g, ethyl acetate/hexane=40/60 - 70/30). The obtained solid was washed with methylene chloride to give k-3:H-4 (17.9 mg, 0.0501 mmol, yield 4.9%) as a light brown solid.

### [Synthesis of k-3:H-5]

k-3 (200 mg, 1.02 mmol), H-5 (224 mg, 1.22 mmol) were dissolved in DMSO (2 mL) and the mixture was stirred at 100°C for 6 days. The mixture was allowed to cool, distilled water (15 mL) was added and the mixture was decantated. The obtained residue was dissolved in methylene chloride (3 mL), hexane (1 mL) was added and the precipitated solid was collected by filtration and purified by moderate-pressure silica gel column chromatography (silica gel 30 g, ethyl acetate/hexane=40/60 - 70/30). The obtained solid was washed with methylene chloride to give k-3:H-5 (13.7 mg, 0.0379 mmol, yield 3.7%) as a light brown solid.

### [Synthesis of k-3:H-6]

k-3 (100 mg, 0.510 mmol), H-6 (121 mg, 0.561 mmol) were dissolved in DMSO (2 mL) and the mixture was stirred at 100°C for 4 days. The mixture was allowed to cool, distilled water (15 mL) was added and the mixture was decantated. The obtained residue was purified by moderate-pressure silica gel column chromatography (silica gel 30 g, ethyl acetate/hexane=40/60 - 70/30). The obtained solid was washed with methylene chloride to give k-3:H-6 (17.0 mg, 0.0432 mmol, yield 8.5%) as an orange solid.

### [Synthesis of k-3:H-7]

k-3 (200 mg, 1.02 mmol), H-7 (256 mg, 1.33 mmol) were dissolved in DMSO (2 mL) and the mixture was stirred at 100°C for 3 days. The mixture was allowed to cool, distilled water (15 mL) was added and the mixture was decantated. The obtained residue was purified by moderate-pressure silica gel column chromatography (silica gel 30 g, ethyl acetate/hexane=40/60 - 70/30). The obtained solid was washed with methylene chloride to give k-3:H-7 (28.3 mg, 0.762 mmol, yield 7.5%) as a white solid.

### [Synthesis of k-3:H-9]

k-3 (200 mg, 1.02 mmol), H-9 (277 mg, 1.33 mmol) were dissolved in DMSO (2 mL) and the mixture was stirred at 100°C for 4 days. The mixture was allowed to cool, distilled water (15 mL) was added and the precipitated solid was filtered and purified by moderate-pressure silica gel column chromatography (silica gel 30 g, ethyl acetate/hexane=40/60 - 70/30). The obtained solid was washed with methylene chloride to give k-3:H-9 (11.2 mg, 0.0289 mmol, yield 2.8%) as a light brown solid.

### [Synthesis of k-5:H-1]

k-5 (100 mg, 0.48 mmol), H-1 (95.2 mg, 0.576 mmol) were dissolved in DMSO (1 mL) and the mixture was stirred at 100°C for 4 days. The mixture was allowed to cool, distilled water (10 mL) was added and the precipitated solid was collected by filtration and washed successively with methylene chloride and methanol to give k-5:H-1 (38.1 mg, 0.107 mmol, yield 22%) as a yellow solid.

### [Synthesis of k-5:H-2]

k-5 (100 mg, 0.48 mmol), H-2 (112 mg, 0.625 mmol) were dissolved in DMSO (1 mL) and the mixture was stirred at 100°C for 3 days. Distilled water (10 mL) was added and the mixture was decantated, and the residue was dissolved in methylene chloride (2 mL), dried over salt cake, filtered, and concentrated under reduced pressure. Methylene chloride (1 mL) was added to the obtained residue, and the mixture was subjected to ultrasonication and the precipitated solid was collected by filtration to give k-5:H-2 (18.6 mg, 0.0503 mmol, yield 10%) as a yellow solid.

### [Synthesis of k-5:H-3]

k-5 (100 mg, 0.480 mmol), H-3 (103 mg, 0.576 mmol) were dissolved in DMSO (1 mL) and the mixture was stirred at 100°C for 4 days. The mixture was allowed to cool, distilled water (10 mL) was added and the precipitated solid was collected by filtration and washed with methylene chloride to give k-5:H-3 (44.6 mg, 0.121 mmol, yield 25%) as a yellow solid.

### [Synthesis of k-5:H-4]

k-5 (100 mg, 0.480 mmol), H-4 (112 mg, 0.625 mmol) were dissolved in DMSO (1 mL) and the mixture was stirred at 100°C for 3 days. Distilled water (10 mL) was added and the precipitated solid was collected by filtration and washed with methylene chloride to give k-5:H-4 (44.4 mg, 0.120 mmol, yield 25%) as a yellow solid.

### [Synthesis of k-5:H-5]

k-5 (100 mg, 0.480 mmol), H-5 (106 mg, 0.576 mmol) were dissolved in DMSO (2 mL) and the mixture was stirred at 100°C for 4 days. The mixture was allowed to cool, distilled water (10 mL) was added and the precipitated solid was collected by filtration to give k-5:H-5 (37.4 mg, 0.100 mmol, yield 21%) as a yellow solid.

### [Synthesis of k-5:H-6]

k-5 (100 mg, 0.480 mmol), H-6 (124 mg, 0.576 mmol) were dissolved in DMSO (1 mL) and the mixture was stirred at 100°C for 5 days. The mixture was allowed to cool, distilled water (10 mL) was added and the precipitated solid was collected by filtration and purified by moderate-pressure silica gel column chromatography (silica gel 10 g, ethyl acetate/hexane=20/80 - 50/50). The obtained solid was washed with methanol to give k-5:H-6 (28.1 mg, 0.0693 mmol, yield 14%) as a light yellow solid.

### [Synthesis of k-5:H-7]

k-5 (100 mg, 0.480 mmol), H-7 (121 mg, 0.626 mmol) were dissolved in DMSO (1 mL) and the mixture was stirred at 100°C for 4 days. The mixture was allowed to cool, distilled water (10 mL) was added and the precipitated solid was collected by filtration and washed successively with ethyl acetate, methylene chloride, and methanol to give k-5:H-7 (43.6 mg, 0.114 mmol, yield 24%) as a white solid.

### [Synthesis of k-5:H-9] (Reference Example)

k-5 (150 mg, 0.72 mmol), H-9 (196 mg, 0.937 mmol) were suspended in DMSO (1.4 mL) at 100°C for 3 days. The mixture was allowed to cool, distilled water (12 mL), methylene chloride (20 mL) were added and the mixture was partitioned. The organic layer was washed successively with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 50/50) to give k-5:H-9 (72.0 mg, 0.180 mmol, yield 25%) as a light yellow solid.

In addition, compound Nos. k-1:D-2 and k-1:D-4 can also be synthesized by methods according to the above-mentioned synthesis methods.

### [Synthetic Example 2] Synthesis of k-1:A-1

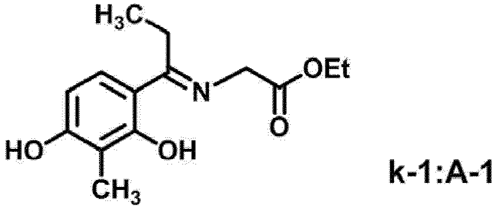

k-1 (100 mg, 0.55 mmol), glycine ethyl ester hydrochloride (A-1) (100 mg, 0.72 mmol), sodium acetate (64 mg, 0.78 mmol) were dissolved in DMSO (1.1 mL), and the mixture was stirred at 100°C for 2 hr. The mixture was allowed to cool, water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 50/50). The obtained solid was washed with IPE to give k-1:A-1 (21.9 mg, 0.0825 mmol, yield 15%) as a yellow solid.

¹H-NMR (270 MHz); 59.69(s, 1H), 7.32(d, J=8.1Hz, 1H), 6.30(d, J=10.8Hz, 1H), 5.76(s, 1H), 4.48(s, 2H), 4.19(q, J=8.1Hz, 2H), 2.67(q, J=8.1Hz, 2H), 1.94(s, 3H), 1.24(t, J=8.1Hz, 3H), 1.08(t, J=8.1Hz, 3H).

### [Synthetic Example 3] Synthesis of k-1:A-2

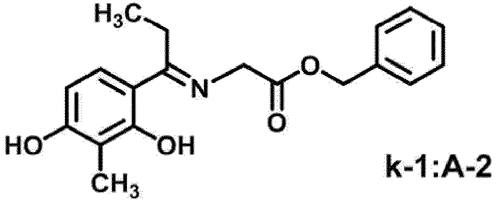

k-1 (100 mg, 0.55 mmol), glycine benzyl ester p-toluenesulfonate (A-2) (243 mg, 0.721 mmol), sodium acetate (64 mg, 0.78 mmol) were dissolved in DMSO (1.1 mL), and the mixture was stirred at 100°C for 2 hr. The mixture was allowed to cool, water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=5/95 - 50/50). The obtained solid was washed with IPE to give k-1:A-2 (25.0 mg, 0.0764 mmol, yield 14%) as a yellow solid.

¹H-NMR (270 MHz); δ9.69(s, 1H), 7.45-7.35 (m, 6H), 7.32(d, J=10.8Hz, 1H), 6.31(d, J=10.8Hz, 1H), 5.23(s, 2H), 4.56(s, 2H), 2.70 (q, J=8.1Hz, 2H), 1.94 (s, 3H), 1.08(t, J=8.1Hz, 3H).

### [Synthetic Example 4] Synthesis of k-1:A-3

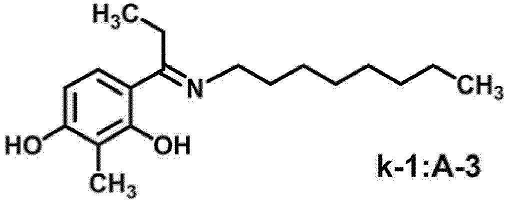

k-1 (100 mg, 0.55 mmol) was dissolved in DMSO (1.1 mL), n-octylamine (A-3) (120 mL, 0.72 mmol) was added and the mixture was stirred at room temperature for 2 hr and at 100°C for 17 hr. The mixture was allowed to cool, water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=0/100 - 20/80). The obtained solid was washed with IPE to give k-1:A-3 (60.1 mg, 0.206 mmol, yield 37%) as a yellow solid.

¹H-NMR (270 MHz); δ9.52(s, 1H), 7.25(d, J=8.1Hz, 1H), 6.20(d, J=8.1Hz, 1H), 5.31 (t, J=8.1Hz, 2H), 2.73(q, J=8.1Hz, 2H), 1.90(s, 3H), 1.64(m, 2H), 1.30-1.05(m, 10H), 1.11(t, J=8.1Hz, 3H), 0.86(t, J=8.1Hz, 3H).

### [Synthetic Example 5] Synthesis of k-1:A-5

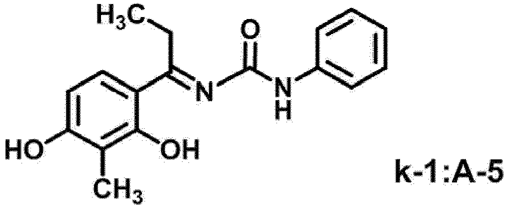

k-1 (300 mg, 1.7 mmol) was dissolved in 2 M ammonia/methanol solution (17 mL, 33 mmol), and the mixture was stirred at room temperature for 1 week. During that time, ammonia gas was bubbled every day for 15 min. The reaction solution was concentrated under reduced pressure to give a mixture of 4-(1-iminopropyl)-2-methylbenzene-1,3-diol (k-1') and k-1 (292 mg, k-1':k-1=1:0.2) as a yellow ocher solid. A mixture (292 mg) of k-1' and k-1 obtained as mentioned above was dissolved in THF (6.5 mL), and phenylisocyanate (A-5) (158 mL, 1.46 mmol) was added under ice-cooling. After stirring for 30 min at the same temperature, water (30 mL), ethyl acetate (30 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel column chromatography (silica gel 10 g, ethyl acetate/hexane=5/95 - 45/55). The obtained solid was washed with IPE to give k-1:A-5 (160 mg, 0.536 mmol, 2 step yield 32%) as a light yellow solid.

¹H-NMR (270 MHz); δ13.74(s, 1H), 10.22(s, 1H), 10.11(s, 1H), 7.63(d, J=8.1Hz, 2H), 7.53 (d, J=8.1Hz, 1H), 7.32 (t, J=8.1Hz, 2H), 7.06 (t, J=8.1Hz, 1H), 6.49 (d, J=8.1Hz, 1H), 2.84(q, J=8.1Hz, 2H), 2.00(s, 3H), 1.24(t, J=8.1Hz, 3H).

### [Synthetic Example 6] Synthesis of k-1:H-10

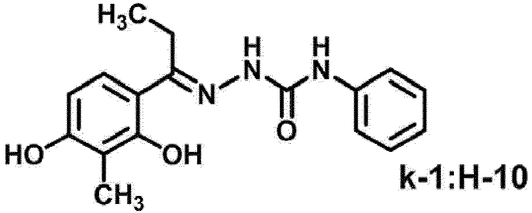

k-1 (100 mg, 0.55 mmol), 4-phenylsemicarbazide (H-10) (109 mg, 0.721 mmol) were dissolved in DMSO (1.1 mL), and the mixture was stirred at 100°C for 3.5 hr. The mixture was allowed to cool, water (20 mL), ethyl acetate (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 10 g, ethyl acetate/hexane=10/90 - 50/50). The obtained solid was washed with IPE to give k-1:H-10 (19.1 mg, 0.0610 mmol, yield 11%) as a white solid.

¹H-NMR (270 MHz):δ13.50(s, 1H), 9.75(s, 1H), 9.59(s, 1H), 8.82(s, 1H), 7.49(d, J=8.1Hz, 2H), 7.30(t, J=8.1Hz, 2H), 7.21(d, J=8.0Hz, 1H), 6.99(t, J=8.1Hz, 1H), 6.39(d, J=8.1Hz, 1H), 2.70(q, J=8.1Hz, 2H), 1.99(s, 3H), 1.14(t, J=8.1Hz, 3H).

### [Synthetic Example 7] Synthesis of k-1:I-1

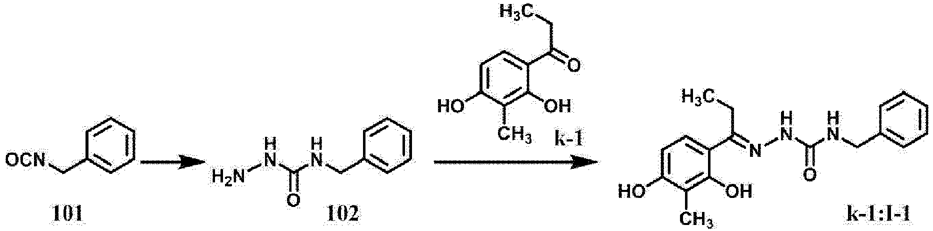

Hydrazine monohydrate (0.26 mL, 5.3 mmol) was dissolved in methylene chloride (5.3 mL), and benzyl isocyanate (101) (0.324 mL, 2.63 mmol) was slowly added under ice-cooling. The mixture was stirred at room temperature for 3 hr, and the precipitated solid was washed with IPE, and dried under reduced pressure to give 102 (352 mg, 2.13 mmol, yield 82%) as a white solid.

102 (155 mg, 0.938 mmol) obtained as mentioned above and k-1 (130 mg, 0.72 mmol) were dissolved in DMSO (1.4 mL), and the mixture was stirred at 100°C for 3.5 hr. The mixture was allowed to cool, distilled water (10 mL) was added, and the precipitated solid was collected by filtration, and purified by moderate-pressure silica gel column chromatography (silica gel 10 g, ethyl acetate/hexane=10/90 - 55/45). The obtained solid was washed with methylene chloride, and dried under reduced pressure to give k-1:I-1 (55 mg, 0.17 mmol, yield 24%) as a white solid.

¹H-NMR (400 MHz); δ9.59(s, 1H), 9.56(s, 1H), 7.40-7.25(m, 5H), 7.17(d, J=12.0Hz, 1H), 6.84(t, J=8.0Hz, NH), 6.37(d, J=12.0Hz, 1H), 4.34(d, J=8.0Hz, 2H), 2.65(q, J=8.0Hz, 2H), 1.97(s, 3H), 1.08(t, J=8.0Hz, 3H).(one signal of NH was not observed)

### [Synthetic Example 8] Synthesis of k-1:I-3

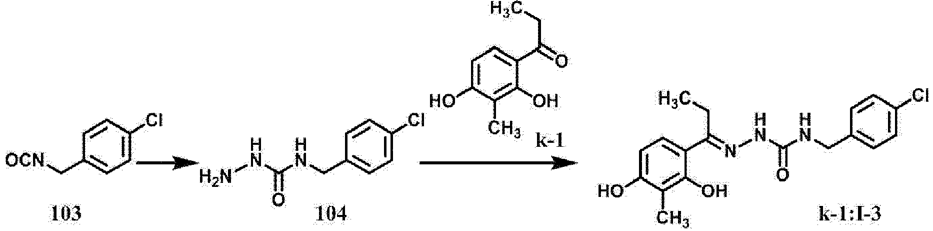

Hydrazine monohydrate (0.20 mL, 4.2 mmol) was dissolved in methylene chloride (4.2 mL), and 4-chlorobenzyl isocyanate (103) (0.278 mL, 2.09 mmol) was slowly added under ice-cooling. The mixture was stirred at room temperature for 1.5 hr, and the precipitated solid was collected by filtration and dried under reduced pressure to give 104 (315 mg, 1.58 mmol, yield 75%) as a white solid.

104 (187 mg, 0.937 mmol) obtained as mentioned above, k-1 (130 mg, 0.72 mmol) were dissolved in DMSO (1.4 mL), and the mixture was stirred at 100°C for 22 hr. The reaction solution was directly purified by moderate-pressure silica gel column chromatography (silica gel 10 g, ethyl acetate/hexane=10/90 - 65/35), the obtained purified product was dissolved in ethyl acetate, hexane was added and the precipitated solid was collected by filtration, and dried under reduced pressure to give k-1:I-3 (155 mg, 0.428 mmol, yield 59%) as a white solid.

¹H-NMR (400 MHz); δ9.63(s, 1H), 9.56(s, 1H), 7.41(d, J=8.0Hz, 2H), 7.34(d, J=8.0Hz, 2H), 7.17(d, J=8.0Hz, 1H), 6.89(t, J=8.0Hz, NH), 6.36(d, J=8.0Hz, 1H), 4.31(d, J=8.0Hz, 2H), 2.65(q, J=8.0Hz, 2H), 1.97(s, 3H), 1.08(t, J=8.0Hz, 3H).(one signal of NH was not observed)

### [Synthetic Example 9] (Reference Example) Synthesis of k-1:I-6

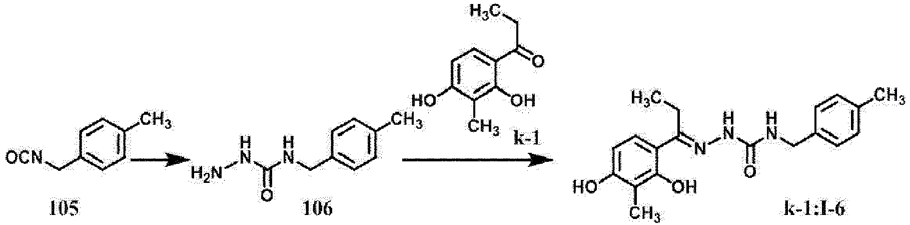

Hydrazine monohydrate (0.23 mL, 4.8 mmol) was dissolved in methylene chloride (8 mL), and 4-methylbenzyl isocyanate(105) (350 mg, 2.4 mmol) was slowly added under ice-cooling. The mixture was stirred at room temperature for 2 hr, and the precipitated solid was collected by filtration and dried under reduced pressure to give 106 (297 mg, 1.66 mmol, yield 69%) as a white solid.

106 (168 mg, 0.937 mmol) obtained as mentioned above, k-1 (130 mg, 0.72 mmol) were suspended in DMSO (2.8 mL) and the mixture was stirred at 100°C for 22 hr. The reaction solution was directly purified by moderate-pressure silica gel column chromatography (silica gel 10 g, ethyl acetate/hexane=10/90 - 60/40), and the obtained purified product was dissolved in ethyl acetate (30 mL). The mixture was washed successively with water (20 mL), saturated brine (30 mL), dried over anhydrous sodium sulfate, filtered, and concentrated under reduced pressure. The obtained solid was washed with methylene chloride, and dried under reduced pressure to give k-1:I-6 (118 mg, 0.346 mmol, yield 48%) as a white solid.

¹H-NMR (400 MHz); δ9.56(s, 1H), 9.55(s, 1H), 7.20(d, J=8.0Hz, 2H), 7.16(d, J=8.0Hz, 2H), 7.15(d, J=8.0Hz, 1H), 6.78(t, J=8.0Hz, NH), 6.36(d, J=8.0Hz, 1H), 4.29(d, J=8.0Hz, 2H), 2.64(q, J=8.0Hz, 2H), 2.28(s, 3H), 1.97(s, 3H), 1.07(t, J=8.0Hz, 3H).(one signal of NH was not observed)

### [Synthetic Example 10] (Reference Example) Synthesis of k-1:I-7

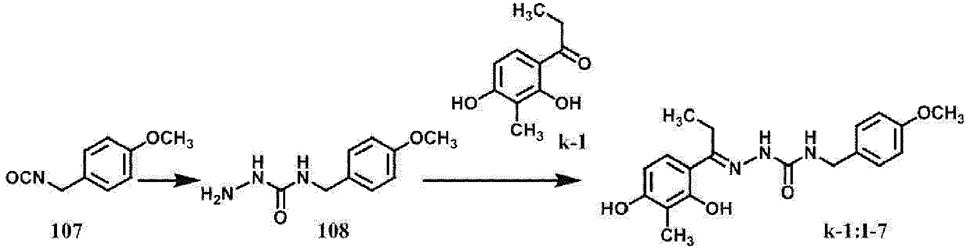

Hydrazine monohydrate (0.21 mL, 4.3 mmol) was dissolved in methylene chloride (4.3 mL), under ice-cooling 4-methoxybenzyl isocyanate (107) (350 mg, 2.15 mmol) was slowly added. The mixture was stirred at room temperature for 2 hr, and the precipitated solid was collected by filtration and dried under reduced pressure to give 108 (381 mg, 1.95 mmol, yield 93%) as a white solid.

108 (183 mg, 0.937 mmol) obtained as mentioned above, k-1 (130 mg, 0.72 mmol) were suspended in DMSO (2.8 mL) and the mixture was stirred at 100°C for 5 hr. The mixture was allowed to cool, distilled water (15 mL) was added, and the precipitated solid was collected by filtration, and purified by moderate-pressure silica gel column chromatography (silica gel 10 g, ethyl acetate/hexane=15/85 - 65/35). The obtained solid was washed with methylene chloride and dried under reduced pressure to give k-1:I-7 (55.0 mg, 0.154 mmol, yield 21%) as a white solid.

¹H-NMR (400 MHz); 59.55(s, 2H), 7.25(d, J=8.0Hz, 2H), 7.16(d, J=8.0Hz, 1H), 6.91(d, J=8.0Hz, 2H), 6.75(t, J=8.0Hz, NH), 6.36(d, J=8.0Hz, 1H), 4.26(d, J=8.0Hz, 2H), 3.73(s, 3H), 2.63(q, J=8.0Hz, 2H), 1.97(s, 3H), 1.07(t, J=8.0Hz, 3H).(one signal of NH was not observed)

The compounds synthesized by methods according to the above-mentioned synthesis methods are shown in the third table.

### [the third table]

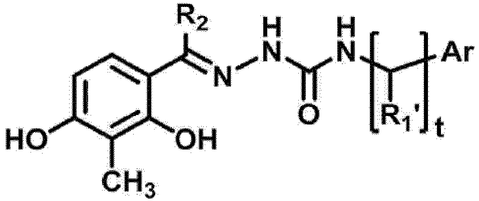

**[Table 3]**

| compound No. | R₂ | R₁' | t | Ar |
|---|---|---|---|---|
| k-1:I-8 | Et | H | 1 | 2-OH-Ph |
| k-1:I-9 | Et | H | 1 | 4-OH-Ph |
| k-1:I-10 | Et | Me | 1 | 3-OH-Ph |
| k-1:I-11 | Et | H | 2 | 3-OH-Ph |
| k-1:I-12 | Et | H | 1 | 3,5-di-OH-Ph |

The measurement results of ¹H-NMR of the compounds described in the third table are shown in the fourth table.

### [the fourth table]

**[Table 4]**

| compound No. | ¹H-NMR (270 MHz) |
|---|---|
| k-1:I-8 | δ13.46(s, 1H), 9.66(s, 1H), 9.63(s, 1H), 9.53(s, 1H), 7.20-7.05(m, 3H), 6.85-6.70(m, 3H), 6.37(d, J=8.1Hz, 1H),.4.26(d, J=5.4Hz, 2H), 2.64(q, J=8.1Hz, 2H), 1.97(s, 3H), 1.07(t, J=8.1Hz, 3H). |
| k-1:I-9 | δ13.47 (s, 1H), 9.53(s, 1H), 9.51(s, 1H), 9.31(s, 1H), 7.16(d, J=8.1Hz, 1H), 7.13(d, J=8.1Hz, 2H), 6.73(d, J=8.1Hz, 2H), 6.71(t, J=8.1Hz, NH), 6.36(d, J=8.1Hz, 1H), 4.20(d, J=8.1Hz, 2H), 2.62(q, J=8.1Hz, 2H), 1.97(s, 3H), 1.06(t, J=8.1Hz, 3H). |
| k-1:I-10 | δ13.50 (s, 1H), 9.51(s, 1H), 9.46(s, 1H), 9.37(s, 1H), 7.16(d, J=8.1Hz, 1H), 7.13(d, J=8.1Hz, 1H), 6.80-6.70(m, 3H), 6.65(d, J=8.1Hz, NH), 6.35(d, J=8.1Hz, 1H), 4.74(m, 1H), 2.63(q, J=8.1Hz, 2H), 1.96(s, 3H), 1.38(d, J=5.4Hz, 3H), 1.06(t, J=8.1Hz, 3H). |
| k-1:I-11 | δ13.48 (s, 1H), 9.53 (s, 2H), 9.31(s, 1H), 7.15(d, J=8.1Hz, 1H), 7.09(d, J=8.1Hz, 1H), 6.70-6.60(m, 3H), 6.38(d, J=8.1Hz, 1H), 6.34(t, J=8.1Hz, NH), 3.40-3.30(m, 2H), 2.69(q, J=8.1Hz, 2H), 2.60-2.50(m, 2H), 1.97(s, 3H), 1.06(t, J=8.1Hz, 3H). |
| k-1:I-12 | δ13.47 (s, 1H), 9.56(s, 1H), 9.54(s, 1H), 9.19(s, 2H), 7.17(d, J=8.1Hz, 1H), 6.70(t, J=8.1Hz, NH), 6.37(d, J=8.1Hz, 1H), 6.17(s, 1H), 6.16(s, 1H), 6.08(s, 1H), 4.15(d, J=5.4Hz, 2H), 2.65(q, J=8.1Hz, 2H), 1.97(s, 3H), 1.08(t, J=8.1Hz, 3H). |

### [Synthetic Example 11] Synthesis of k-1:B-1

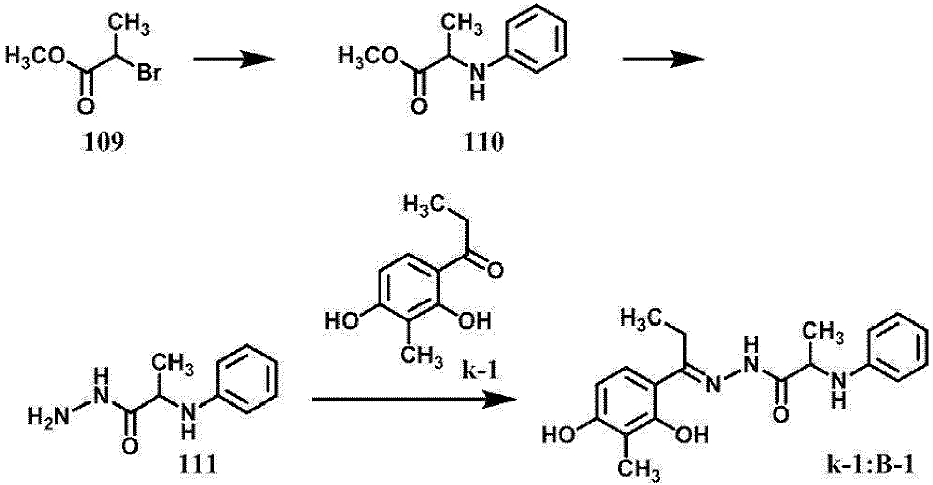

Methyl 2-bromopropionate (109) (500 mg, 3.0 mmol) was dissolved in DMSO (6 mL), aniline (0.36 mL, 3.9 mmol), potassium carbonate (0.54 g, 3.9 mmol) were added and the mixture was stirred at room temperature for 21 hr. Ethyl acetate (30 mL), water (50 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel chromatography (silica gel 30 g, ethyl acetate/hexane=2/98 - 15/85) to give 110 (343 mg, 1.91 mmol, yield 64%) as a light yellow liquid.

110 (340 mg, 1.9 mmol) obtained as mentioned above was dissolved in methanol (3.8 mL), hydrazine monohydrate (0.18 mL, 3.8 mmol) was added, and the mixture was stirred at 60°C for 24 hr. Hydrazine monohydrate (0.36 mL, 7.4 mmol) was added and the mixture was further stirred at 60°C for 17 hr. The reaction solution was concentrated under reduced pressure. The obtained residue was purified by moderate-pressure silica gel chromatography (aminesilica gel 10 g, ethyl acetate/methylene chloride =0/100 - 20/80) to give 111 (321 mg, 1.79 mmol, yield 94%) as a white solid.

111 (168 mg, 0.937 mmol) obtained as mentioned above, k-1 (130 mg, 0.72 mmol) were dissolved in DMSO (1.4 mL), and the mixture was stirred at 100°C for 19 hr. The mixture was allowed to cool, distilled water (15 mL) was added, and the precipitated solid was collected by filtration, and dried. The obtained yellow solid was washed with methylene chloride, and dried under reduced pressure to give k-1:B-1 (173 mg, 0.507 mmol, yield 70%) as a light yellow solid.

¹H-NMR (400 MHz):δ10.82(s, 1H), 9.71(s, 1H), 7.25(d, J=8.0Hz, 1H), 7.07(t, J=8.0Hz, 2H), 6.63(d, J=8.0Hz, 2H), 6.56(t, J=8.0Hz, 1H), 6.39(d, J=8.0Hz, 1H), 5.93(d, J=12Hz, NH), 4.28(m, 1H), 2.80(q, J=8.0Hz, 2H), 1.95(s, 3H), 1.40(d, J=8.0Hz, 3H), 1.03(t, J=8.0Hz, 3H).(one signal of NH was not observed)

### [Synthetic Example 12] Synthesis of k-1:B-2

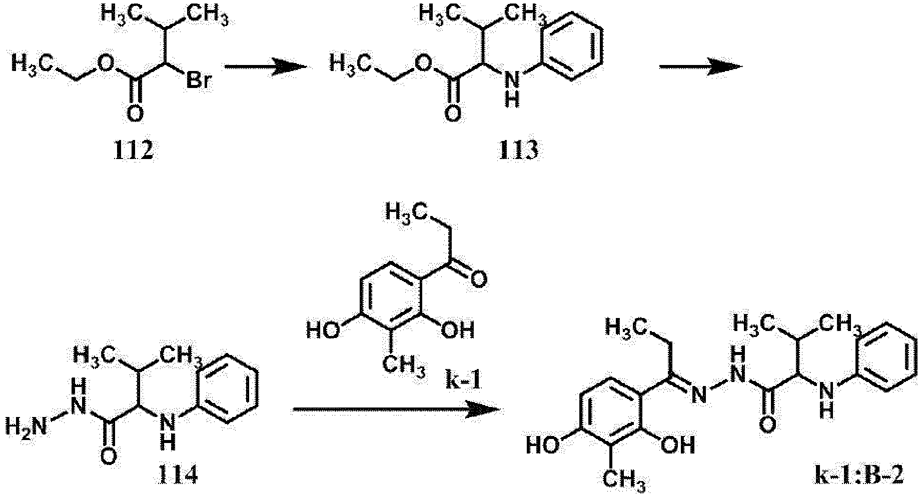

Ethyl 2-bromoisovalerate (112) (700 mg, 3.3 mmol) was dissolved in DMSO (7 mL), aniline (0.40 mL, 4.4 mmol), potassium carbonate (0.60 g, 4.4 mmol) were added, and the mixture was stirred at room temperature for 21 hr, at 80°C for 6 hr, and at 120°C for 20 hr. Ethyl acetate (30 mL), water (50 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel chromatography (silica gel 30 g, ethyl acetate/hexane=0/100 - 5/95) to give 113 (110 mg, 0.497 mmol, yield 15%) as a yellow liquid.

113 (96 mg, 0.43 mmol) obtained as mentioned above was dissolved in methanol (1 mL), hydrazine monohydrate (0.042 mL, 0.87 mmol) was added and the mixture was stirred at 50°C for 4 hr. Hydrazine monohydrate (0.2 mL, 4.1 mmol) was added and the mixture was stirred for 20 hr, hydrazine monohydrate (0.1 mL, 2.1 mmol) was added and the mixture was further stirred for 23 hr. The reaction solution was concentrated under reduced pressure, and the obtained residue was purified by moderate-pressure silica gel chromatography (aminesilica gel 10 g, ethyl acetate/methylene chloride =0/100 - 5/95) to give 114 (80.7 mg, 0.389 mmol, yield 90%) as a white solid.

114 (75 mg, 0.36 mmol) obtained as mentioned above, k-1 (50 mg, 0.28 mmol) were dissolved in DMSO (0.6 mL), and the mixture was stirred at 100°C for 18 hr. The mixture was allowed to cool, and the reaction solution was directly purified by moderate-pressure silica gel column chromatography (silica gel 10 g, ethyl acetate/hexane=10/90 - 75/25). The obtained solid was washed with methylene chloride/IPE, and dried under reduced pressure to give k-1:B-2 (37.6 mg, 0.102 mmol, yield 36%) as a white solid.

¹H-NMR (400 MHz) :δ10.83 (s, 1H), 9.72(s, 1H), 7.26(d, J=8.0Hz, 1H), 7.05(t, J=8.0Hz, 2H), 6.71(d, J=8.0Hz, 2H), 6.53(t, J=8.0Hz, 1H), 6.39(d, J=8.0Hz, 1H), 5.78(d, J=12Hz, NH), 3.97(m, 1H), 2.82(q, J=8.0Hz, 2H), 2.03(m, 1H), 1.95(s, 3H), 1.06(t, J=8.0Hz, 3H), 0.97(d, J=8.0Hz, 6H).(one signal of NH was not observed)

### [Synthetic Example 13] Synthesis of k-1:B-3

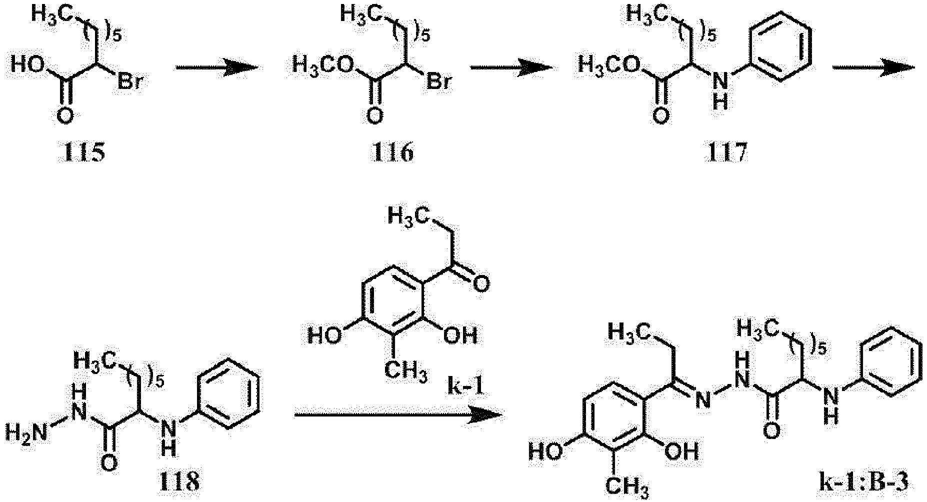

2-Bromo-n-octanoic acid (115) (600 mg, 2.7 mmol) was dissolved in methanol (5.5 mL), concentrated hydrochloric acid (3 drops) was added, and the mixture was stirred at 50°C for 18 hr. The mixture was allowed to cool, diethyl ether (30 mL), saturated aqueous sodium hydrogen carbonate solution (20 mL) were added and the mixture was partitioned. The organic layer was washed with saturated brine (20 mL), dried over anhydrous sodium sulfate, filtered, and concentrated under reduced pressure to give 116 (588 mg, 2.48 mmol, yield 92%) as a yellow liquid.

116 (585 mg, 2.47 mmol) obtained as mentioned above was dissolved in DMSO (5 mL), aniline (0.29 mL, 3.2 mmol), potassium carbonate (0.44 g, 3.2 mmol) were added, and the mixture was stirred at room temperature for 21 hr, and at 60°C for 16 hr. Ethyl acetate (30 mL), water (50 mL) were added and the mixture was partitioned. The organic layer was washed with saturated brine (30 mL), dried over anhydrous sodium sulfate, filtered, and concentrated under reduced pressure. The obtained residue was purified by moderate-pressure silica gel chromatography (silica gel 30 g, ethyl acetate/hexane=1/99 - 5/95) to give 117 (277 mg, 1.11 mmol, yield 45%) as a yellow liquid.

117 (256 mg, 1.08 mmol) obtained as mentioned above was dissolved in methanol (2.2 mL), hydrazine monohydrate (0.10 mL, 2.2 mmol) was added and the mixture was stirred at 50°C for 4 hr. Hydrazine monohydrate (0.50 mL, 10.3 mmol) was added and the mixture was stirred for 20 hr, hydrazine monohydrate (0.25 mL, 5.2 mmol) was added and the mixture was further stirred for 23 hr. The reaction solution was concentrated under reduced pressure, and the obtained residue was purified by moderate-pressure silica gel chromatography (aminesilica gel 10 g, ethyl acetate/methylene chloride =0/100 - 5/95) to give 118 (268 mg, 1.07 mmol, yield 99%) as a light yellow solid.

118 (144 mg, 0.58 mmol) obtained as mentioned above, k-1 (80 mg, 0.44 mmol) were dissolved in DMSO (0.9 mL), and the mixture was stirred at 100°C for 18 hr. The mixture was allowed to cool, and the reaction solution was directly purified by moderate-pressure silica gel column chromatography (silica gel 10 g, ethyl acetate/hexane=5/95 - 75/25), and continuously purified by moderate-pressure silica gel column chromatography (silica gel 10 g, ethyl acetate/methylene chloride =1/99 - 5/95). The obtained solid was washed with methylene chloride/IPE, and dried under reduced pressure to give k-1:B-3 (88.7 mg, 0.216 mmol, yield 49%) as a white solid.

¹H-NMR (400 MHz):δ10.82(s, 1H), 9.72(s, 1H), 7.25(d, J=8.0Hz, 1H), 7.06(t, J=8.0Hz, 2H), 6.65(d, J=8.0Hz, 2H), 6.54(t, J=8.0Hz, 1H), 6.39(d, J=8.0Hz, 1H), 5.87(d, J=8.0Hz, NH), 4.19(m, 1H), 2.81(q, J=8.0Hz, 2H), 1.95(s, 3H), 1.73(q, J=8.0Hz, 2H), 1.33(m, 4H), 1.27(m, 4H), 1.03(t, J=8.0Hz, 3H), 0.86(t, J=8.0Hz, 3H).(one signal of NH was not observed)

### [Synthetic Example 14] (Reference Example) Synthesis of k-1:B-5

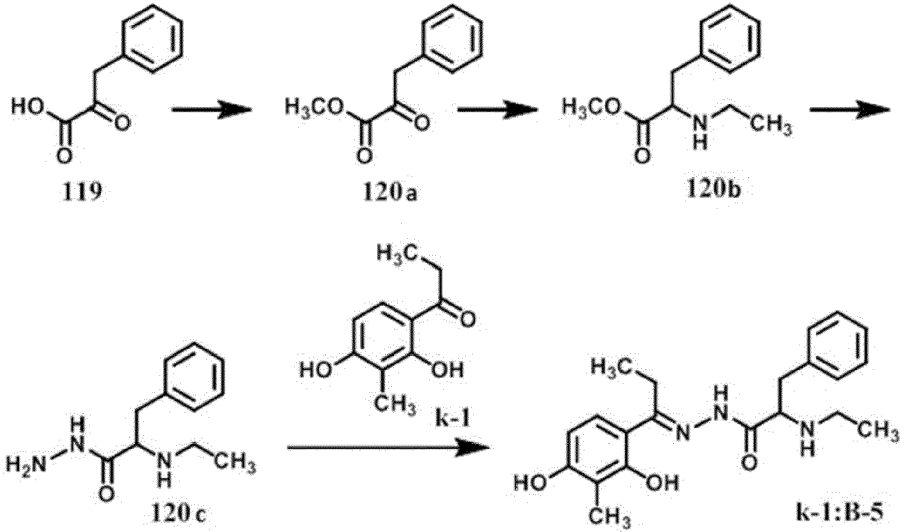

Phenylpyruvic acid (119) (1.0 g, 6.1 mmol) was dissolved in DMF (5 mL), DBU (1.0 mL, 6.7 mmol), methyl iodide (0.42 mL, 6.7 mmol) were added under ice-cooling and the mixture was stirred at room temperature for 3 hr. Ethyl acetate (30 mL), 1 M hydrochloric acid (50 mL) were added and the mixture was partitioned. The organic layer was washed with saturated brine (50 mL), dried over anhydrous sodium sulfate, filtered, and concentrated under reduced pressure. The obtained residue was purified by moderate-pressure silica gel chromatography (silica gel 30 g, ethyl acetate/hexane=0/100 - 13/87) to give 120a (298 mg, 1.67 mmol, yield 27%) as a white solid.

120a (295 mg, 1.66 mmol) obtained as mentioned above was dissolved in methanol (6.6 mL), acetic acid (0.66 mL), 2 M ethylamine THF solution (0.87 mL, 1.7 mmol), picoline borane (0.35 g, 3.3 mmol) was added under ice-cooling and the mixture was stirred at room temperature for 2.5 hr. 4 M hydrochloric acid (3 mL) was added and the mixture was heated at 50°C for 30 min, and allowed to cool. Ethyl acetate (20 mL), saturated aqueous sodium hydrogen carbonate solution (50 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel chromatography (silica gel 10 g, ethyl acetate/hexane=2/98 - 30/70) to give 120b (48.9 mg, 0.236 mmol, yield 14%).

120b (45 mg, 0.22 mmol) obtained as mentioned above was dissolved in methanol (0.9 mL), hydrazine monohydrate (0.021 mL, 0.43 mmol) was added and the mixture was stirred at 50°C for 3 hr. Methanol (0.9 mL), hydrazine monohydrate (0.021 mL, 0.43 mmol) were added and the mixture was stirred for 12 hr, methanol (0.9 mL), hydrazine monohydrate (0.021 mL, 0.43 mmol) were added and the mixture was further stirred for 3 hr. The reaction solution was concentrated under reduced pressure, and the obtained residue was purified by moderate-pressure silica gel chromatography (aminesilica gel 10 g, ethyl acetate/methylene chloride =0/100 - 20/80) to give 120c (32 mg, 0.15 mmol, yield 68%) as a light yellow liquid.

120c (30 mg, 0.14 mmol) obtained as mentioned above, k-1 (25 mg, 0.14 mmol) were dissolved in DMSO (0.3 mL), and the mixture was stirred at 100°C for 18 hr. The mixture was allowed to cool, and the reaction solution was directly purified by moderate-pressure silica gel column chromatography (silica gel 10 g, ethyl acetate/methylene chloride =10/90 - 60/40). The obtained solid was washed with IPE, and dried under reduced pressure to give k-1:B-5 (18.4 mg, 0.0498 mmol, yield 36%) as a white solid.

¹H-NMR (400 MHz):δ9.72(s, 1H), 7.30-7.10(m, 6H), 6.38(d, J=8.0Hz, 1H), 3.62(m, J=8.0Hz, 1H), 2.86(q, J=8.0Hz, 2H), 2.66(q, J=8.0Hz, 2H), 1.97(s, 3H), 1.04(d, J=8.0Hz, 2H), 0.98(t, J=8.0Hz, 3H), 0.92(t, J=8.0Hz, 3H).(two signals of NH and one signal of OH were not observed)

### [Synthetic Example 15] Synthesis of k-1:C-1

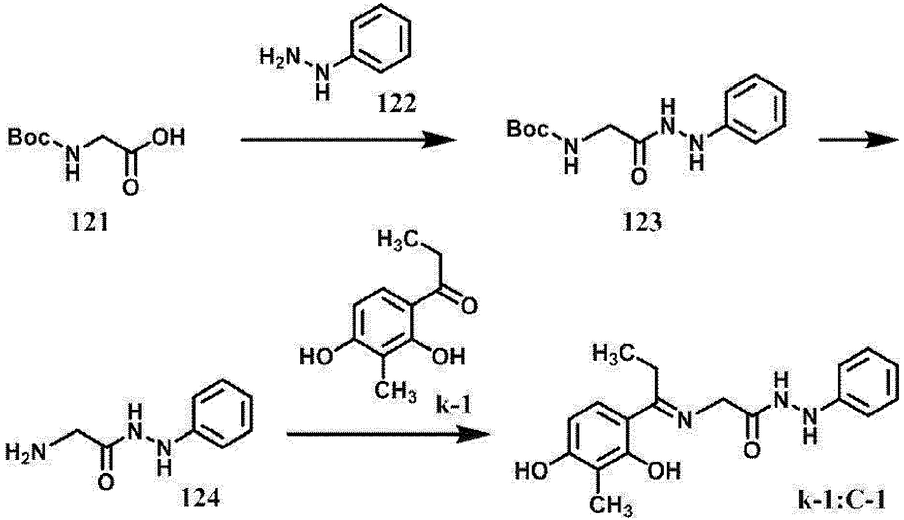

Boc-Gly-OH (121) (0.70 g, 4.0 mmol) was dissolved in methylene chloride (13 mL), WSC (0.84 g, 4.4 mmol), phenylhydrazine (122) (0.47 mL, 4.8 mmol) were added and the mixture was stirred at room temperature for 4 hr. Water (20 mL) was added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 30 g, ethyl acetate/hexane=15/85 - 65/35) to give 123 (853 mg, 3.22 mmol, yield 81%) as a colorless amorphous form.

To 123 (0.64 g, 2.4 mmol) obtained as mentioned above was added 4 M hydrochloric acid/dioxane (6 mL) and the mixture was stirred at room temperature for 3 hr. The precipitated solid was collected by filtration and purified by moderate-pressure silica gel column chromatography (aminesilica gel 30 g, methanol/methylene chloride =0/100 - 8/92) to give 124 (290 mg, 1.76 mmol, yield 73%) as a light yellow solid.

124 (120 mg, 0.72 mmol) obtained as mentioned above, k-1 (100 mg, 0.55 mmol) were dissolved in DMSO (1.1 mL), and the mixture was stirred at 100°C for 22 hr. The mixture was allowed to cool, and the reaction solution was directly purified by moderate-pressure silica gel column chromatography (silica gel 10 g, ethyl acetate/hexane=20/80 - 90/10). The obtained solid was washed with methylene chloride, and dried under reduced pressure to give k-1:C-1 (20.2 mg, 0.0617 mmol, yield 11%) as a yellow solid.

¹H-NMR (400 MHz):δ9.89 (s, 1H), 9.67(s, 1H), 7.83(s, 1H), 7.25(d, J=8.0Hz, 1H), 7.14(t, J=8.0Hz, 2H), 6.77(d, J=8.0Hz, 2H), 6.71(t, J=8.0Hz, 1H), 6.29(d, J=8.0Hz, 1H), 4.35(s, 2H), 2.74(q, J=8.0Hz, 2H), 1.92(s, 3H), 1.13(t, J=8.0Hz, 3H).(one signal of NH was not observed)

### [Synthetic Example 16] Synthesis of k-1:C-2

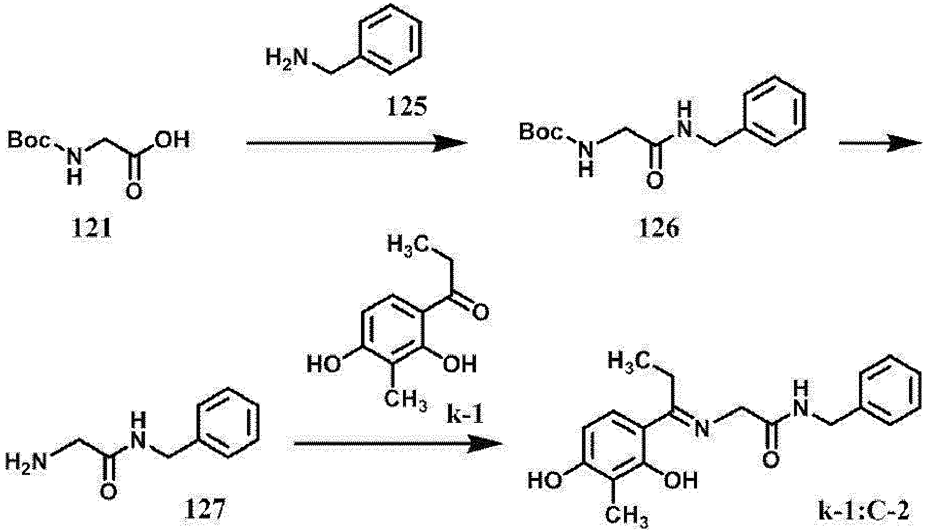

Boc-Gly-OH (121) (0.70 g, 4.0 mmol) was dissolved in methylene chloride (13 mL), WSC (0.84 g, 4.4 mmol), benzylamine (125) (0.52 mL, 4.8 mmol) were added and the mixture was stirred at room temperature for 3 hr. Water (20 mL), methylene chloride (20 mL) were added and the mixture was partitioned. The organic layer was washed with saturated brine (20 mL), dried over anhydrous sodium sulfate, filtered, and concentrated under reduced pressure. The obtained residue was purified by moderate-pressure silica gel column chromatography (silica gel 30 g, ethyl acetate/hexane=10/90 - 65/35) to give 126 (937 mg, 3.54 mmol, yield 89%) as a colorless liquid.

To 126 (0.93 g, 3.5 mmol) obtained as mentioned above was added TFA (7 mL) and the mixture was stirred at room temperature for 3.5 hr. The reaction solution was concentrated under reduced pressure. The obtained residue was purified by moderate-pressure silica gel column chromatography (aminesilica gel 30 g, ethyl acetate/methylene chloride =10/90 - 95/5) to give 127 (457 mg, 2.78 mmol, yield 79%) as a light yellow liquid.

127 (120 mg, 0.72 mmol) obtained as mentioned above, k-1 (100 mg, 0.55 mmol) were dissolved in DMSO (1.1 mL), and the mixture was stirred at 100°C for 22 hr. The mixture was allowed to cool, and the reaction solution was directly purified by moderate-pressure silica gel column chromatography (silica gel 10 g, ethyl acetate/hexane=10/90 - 80/20). The obtained solid was washed with IPE, methylene chloride, and dried under reduced pressure to give k-1:C-2 (29.9 mg, 0.0908 mmol, yield 17%) as a yellow solid.

¹H-NMR (400 MHz):δ9.63 (s, 1H), 8.55(m, NH), 7.40-7.20(m, 6H), 6.25(d, J=8.0Hz, 1H), 4.32(d, J=8.0Hz, 2H), 4.29(s, 2H), 2.70(q, J=8.0Hz, 2H), 1.91(s, 3H), 1.09(t, J=8.0Hz, 3H).(one signal of OH was not observed)

### [Synthetic Example 17] Synthesis of k-1:D-1

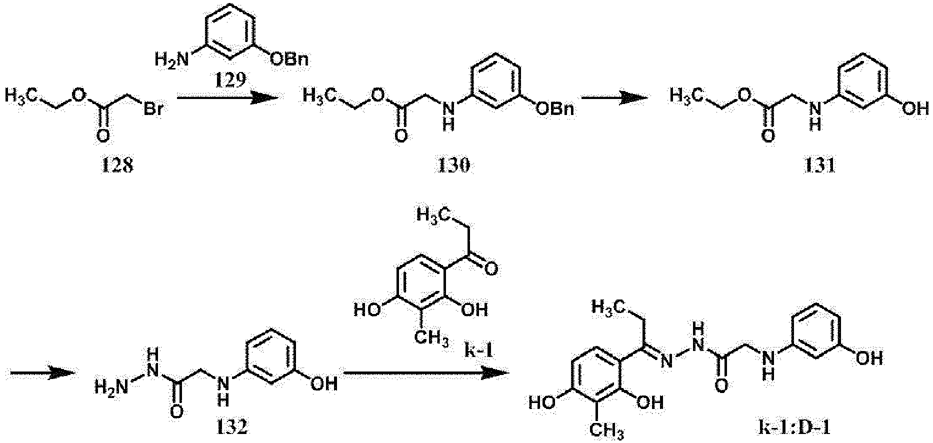

3-Benzyloxyaniline (129) (2.44 g, 12.2 mmol) was dissolved in DMF (24 mL), sodium acetate (1.10 g, 13.5 mmol), ethyl bromoacetate (128) (1.49 mL, 13.5 mmol) were added and the mixture was stirred at room temperature for 4 hr. Water (300 mL), ethyl acetate (150 mL) were added and the mixture was partitioned. The organic layer was washed with saturated brine (100 mL), dried over anhydrous sodium sulfate, filtered, and concentrated under reduced pressure. The obtained residue was purified by moderate-pressure silica gel column chromatography (silica gel 50 g, ethyl acetate/hexane=2/98 - 10/90) to give 130 (2.82 g, 9.88 mmol, yield 81%) as a light yellow solid.

130 (1.0 g, 3.5 mmol) obtained as mentioned above was suspended in methanol (18 mL), 10% Pd/C (0.1 g) was added and the mixture was stirred under a hydrogen atmosphere at room temperature for 5 hr. The reaction solution was filtered through celite, and the filtrate was concentrated under reduced pressure. The obtained residue was purified by moderate-pressure silica gel column chromatography (silica gel 30 g, ethyl acetate/hexane=3/97 - 35/65) to give 131 (295 mg, 1.51 mmol, yield 43%) as a light yellow liquid.

131 (0.29 g, 1.5 mmol) obtained as mentioned above was dissolved in ethanol (3.5 mL), hydrazine monohydrate (0.32 mL, 6.5 mmol) was added, and the mixture was stirred at 60°C for 18 hr. The reaction solution was concentrated under reduced pressure, and the obtained residue was purified by moderate-pressure silica gel chromatography (aminesilica gel 10 g, methanol/methylene chloride =1/99 - 10/90). The obtained solid was washed with IPE, and dried under reduced pressure to give 132 (239 mg, 1.32 mmol, yield 88%) as a light yellow solid.

132 (130 mg, 0.72 mmol) obtained as mentioned above, k-1 (100 mg, 0.55 mmol) were dissolved in DMSO (1.1 mL), and the mixture was stirred at 100°C for 21 hr. The mixture was allowed to cool, and the reaction solution was directly purified by moderate-pressure silica gel column chromatography (silica gel 10 g, ethyl acetate/methylene chloride =5/95 - 50/50). The obtained solid was washed with water and IPE, and dried under reduced pressure to give k-1:D-1 (95.9 mg, 0.279 mmol, yield 51%) as a white solid.

¹H-NMR (270 MHz); δ 13.66(s, 1H), 10.76(s, 1H), 9.70(s, 1H), 8.98(s, 1H), 7.25(d, J=10.8Hz, 1H), 6.86(t, J=10.8Hz, 1H), 6.40(d, J=8.1Hz, 1H), 6.20-5.95(m, 3H), 5.87(t, J=5.4Hz, NH), 3.88(d, J=5.4Hz, 2H), 2.78(q, J=8.0Hz, 2H), 1.97(s, 3H), 1.05(t, J=8.1Hz, 3H).

### [Synthetic Example 18] Synthesis of k-1:D-3

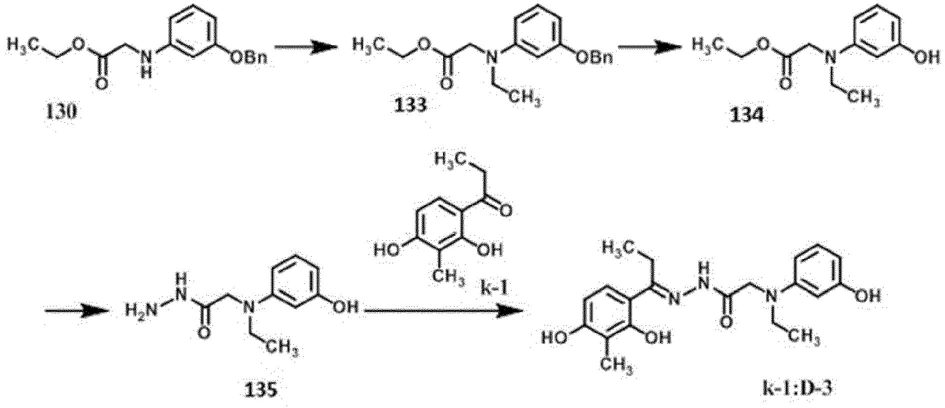

130 (1.2 g, 4.2 mmol) obtained in the step of Synthetic Example 17 was dissolved in DMF (12 mL), potassium carbonate (1.16 g, 8.4 mmol), iodoethane (1.35 mL, 16.8 mmol) were added and the mixture was stirred at 100°C for 3 hr. Iodoethane (0.7 mL, 8.7 mmol) was added and the mixture was stirred for 1.5 hr, and at room temperature for 20 hr. Water (100 mL), ethyl acetate (50 mL) were added and the mixture was partitioned. The organic layer was washed with saturated brine (50 mL), dried over anhydrous sodium sulfate, filtered, and concentrated under reduced pressure. The obtained residue was purified by moderate-pressure silica gel column chromatography (silica gel 10 g, ethyl acetate/hexane=2/98 - 10/90) to give 133 (1.21 g, 3.86 mmol, yield 92%) as a colorless liquid.

Compound 133 (1.2 g, 3.9 mmol) obtained as mentioned above was dissolved in methanol (19 mL), 10% Pd/C (0.1 g) was added and the mixture was stirred under a hydrogen atmosphere at room temperature for 15 hr. The reaction solution was filtered through celite, and the filtrate was concentrated under reduced pressure. The obtained residue was purified by moderate-pressure silica gel column chromatography (silica gel 30 g, ethyl acetate/hexane=5/95 - 25/75) to give 134 (194 mg, 0.869 mmol, yield 22%).

134 (0.20 g, 0.90 mmol) obtained as mentioned above was dissolved in ethanol (2.2 mL), hydrazine monohydrate (0.22 mL, 4.5 mmol) was added and the mixture was stirred at 50°C for 18 hr, hydrazine monohydrate (0.22 mL, 4.5 mmol) was added and the mixture was further stirred at 60°C for 24 hr. The reaction solution was concentrated under reduced pressure, and the obtained residue was purified by moderate-pressure silica gel chromatography (aminesilica gel 10 g, methanol/methylene chloride =1/99 - 6/94). The obtained solid was washed with IPE, and dried under reduced pressure to give 135 (151 mg, 0.722 mmol, yield 80%) as a light yellow solid.

135 (150 mg, 0.72 mmol) obtained as mentioned above, k-1 (100 mg, 0.55 mmol) were dissolved in DMSO (1.1 mL), and the mixture was stirred at 100°C for 23 hr. The mixture was allowed to cool, and the reaction solution was directly purified by moderate-pressure silica gel column chromatography (silica gel 10 g, ethyl acetate/hexane=15/85 - 80/20). The obtained solid was washed with a mixed solvent of IPE/methylene chloride (1/1), and dried under reduced pressure to give k-1:D-3 (118 mg, 0.318 mmol, yield 58%) as a yellow solid.

¹H-NMR (270 MHz); δ 13.68(s, 1H), 10.78(s, 1H), 9.69(s, 1H), 9.00(s, 1H), 7.26(d, J=8.1Hz, 1H), 6.91(t, J=8.1Hz, 1H), 6.40(d, J=8.1Hz, 1H), 6.11(d, J=8.1Hz, 1H), 6.10-6.05(m, 2H), 4.12(s, 2H), 3.42(q, J=8.1Hz, 2H), 2.79(q, J=8.1Hz, 2H), 1.96(s, 3H), 1.13(t, J=8.1Hz, 3H), 1.05(t, J=8.1Hz, 3H).

### [Synthetic Example 19] Synthesis of k-1:E-1

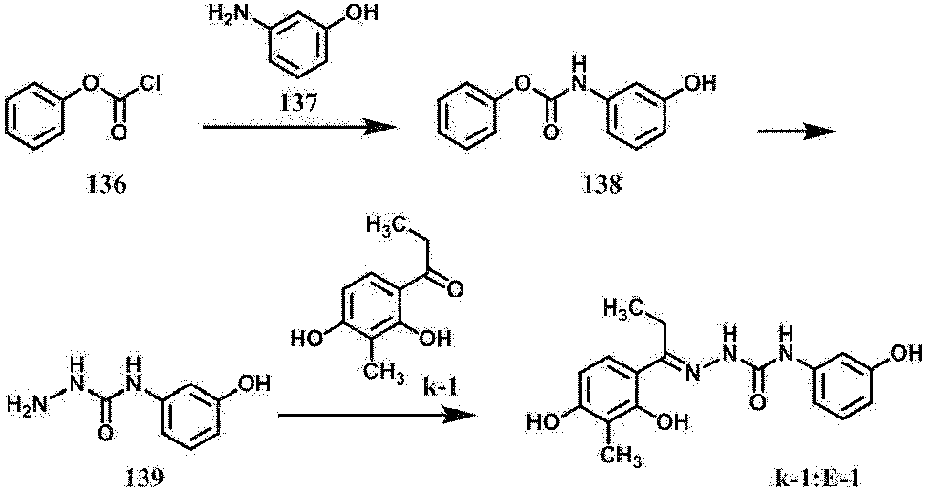

Aminophenol (137) (500 mg, 4.6 mmol) was dissolved in THF (5 mL), water (5 mL), sodium hydrogen carbonate (0.77 g, 9.2 mmol) was added and phenyl chloroformate (136) (0.61 mL, 4.8 mmol) was slowly added dropwise under ice-cooling. The mixture was stirred at the same temperature for 2 hr, ethyl acetate (20 mL), 2 M hydrochloric acid (20 mL) were added and the mixture was partitioned. The organic layer was washed with saturated brine (20 mL), dried over anhydrous sodium sulfate, filtered, and concentrated under reduced pressure. The obtained solid was washed with IPE, and dried under reduced pressure to give 138 (0.94 g, 4.1 mmol, yield 89%) as a white solid.

138 (0.94 g, 4.1 mmol) obtained as mentioned above was dissolved in acetonitrile (4 mL), hydrazine monohydrate (1.0 mL, 21 mmol) was added, and the mixture was stirred at 60°C for 23 hr. The reaction solution was concentrated under reduced pressure, and the obtained residue was purified by moderate-pressure silica gel chromatography (aminesilica gel 30 g, methanol/methylene chloride =0/100 - 12/88) to give 139 (664 mg, 3.97 mmol, yield 97%) as a light yellow solid.

139 (120 mg, 0.72 mmol) obtained as mentioned above, k-1 (100 mg, 0.55 mmol) were dissolved in DMSO (1.1 mL), and the mixture was stirred at 100°C for 16 hr. The mixture was allowed to cool, and the reaction solution was directly purified by moderate-pressure silica gel column chromatography (silica gel 10 g, ethyl acetate/hexane=10/90 - 80/20), and continuously by moderate-pressure silica gel column chromatography (silica gel 10 g, methanol/methylene chloride =1/99 - 5/95). The obtained solid was washed with IPE to give k-1:E-1 (19.7 mg, 0.0598 mmol, yield 11%) as a white solid.

¹H-NMR (400 MHz); δ 9.70(s, 1H), 9.60(s, 1H), 9.38(s, 1H), 8.73(s, 1H), 7.21(d, J=8.0Hz, 1H), 7.06(t, J=8.0Hz, 1H), 7.05(s, 1H), 6.82(d, J=8.0Hz, 1H), 6.39(d, J=8.0Hz, 2H), 5.76(s, 1H), 2.69(q, J=8.0Hz, 2H), 1.99(s, 3H), 1.13(t, J=8.0Hz, 3H).

### [Synthetic Example 20] (Reference Example) Synthesis of k-1:F-1

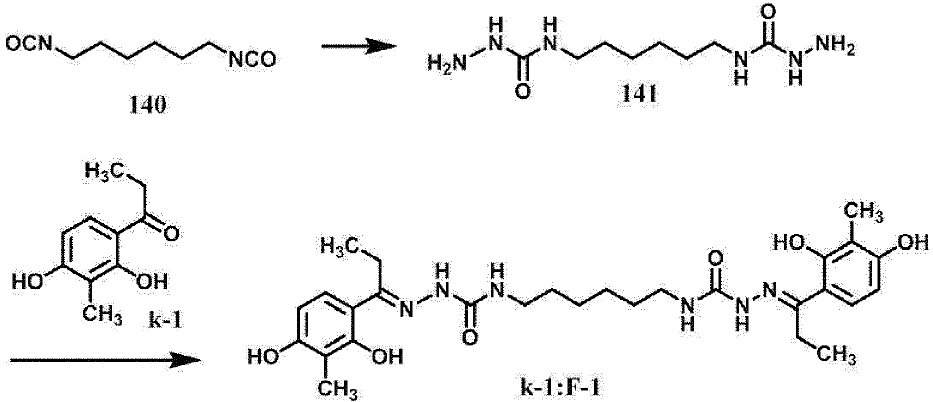

Hydrazine monohydrate (0.58 mL, 12 mmol) was dissolved in methylene chloride (6 mL), hexamethylene diisocyanate (140) (0.48 mL, 3.0 mmol) was added under ice-cooling and the mixture was stirred at room temperature for 1.5 hr. Hydrazine monohydrate (0.29 mL, 6.0 mmol) was added and the mixture was stirred for 2 hr. The resulting solid was collected by filtration, and dried under reduced pressure to give 141 (0.60 g, 2.6 mmol, yield 87%) as a white solid.

141 (100 mg, 0.43 mmol) obtained as mentioned above, k-1 (233 mg, 1.29 mmol) were suspended in DMSO (1.4 mL) and the mixture was stirred at 100°C for 20 hr. The mixture was allowed to cool, methylene chloride was added, an insoluble material was filtered off, and the filtrate was concentrated under reduced pressure. The obtained solid was washed with methylene chloride and water, and dried under reduced pressure to give k-1:F-1 (88.6 mg, 0.159 mmol, yield 37%) as a light yellow solid.

¹H-NMR (400 MHz); δ 9.44(s,4H), 7.15(d, J=8.0Hz, 2H), 6.40-6.30(m, 2H), 3.13(q, J=8.0Hz, 4H), 2.63 (q, J=8.0Hz, 4H),1.97(s, 6H), 1.47(m, 4H), 1.38(m, 4H),1.07(t, J=8.0Hz, 6H).

### [Synthetic Example 21] Synthesis of k-1:G-1

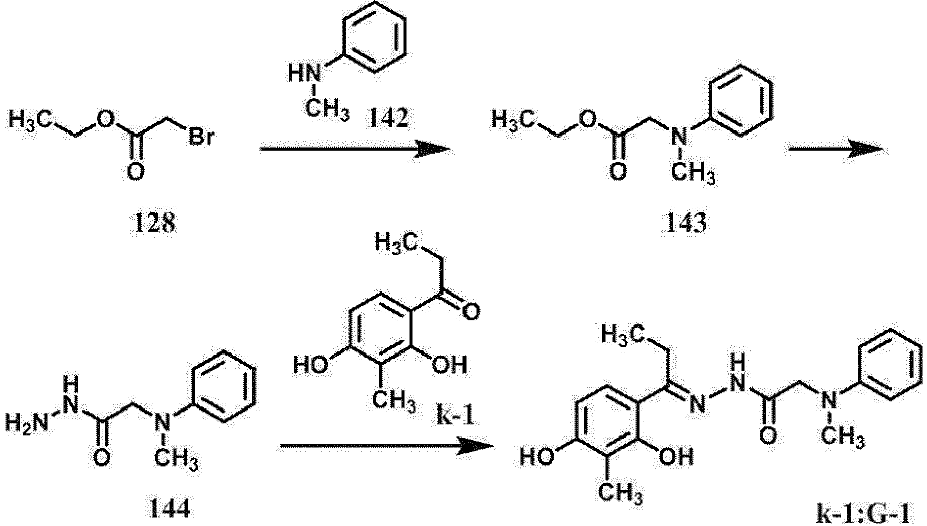

N-methyl-aniline (142) (0.50 g, 4.7 mmol) was dissolved in ethanol (9 mL), potassium carbonate (0.97 g, 7.0 mmol), ethyl bromoacetate (128) (0.57 mL, 5.1 mmol) were added, and the mixture was stirred at 60°C for 21 hr. Insoluble materials were filtered off, and the filtrate was concentrated under reduced pressure. To the obtained residue were added ethyl acetate (30 mL), water (30 mL) and the mixture was partitioned. The organic layer was washed with saturated brine (30 mL), dried over anhydrous sodium sulfate, filtered, and concentrated under reduced pressure. The obtained residue was purified by moderate-pressure silica gel column chromatography (silica gel 10 g, ethyl acetate/hexane=0/100 - 5/95) to give 143 (496 mg, 2.57 mmol, yield 55%) as a light yellow liquid.

143 (0.49 g, 2.6 mmol) obtained as mentioned above was dissolved in methanol (5 mL), hydrazine monohydrate (0.25 mL, 5.1 mmol) was added, and the mixture was stirred at 55°C for 15 hr. Hydrazine monohydrate (0.50 mL, 10 mmol) was added and the mixture was further stirred at 60°C for 18 hr. The reaction solution was concentrated under reduced pressure, and the obtained solid was washed with IPE, and dried under reduced pressure to give 144 (389 mg, 2.17 mmol, yield 83%) as a white solid.

144 (130 mg, 0.72 mmol) obtained as mentioned above, k-1 (100 mg, 0.55 mmol) were dissolved in DMSO (1.1 mL), and the mixture was stirred at 100°C for 17 hr. The mixture was allowed to cool, and the reaction solution was directly purified by moderate-pressure silica gel column chromatography (silica gel 10 g, ethyl acetate/hexane=10/90 - 80/20). The obtained solid was washed with IPE, and dried under reduced pressure to give k-1:G-1 (92 mg, 0.27 mmol, yield 49%) as a white solid.

¹H-NMR (270 MHz); δ 13.64(s, 1H), 10.86(s, 1H), 9.67(s, 1H), 7.24(d, J=10.8Hz, 1H), 7.15(t, J=10.8Hz, 2H), 6.71(d, J=8.1Hz, 2H), 6.62(t, J=8.1Hz, 1H), 6.38(d, J=8.1Hz, 1H), 4.23(s, 2H), 3.04(s, 3H), 2.89(q, J=8.1Hz, 2H), 1.93(s, 3H), 1.05(t, J=8.1Hz, 3H) .

### [Synthetic Example 22] Synthesis of k-1:L-1

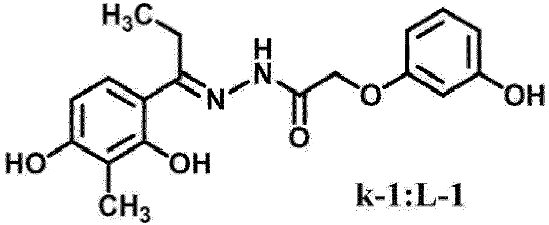

k-1:L-1 (65 mg, white solid) was synthesized by a method according to the above-mentioned Synthetic Example 21, and using resorcinol as a starting material.

¹H-NMR (270 MHz); δ 13.63(s, 1H), 10.99(s, 1H), 9.74(s, 1H), 9.46(s, 1H), 7.28(d, J=8.1Hz, 1H), 7.07(t, J=8.1Hz, 1H), 6.45-6.35(m, 4H), 4.72(s, 2H), 2.81(q, J=8.1Hz, 2H), 1.97(s, 3H), 1.08(t, J=8.1Hz, 3H).

### [Synthetic Example 23] Synthesis of k-1:M-1

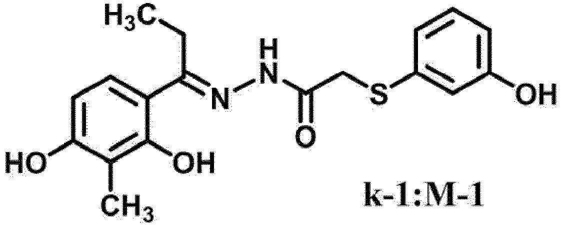

k-1:M-1 (119 mg, white solid) was synthesized by a method according to the above-mentioned Synthetic Example 21, and using 3-mercaptophenol as a starting material.

¹H-NMR (270 MHz); δ13.64(s, 1H), 10.96(s, 1H), 9.72(s, 1H), 9.57(s, 1H), 7.26(d, J=8.1Hz, 1H), 7.11(t, J=8.1Hz, 1H), 6.82(d, J=8.1Hz, 1H), 6.80(s, 1H), 6.62(d, J=8.1Hz, 1H), 6.40(d, J=8.1Hz, 1H), 3.88(s, 2H), 2.78(q, J=8.1Hz, 2H), 1.97(s, 3H), 1.06(t, J=8.1Hz, 3H).

### [Synthetic Example 24] Synthesis of k-1:G-2

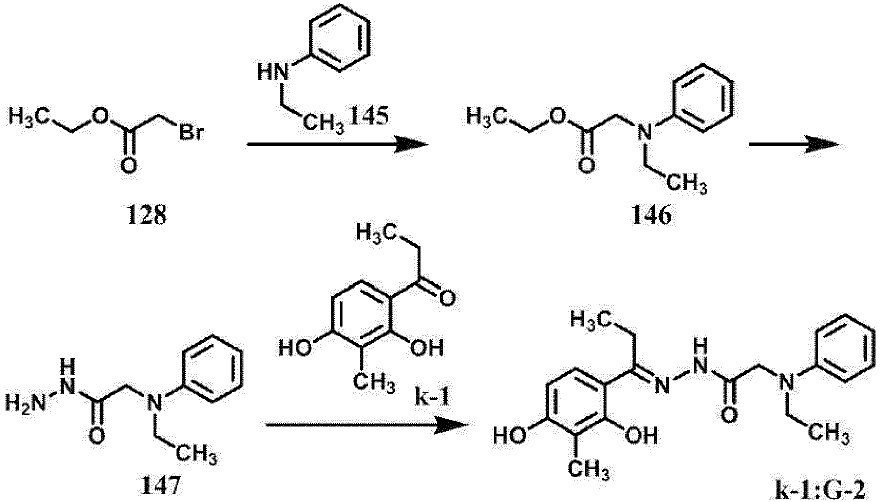

N-ethyl-aniline (145) (0.50 g, 4.1 mmol) was dissolved in ethanol (8 mL), potassium carbonate (0.86 g, 6.2 mmol), ethyl bromoacetate (128) (0.50 mL, 4.1 mmol) were added, and the mixture was stirred at 60°C for 21 hr. Insoluble materials were filtered off, and the filtrate was concentrated under reduced pressure. To the obtained residue were added ethyl acetate (30 mL), water (30 mL) and the mixture was partitioned. The organic layer was washed with saturated brine (30 mL), dried over anhydrous sodium sulfate, filtered, and concentrated under reduced pressure. The obtained residue was purified by moderate-pressure silica gel column chromatography (silica gel 10 g, ethyl acetate/hexane=0/100 - 5/95) to give 146 (473 mg, 2.28 mmol, yield 56%) as a light yellow liquid.

146 (0.47 g, 2.3 mmol) obtained as mentioned above was dissolved in methanol (4.5 mL), hydrazine monohydrate (0.22 mL, 4.5 mmol) was added, and the mixture was stirred at 55°C for 15 hr. Hydrazine monohydrate (0.44 mL, 9.1 mmol) was added and the mixture was further stirred at 60°C for 18 hr. The reaction solution was concentrated under reduced pressure, and the obtained solid was washed with IPE, and dried under reduced pressure to give 147 (309 mg, 1.60 mmol, yield 70%) as a white solid.

147 (140 mg, 0.72 mmol) obtained as mentioned above, k-1 (100 mg, 0.55 mmol) were dissolved in DMSO (1.1 mL), and the mixture was stirred at 100°C for 17 hr. The mixture was allowed to cool, and the reaction solution was directly purified by moderate-pressure silica gel column chromatography (silica gel 10 g, ethyl acetate/hexane=10/90 - 80/20). The obtained solid was washed with IPE, and dried under reduced pressure to give k-1:G-2 (96 mg, 0.27 mmol, yield 49%) as a white solid.

¹H-NMR (270 MHz); δ 13.66(s, 1H), 10.82(s, 1H), 9.68(s, 1H), 7.25(d, J=8.1Hz, 1H), 7.13(t, J=8.1Hz, 2H), 6.65(d, J=8.1Hz, 2H), 6.59(t, J=8.1Hz, 1H), 6.38(d, J=10.8Hz, 1H), 4.17(s, 2H),3.46(q, J=8.1Hz, 2H), 2.79(q, J=8.1Hz, 2H), 1.94(s, 3H), 1.13(t, J=8.1Hz, 3H), 1.03(t, J=8.1Hz, 3H).

### [Synthetic Example 25] Synthesis of k-1:J-1

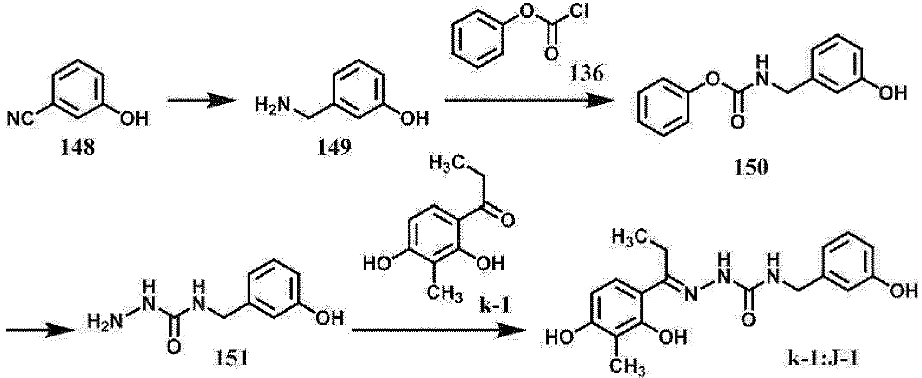

To ice-cooled THF (10 mL) were successively added hydrogenated aluminum (1.0 g, 26 mmol), 3-cyanophenol (148) (0.63 g, 5.3 mmol), and the mixture was stirred at room temperature for 1.5 hr, and at 60°C for 3.5 hr. The mixture was allowed to cool, hydrogenated aluminum (1.0 g, 26 mmol), THF (10 mL) were added and the mixture was further stirred at 60°C for 16 hr. The reaction solution was ice-cooled, water (1.5 mL), 15% aqueous sodium hydroxide solution (1.5 mL), water (4.5 mL) were successively added and the mixture was stirred at room temperature for 3 hr. The suspended solution was filtered through celite, and the filtrate was concentrated under reduced pressure. The obtained residue was purified by moderate-pressure silica gel column chromatography (aminesilica gel 10 g, methanol/methylene chloride =0/100 - 8/92). The obtained solid was washed with IPE, and dried under reduced pressure to give 149 (477 mg, 3.87 mmol, yield 73%) as a white solid.

149 (200 mg, 1.6 mmol) obtained as mentioned above was dissolved in methylene chloride (2 mL), water (2 mL), sodium hydrogen carbonate (0.27 g, 3.2 mmol) was added and phenyl chloroformate (136) (0.22 mL, 1.7 mmol) was slowly added dropwise under ice-cooling. The mixture was stirred at room temperature for 20 hr, ethyl acetate (20 mL), water (20 mL) were added and the mixture was partitioned. The organic layer was washed with saturated brine (20 mL), dried over anhydrous magnesium sulfate, filtered, and concentrated under reduced pressure. The obtained residue was purified by moderate-pressure silica gel column chromatography (silica gel 10 g, ethyl acetate/hexane=5/95 - 35/65) to give 150 (369 mg, 1.52 mmol, yield 95%) as a colorless liquid.

150 (365 mg, 1.50 mmol) obtained as mentioned above was suspended in acetonitrile (3.8 mL), hydrazine monohydrate (0.18 mL, 3.8 mmol) was added and the mixture was stirred at room temperature for 2.5 hr. Hydrazine monohydrate (0.18 mL, 3.8 mmol) was added and the mixture was further stirred at 55°C for 20 hr. The reaction solution was concentrated under reduced pressure, and the obtained solid was washed with IPE/methylene chloride (3/1), and dried under reduced pressure to give 151 (233 mg, 1.29 mmol, yield 86%) as a white solid.

151 (130 mg, 0.72 mmol) obtained as mentioned above, k-1 (100 mg, 0.55 mmol) were dissolved in DMSO (1.1 mL), and the mixture was stirred at 100°C for 15 hr. The mixture was allowed to cool, and the reaction solution was directly purified by moderate-pressure silica gel column chromatography (silica gel 10 g, ethyl acetate/methylene chloride =10/90 - 55/45). To the obtained purified product was added water and the precipitated solid was collected by filtration and dried under reduced pressure to give k-1:J-1 (102 mg, 0.297 mmol, yield 54%) as a white solid.

¹H-NMR (270 MHz); δ13.45(brs, 1H), 9.57(s, 1H), 9.53(s, 1H), 9.36(s, 1H), 7.17(d, J=8.1Hz, 1H), 7.11(d, J=8.1Hz, 1H), 6.80-6.60(m, 4H), 6.37(d, J=8.1Hz, 1H), 4.25(d, J=5.4Hz, 2H), 2.65(q, J=8.1Hz, 2H), 1.97(s, 3H), 1.08(t, J=8.1Hz, 3H).

### [Synthetic Example 26] (Reference Example) Synthesis of k-1:J-5

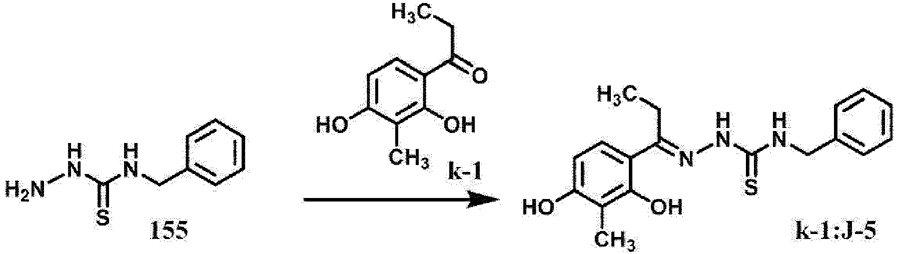

4-Benzyl-3-thiosemicarbazide (155) (130 mg, 0.72 mmol), k-1 (100 mg, 0.55 mmol) were dissolved in DMSO (1.1 mL), and the mixture was stirred at 100°C for 17 hr. The mixture was allowed to cool, and the reaction solution was directly purified by moderate-pressure silica gel column chromatography (silica gel 10 g, ethyl acetate/hexane=5/95 - 80/20), and continuously by moderate-pressure silica gel column chromatography (silica gel 10 g, ethyl acetate/hexane=10/90 - 50/50). The obtained solid was washed with IPE, and dried under reduced pressure to give k-1:J-5 (49.9 mg, 0.145 mmol, yield 26%) as a white solid.

¹H-NMR (400 MHz); δ10.61(brs, 1H), 9.69(s, 1H), 8.43(brs, 1H), 7.40-7.20(m, 6H), 6.40 (d, J=8.0Hz, 1H), 4.78(d, J=8.0Hz, 2H), 2.74(q, J=8.1Hz, 2H), 1.99(s, 3H), 1.09(t, J=8.1Hz, 3H). (one signal of NH was not observed)

### [Synthetic Example 27] (Reference Example) Synthesis of k-1:J-6

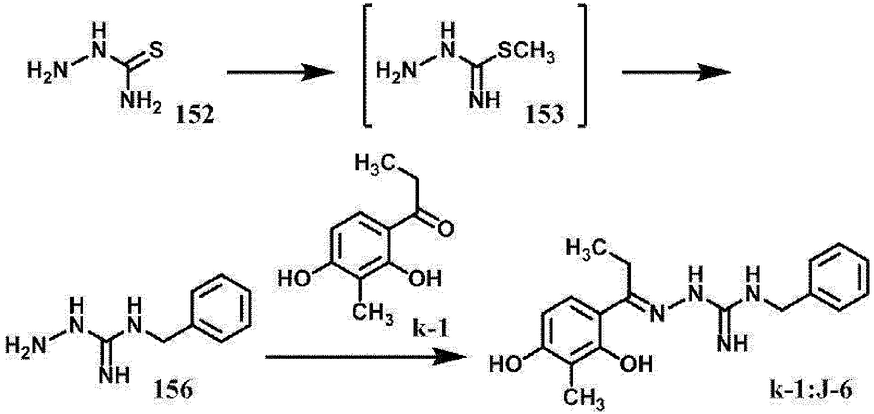

Thiosemicarbazide (152) (0.50 g, 5.5 mmol) was suspended in methanol (5.5 mL), methyl iodide (0.41 mL, 6.6 mmol) was added and the mixture was stirred at 60°C for 1.5 hr, and at 70°C for 50 min with the lid of the reaction container opened. The mixture was allowed to cool, benzylamine (0.61 mL, 5.5 mmol) was added, and the mixture was stirred at 55°C for 17 hr. The reaction solution was concentrated under reduced pressure, and the obtained residue was washed with a mixed solvent of IPE/methylene chloride (1/4). The obtained crude purified product was purified by moderate-pressure silica gel column chromatography (aminesilica gel 30 g, methanol/methylene chloride =0/100 - 15/85), and the obtained solid was washed with methylene chloride and dried under reduced pressure to give 156 (626 mg, 3.81 mmol, yield 69%) as an orange solid.

156 (120 mg, 0.72 mmol) obtained as mentioned above, k-1 (100 mg, 0.55 mmol) were dissolved in DMSO (1.1 mL), and the mixture was stirred at 100°C for 27 hr. The mixture was allowed to cool, and the reaction solution was directly purified by moderate-pressure silica gel column chromatography (silica gel 10 g, ethyl acetate/methylene chloride =10/90 - 80/20). To the obtained crude purified product were added methylene chloride, water and the mixture was partitioned. The organic layer was dried over anhydrous magnesium sulfate, filtered, and concentrated under reduced pressure. The obtained residue was purified by moderate-pressure silica gel column chromatography (silica gel 10 g, ethyl acetate/hexane=10/90 - 60/40) to give k-1:J-6 (29.9 mg, 0.0916 mmol, yield 17%) as a yellow amorphous form.

¹H-NMR (270 MHz); δ14.51(brs, 1H), 9.39(s, 1H), 7.36-7.05 (m, 6H), 6.32(d, J=8.1Hz, 1H), 6.20 (m, NH), 5.30(brs, NH), 4.34(d, J=5.4Hz, 2H), 2.84(q, J=8.1Hz, 2H), 1.96(s, 3H), 0.96(t, J=8.1Hz, 3H).

### [Synthetic Example 28] Synthesis of k-1:N-1

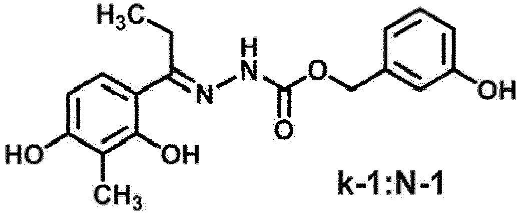

2-Hydroxybenzyl alcohol (1.0 g, 8.1 mmol) was suspended in methylene chloride (10 mL), water (10 mL) and sodium hydrogen carbonate (2.0 g, 24 mmol) was added, and phenyl chloroformate (2.0 mL, 16 mmol) was slowly added dropwise under ice-cooling. The temperature was gradually rose to room temperature and the mixture was stirred for 5.5 hr. Methylene chloride (20 mL), water (20 mL) were added and the mixture was partitioned. The organic layer was washed with saturated brine (30 mL), dried over anhydrous magnesium sulfate, filtered, and concentrated under reduced pressure. The obtained residue was purified by moderate-pressure silica gel column chromatography (silica gel 30 g, ethyl acetate/hexane=2/98 - 35/65) to give 3-(hydroxymethyl)phenyl phenyl carbonate (1.45 g, 5.94 mmol, yield 73%) as a white solid.

3-(Hydroxymethyl)phenyl phenyl carbonate obtained as mentioned above was dissolved in methylene chloride (7 mL), pyridine (0.72 mL, 8.9 mmol) and a small amount of DMAP were added, and phenyl chloroformate (0.90 mL, 7.1 mmol) was slowly added dropwise under ice-cooling. The mixture was stirred at room temperature for 2 hr, methylene chloride (10 mL), water (30 mL) were added and the mixture was partitioned. The organic layer was washed with saturated brine (30 mL), dried over anhydrous magnesium sulfate, filtered, and concentrated under reduced pressure. The obtained residue was purified by moderate-pressure silica gel column chromatography (silica gel 30 g, ethyl acetate/hexane=3/97 - 25/75) to give 3-[(phenoxycarbonyl)oxy]benzyl phenyl carbonate (2.20 g, 6.04 mmol, yield 102%) as a colorless liquid.

3-[(Phenoxycarbonyl)oxy]benzyl phenyl carbonate (2.2 g, 6.0 mmol) obtained as mentioned above was suspended in ethanol (10 mL), hydrazine monohydrate (2.9 mL, 60 mmol) was added, and the mixture was stirred at 60°C for 21 hr. Insoluble materials were filtered off, and the filtrate was concentrated under reduced pressure. The obtained residue was purified by moderate-pressure silica gel column chromatography (silica gel 30 g, methanol/methylene chloride =0/100 - 10/90). The obtained solid was suspension washed with methylene chloride to give 3-hydroxybenzyl hydrazinecarboxylate (808 mg, 4.44 mmol, yield 74%) as a white solid.

3-Hydroxybenzyl hydrazinecarboxylate (130 mg, 0.72 mmol) obtained as mentioned above, 2',4'-dihydroxy-3'-methylpropiophenone (100) (100 mg, 0.55 mmol) were suspended in DMSO (1.1 mL) and the mixture was stirred at 100°C for 22 hr. The mixture was allowed to cool, and the reaction solution was directly purified by moderate-pressure silica gel column chromatography (silica gel 10 g, ethyl acetate/methylene chloride =2/98 - 30/70). To the obtained purified product was added water and the precipitated solid was collected by filtration to give k-1:N-1 (85.5 mg, 0.248 mmol, yield 45%) as a white solid.

¹H-NMR (270 MHz); δ13.45(brs, 1H), 10.78(brs, 1H), 9.63 (s, 1H), 9.49 (s, 1H), 7.21(d, J=8.1Hz, 1H), 7.18(t, J=8.1Hz, 1H), 6.84(d, J=8.1Hz, 1H), 6.83(s, 1H), 6.73(d, J=8.1Hz, 1H), 6.38(d, J=8.1Hz, 1H), 5.14(s, 2H), 2.75(q, J=8.1Hz, 2H), 1.97(s, 3H), 1.03(t, J=8.1Hz, 3H).

The results of the action test performed using the compound to be used in the present invention are described in the following.

### [Experimental Example 1] Study of cell proliferation activity of each compound

A cell proliferation promoting effect when the compound to be used in the present invention was added to a three-dimensional medium was studied. Specifically, precultured (adhesion cultured) SKOV3 cells (human ovarian cancer-derived cell line) were recovered and suspended in a three-dimensional cell culture medium ("FCeM (registered trade mark)" (Nissan chemical corporation)) to prepare a cell suspension. The cell suspension was seeded in the wells of a 384 well flat bottom Ultr-low Attachment surface microplate (manufactured by Corning Incorporated, #3827) at 1000 cells/40 µL/well. The cell suspension seeded in the plate was stood overnight at 37°C, 5% CO₂. Then, a DMSO diluted solution (4.44 µL) of the compound to be used in the present invention was added at a final concentration of 5 µM (or two steps of 5 µM and 10 µM) (no stirring after addition). After addition of each compound, the mixtures were stood for 4 days at 37°C, 5% CO₂. An ATP reagent (44.4 µL) (CellTiter-Glo (registered trade mark) Luminescent Cell Viability Assay, manufactured by Promega) was added to and suspended in the culture medium on day 5, and the suspensions were stood for 15 min at room temperature. The luminescence intensity (RLU value) was measured by FlexStation3 (manufactured by Molecular Devices), and cell proliferation was evaluated.

The results are shown in the fifth table and the sixth table. The proliferation rate was calculated based on the control (cells added with DMSO without containing the compound to be used in the present invention) as the standard (100%). The values shown in the tables are average values of the results of two tests.

### [the fifth table]

**[Table 5]**

| compound | proliferation rate (%) | compound | proliferation rate (%) |
|---|---|---|---|
| k-1:H-1 | 233.26 | k-3:H-1 | 133.76 |
| k-1:H-2 | 147.27 | k-3:H-2 | 117.84 |
| k-1:H-3 | 168.98 | k-3:H-3 | 134.65 |
| k-1:H-4* | 105.07 | k-3:H-4 | 135.5 |
| k-1:H-5 | 154.19 | k-3:H-5 | 141.82 |
| k-1:H-6 | 125.68 | k-3:H-6 | 134.6 |
| k-1:H-7 | 236.46 | k-3:H-7 | 126.34 |
| k-1:H-9* | 95.96 | k-3:H-9 | 129.93 |
| k-2:H-1 | 184.61 | k-5:H-1 | 148.88 |
| k-2:H-2 | 131.13 | k-5:H-2 | 133.96 |
| k-2:H-3 | 139.33 | k-5:H-3 | 146.44 |
| k-2:H-4 | 128.2 | k-5:H-4 | 114.76 |
| k-2:H-5 | 158.56 | k-5:H-5 | 145.4 |
| k-2:H-6 | 116.79 | k-5:H-6 | 115.2 |
| k-2:H-7 | 201.93 | k-5:H-7 | 130.63 |
| k-2:H-9 | 131.36 | k-5:H-9 | 38.38 |

| | | | |
|---|---|---|---|
| *Reference Example | | | |

### [the sixth table]

**[Table 6]**

| compound | proliferation rate (%) (5 µM) | proliferation rate (%) (10 µM) |
|---|---|---|
| k-1:A-1 | 119.1 | 119.4 |
| k-1:A-2 | 118.8 | 129.4 |
| k-1:A-3 | 118.1 | 112.6 |
| k-1:A-5 | 113 | 124.3 |
| k-1:H-10 | 127.9 | 138.4 |

The value of proliferation rate is an average of two tests.

### [Experimental Example 2] Study of action of the compound to be used in the present invention on SKOV3 cell - 1

According to the method of patent document 1 (WO 2014/017513), a composition of McCoy's 5a medium (manufactured by Sigma-Aldrich) containing 0.015%(w/v) deacylated gellan gum (KELCOGEL CG-LA, manufactured by Sansho Co., Ltd.) and 15%(v/v) FBS, 100 ng/mL human HB-EGF (manufactured by PEPROTECH) was prepared by a homomixer. As a control, a non-addition medium composition not containing deacylated gellan gum was prepared. Then, human ovarian cancer cell line SKOV3 (manufactured by DS PHARMA BIO MEDICAL) was suspended in the above-mentioned medium composition added with deacylated gellan gum, and dispensed into the wells of a 384 well flat bottom Ultr-low Attachment surface microplate (manufactured by Corning Incorporated, #3827) at 1000 cells/40 µL/well (three-dimensional culture (3D)). In a single layer culture (2D), human ovarian cancer cell line SKOV3 was suspended in the above-mentioned medium composition not containing deacylated gellan gum, and dispensed into the wells of a 384 well flat bottom microplate (manufactured by Corning Incorporated, #3712) at 400 cells/40 µL per well. Each plate was cultured in a standing state in a CO₂ incubator (37°C, 5% CO₂). On day 1 of culture, the compound to be used in the present invention dissolved in dimethyl sulfoxide (DMSO) was added by 4.4 µL each at a final concentration of 0, 0.5, 1, 5, 10, 20 µM, and continuously cultured for 4 days. An ATP reagent (44.4 µL) (CellTiter-Glo (registered trade mark) Luminescent Cell Viability Assay, manufactured by Promega) was added to and suspended in the culture medium on day 5, and the suspension was stood for 15 min at room temperature. The luminescence intensity (RLU value) was measured by FlexStation3 (manufactured by Molecular Devices) and the luminescence value of the medium alone was subtracted to measure the number of viable cells. Compound non-addition RLU value (ATP measurement, luminescence intensity) was taken as 100%, and relative value with addition of each compound is shown in the seventh table. As a result of this test, k-1:H-7 and k-1:H-10 exhibited cell proliferation promoting effect at a wide concentration range under the conditions of three-dimensional culture (3D) of SKOV3 cells, and the SKOV3 cells formed spheres in the medium. On the other hand, when SKOV3 cells were cultured under single layer culture (2D), a favorable cell proliferation promoting effect was not observed even when k-1:H-7 and k-1:H-10 were added to the medium.

### [the seventh table]

**[Table 7]**

| Compound concentration (µM) | | 0 | 0.5 | 1 | 5 | 10 | 20 |
|---|---|---|---|---|---|---|---|
| 2D | k-1:H-7 | 100% | 110% | 105% | 91% | 69% | 47% |
| | k-1:H-10 | 100% | 111% | 108% | 102% | 99% | 66% |
| 3D | k-1:H-7 | 100% | 104% | 137% | 176% | 150% | 110% |
| | k-1:H-10 | 100% | 120% | 120% | 128% | 138% | 129% |

### [Experimental Example 3] Study of action of the compound to be used in the present invention on SKOV3 cell - 2

According to the method of patent document 1 (WO 2014/017513), a composition of McCoy's 5a medium (manufactured by Sigma-Aldrich) containing 0.015%(w/v) deacylated gellan gum (KELCOGEL CG-LA, manufactured by Sansho Co., Ltd.) and 15%(v/v) FBS, 30 ng/mL human EGF (manufactured by PEPROTECH) was prepared using FCeM-series Preparation Kit (manufactured by Wako Pure Chemical Industries, Ltd.). In a single layer culture, a non-addition medium composition not containing deacylated gellan gum was prepared. Then, human ovarian cancer cell line SKOV3 (manufactured by DS PHARMA BIO MEDICAL) was suspended in the above-mentioned medium composition added with deacylated gellan gum, and dispensed into the wells of a 384 well flat bottom Ultr-low Attachment surface microplate (manufactured by Corning Incorporated, #3827) at 1000 cells/36 µL/well (three-dimensional culture (3D)). In a single layer culture (2D), human ovarian cancer cell line SKOV3 was suspended in the above-mentioned medium composition not containing deacylated gellan gum, and dispensed into the wells of a 384 well flat bottom microplate (manufactured by Corning Incorporated, #3712) at 400 cells/36 µL per well. After dispensing, the compound to be used in the present invention dissolved in dimethyl sulfoxide (DMSO) was added by 4 µL each at a final concentration of 0, 1, 5, 10, 20 µM, and continuously cultured for 4 days. Each plate was cultured in a standing state in a CO₂ incubator (37°C, 5% CO₂) for 4 days. An ATP reagent (40 µL) (CellTiter-Glo (registered trade mark) Luminescent Cell Viability Assay, manufactured by Promega) was added to the culture medium on day 4, and the mixture was stirred by a plateshaker (manufactured by AS ONE Corporation, Micro plate mixer NS-P) at room temperature for 15 min. The luminescence intensity (RLU value) was measured by EnSpire (manufactured by Perkin Elmer) and the luminescence value of the medium alone was subtracted to measure the number of viable cells. Compound non-addition RLU value (ATP measurement, luminescence intensity) was taken as 100%, and relative value with addition of each compound is shown in the eighth table.

### [the eighth table]

**[Table 8]**

| Compound concentration (µM) | | 0 | 1 | 5 | 10 | 20 |
|---|---|---|---|---|---|---|
| 3D | k-1:I-1 | 100% | 106% | 111% | 123% | 141% |
| | k-1:B-1 | 100% | 127% | 181% | 177% | 158% |
| | k-1:D-1 | 100% | 140% | 174% | 168% | 136% |
| | k-1:J-1 | 100% | 116% | 170% | 186% | 173% |
| | k-1:D-2 | 100% | 116% | 129% | 143% | 126% |
| | k-1:I-10 | 100% | 100% | 135% | 181% | 197% |
| | k-1:N-1 | 100% | 101% | 134% | 169% | 186% |
| 2D | k-1:I-1 | 100% | 99% | 98% | 94% | 92% |
| | k-1:B-1 | 100% | 95% | 99% | 97% | 74% |
| | k-1:D-1 | 100% | 95% | 102% | 98% | 83% |
| | k-1:J-1 | 100% | 93% | 96% | 97% | 102% |
| | k-1:D-2 | 100% | 101% | 94% | 82% | 59% |
| | k-1:I-10 | 100% | 99% | 98% | 99% | 100% |
| | k-1:N-1 | 100% | 101% | 94% | 89% | 84% |

As a result of this test, k-1:I-1, k-1:B-1, k-1:D-1, k-1:J-1, k-1:D-2, k-1:I-10, and k-1:N-1 exhibited cell proliferation promoting effect at a wide concentration range under the conditions of three-dimensional culture (3D) of SKOV3 cells, and the SKOV3 cells formed spheres in the medium. On the other hand, when SKOV3 cells were cultured under single layer culture (2D), a favorable cell proliferation promoting effect was not observed even when k-1:I-1, k-1:B-1, k-1:D-1, k-1:J-1, k-1:D-2, k-1:I-10, and k-1:N-1 were added to the medium.

### [Experimental Example 4] Study of action of the compound to be used in the present invention on small intestine organoid

About 20 cm of the mouse small intestine was excised, and the adipose tissue and vascular tissue were removed on ice. The contents were sufficiently washed with PBS, cut open with scissors, and fragmented to about 2 mm. They were washed 20 times with 15 mL of PBS and the supernatant was removed. 25 mL of Gentle Cell Dissociation Reagent (manufactured by STEMCELL) was added, and the mixture was incubated at room temperature for 15 min while stirring at 20 rpm. The supernatant was removed, 10 mL of cold 0.1% BSA/PBS was added to the precipitate, and pipetting was performed three times. The supernatant was filtered with a 70 µm cell strainer (manufactured by BD Bioscience), and the filtrate was collected. Similarly, addition of 0.1% BSA/PBS to the remaining precipitate, pipetting, and filtration were repeated three times, and thus series of the four filtrates were prepared. Each filtrate was centrifuged at 290 g for 5 min at 4°C. After removing the supernatant, the cells were resuspended in 10 mL of cold 0.1% BSA/PBS and centrifuged at 200 g for 3 min at 4°C. The supernatant was removed, 10 mL of DMEM/F12 (manufactured by Wako Pure Chemical Industries, Ltd.) was added and the cells were suspended. 1 mL was separated and observed under a microscope. A filtrate having a sufficient intestinal fragment (Crypt) was selected, and the number of Crypt was counted using a hemocytometer. Cold Matrigel (registered trade mark) Matrix GFR(manufactured by Corning) in an equal amount to the cell suspension was added, mixed, and quickly dispensed into a 24-well plate warmed to 37°C at 500 crypts/50 µL/well. The plate was allowed to stand at 37°C for 10 min to complete gelation. 750 µL of IntestiCult (registered trademark) Organoid Growth Medium (manufactured by STEMCELL TECHNOLOGIES) was added to each well, the compound to be used in the present invention dissolved in DMSO was further added at a final concentration of 5 µM. As a control, a well with no addition of the compound was prepared. After culturing for 7 days, the number and diameter of the formed small intestinal organoids were measured under a microscope, and the comparison results with no addition (control) are shown in the ninth table. As a result of this test, it was clarified that k-1:H-7 promotes organoid formation in mouse small intestinal organoid culture. At this time, the diameter of the organoid did not change much, and the addition of k-1:H-7 improved the organoid formation rate from Crypt.

### [the ninth table]

**[Table 9]**

| | control | k-1:H-7 |
|---|---|---|
| organoid number (relative value) | 1 | 2.5 |
| organoid diameter (µm) | 130±18 | 153±21 |

### [Experimental Example 5] Action of the compound to be used in the present invention on various human cancer cells - 1

Various human-derived cancer cells were precultured (single layer culture) in respective media shown below. Human uterus cervix cancer-derived cell line HeLa (manufactured by American Type Culture Collection (hereinafter indicated as ATCC), 10% fetal bovine serum (FBS, manufactured by Corning)-containing Dulbecco's Modified Eagle's Medium (hereinafter abbreviated as DMEM) (manufactured by Wako Pure Chemical Industries, Ltd.)), human malignant melanoma-derived cell line A375 (manufactured by ATCC, 10% FBS-containing DMEM), human epithelial-like cell cancer-derived cell line A431 (manufactured by ATCC, 10% FBS and 1% MEM non-essential amino acid solution (MEM Non-Essential Amino Acids solution (hereinafter abbreviated as NEAA) (manufactured by Wako Pure Chemical Industries, Ltd.))-containing Eagle's Minimum Essential Medium (hereinafter abbreviated as EMEM) (manufactured by Wako Pure Chemical Industries, Ltd.)), human stomach adenocarcinoma-derived cell line AGS (manufactured by DS PHARMA BIO MEDICAL, 10% FBS-containing Ham's F-12 (manufactured by Wako Pure Chemical Industries, Ltd.)), human prostate cancer-derived cell line LNCaP clone FGC (manufactured by ATCC, 10% FBS-containing RPMI1640 (manufactured by Wako Pure Chemical Industries, Ltd.)), human colon adenocarcinoma-derived cell line HCT116 (manufactured by DS PHARMA BIO MEDICAL, 10% FBS-containing McCoy's 5A Medium (manufactured by Sigma-Aldrich)), human alveolar basal epithelial adenocarcinoma-derived cell line A549 (manufactured by DS PHARMA BIO MEDICAL, 10% FBS-containing DMEM), human prostate cancer-derived cell DU145 (manufactured by ATCC, 10% FBS-containing EMEM). The above-mentioned cells in the logarithmic growth phase were washed with PBS, a 0.25w/v% trypsin-1 mmol/L EDTA (ethylenediaminetetraacetic acid) solution (manufactured by Wako Pure Chemical Industries, Ltd.) was added, and adherent cells were detached by incubating at 37°C for 1 - 5 min. Each medium was added and the mixture was centrifuged. After resuspension in the same medium, each cell was recovered.

The aforementioned various cells were suspended in respective deacylated gellan gum-containing or not containing media (deacylated gellan gum concentration was 0.015 w/v%, and 0.020 w/v% for RPMI1640 alone), and seeded in a 96 well low attachment U-bottom plate (manufactured by Corning, deacylated gellan gum-free medium), or a low attachment flat bottom plate (manufactured by Corning, deacylated gellan gum-containing medium) at a cell concentration of 1000 - 12000 cells/135 µL/well (all 3D culture). After static culture overnight in 37°C, 5% CO₂ incubator, a DMSO solution of the compound to be used in the present invention was added to each medium at a final concentration of 5 µM or 10 µM. The amount of each compound solution to be added was 15 µL/well. As a control, a DMSO solution dissolved in a medium was added (DMSO final concentration 0.1%). After continuously culturing in an incubator at 37°C, 5% CO₂ for 4 days, WST-8 solution (manufactured by DOJINDO LABORATORIES) was added at 15 µL/well, and the mixture was reacted in the same incubator for 1 - 2 hr. The absorbance at 450 nm was measured by an absorption spectrometer (manufactured by Molecular Devices, SPECTRA MAX 190) and the absorbance of the medium alone was subtracted to measure the number of viable cells. Furthermore, the absorbance of compound non-addition (control) was taken as 100%, and relative value with addition of each compound was calculated. When compared with the compound no-addition (control), one showing a value of not more than 119% as -, one showing a value of not less than 120% as ○, and one showing a value of not less than 150% as ⊙ are shown in the tenth table.

### [the tenth table]

**[Table 10]**

| cell name | low attachment U-bottom plate | | | | low attachment flat bottom plate | | | |
|---|---|---|---|---|---|---|---|---|
| | k-1:H-7 | | k-1:H-10 | | k-1:H-7 | | k-1:H-10 | |
| | 5 µM | 10 µM | 5 µM | 10 µM | 5 µM | 10 µM | 5 µM | 10 µM |
| HeLa | - | - | - | - | - | ○ | - | - |
| A375 | - | ○ | - | ○ | ○ | - | - | - |
| A431 | ⊙ | ⊙ | - | - | ⊙ | ⊙ | - | - |
| AGS | ○ | ○ | ○ | - | - | ○ | - | - |
| LNCa P | ⊙ | ○ | ○ | ⊙ | - | - | - | - |
| HCT1 16 | - | ○ | - | - | ○ | ⊙ | - | - |
| A549 | - | ○ | - | - | ⊙ | ⊙ | ○ | ○ |
| DU14 5 | ○ | ⊙ | ○ | - | ⊙ | ⊙ | - | ○ |

As a result of this test, it was clarified that k-1:H-7 and k-1:H-10 promoted proliferation activity in a plurality of cancer cell lines under three-dimensional conditions. At this time, the cancer cell lines formed spheres using either the low attachment U-bottom plate or the low attachment flat bottom plate.

### [Experimental Example 6] Action of the compound to be used in the present invention on various human cancer cells - 2

Various human-derived cancer cells were precultured (single layer culture) in respective media shown below. Human ovarian cancer cell line SKOV3 (manufactured by DS PHARMA BIO MEDICAL, 15% fetal bovine serum (FBS, manufactured by Corning)-containing McCoy's 5a medium (manufactured by Sigma-Aldrich)), human alveolar basal epithelial adenocarcinoma-derived cell line A549 (manufactured by DS PHARMA BIO MEDICAL, 10% FBS-containing DMEM (manufactured by Wako Pure Chemical Industries, Ltd.)), human uterus cervix cancer-derived cell line HeLa (manufactured by ATCC, 10% FBS-containing DMEM), human malignant melanoma-derived cell line A375 (manufactured by ATCC, 10% FBS-containing DMEM), human epithelial-like cell cancer-derived cell line A431 (manufactured by ATCC, 10% FBS and 1% MEM non-essential amino acid solution (MEM NEAA, manufactured by Wako Pure Chemical Industries, Ltd.)-containing EMEM (manufactured by Wako Pure Chemical Industries, Ltd.)), human stomach adenocarcinoma-derived cell line AGS (manufactured by DS PHARMA BIO MEDICAL, 10% FBS-containing Ham's F-12 (manufactured by Wako Pure Chemical Industries, Ltd.)), human prostate cancer-derived cell DU145 (manufactured by ATCC, 10% FBS-containing EMEM). The above-mentioned cells in the logarithmic growth phase were washed with PBS, a 0.25w/v% trypsin-1 mmol/L EDTA (ethylenediaminetetraacetic acid) solution (manufactured by Wako Pure Chemical Industries, Ltd.) was added, and adherent cells were detached by incubating at 37°C for 1 - 5 min. Each medium was added and the mixture was centrifuged. After resuspension in the same medium, each cell was recovered.

The aforementioned various cells were suspended in respective deacylated gellan gum-containing or not containing media (deacylated gellan gum concentration was 0.015 w/v%), and seeded in a 96 well low attachment U-bottom plate (manufactured by Corning, #4520, deacylated gellan gum-free medium), or a low attachment flat bottom plate (manufactured by Corning, #3474, deacylated gellan gum-containing medium) at a cell concentration of 700 - 12000 cells/90 µL/well (all 3D culture). Successively, the compound to be used in the present invention dissolved in DMSO was added to each medium at a final concentration of 5 µM or 10 µM. The amount of each compound solution to be added was 10 µL/well. As a control, a DMSO solution dissolved in a medium was added (DMSO final concentration 0.1%). After culturing in an incubator at 37°C, 5% CO₂ for 4 days, an ATP reagent (40 µL) (CellTiter-Glo (registered trade mark) Luminescent Cell Viability Assay, manufactured by Promega) was added to the culture medium on day 4. After stirring by a plate shaker (manufactured by AS ONE, Micro plate mixer NS-P) at room temperature for 15 min, the luminescence intensity (RLU value) was measured by EnSpire (manufactured by Perkin Elmer) and the luminescence value of the medium alone was subtracted to measure the number of viable cells. The RLU value (ATP measurement, luminescence intensity) of compound non-addition (control) was taken as 100%, and relative value with addition of each compound was calculated. When compared with the compound no-addition (control), one showing a value of not more than 119% as -, one showing a value of not less than 120% as ○, and one showing a value of not less than 150% as ⊙ are shown in the eleventh table and the twelfth table. Unperformed test is left blank.

### [the eleventh table]

**[Table 11]**

| low attachment U-bottom plate | | | | | | | | |
|---|---|---|---|---|---|---|---|---|
| cell line | k-1:I-1 | | k-1:B-1 | | k-1:D-1 | | k-1:J-1 | |
| | 5 µM | 10 µM | 5 µM | 10 µM | 5 µM | 10 µM | 5 µM | 10 µM |
| SKOV3 | ○ | ○ | ⊙ | ⊙ | ⊙ | ⊙ | ⊙ | ⊙ |
| A549 | - | - | ⊙ | ⊙ | ⊙ | ⊙ | ⊙ | ⊙ |
| HeLa | - | - | ⊙ | ⊙ | | | | |
| A375 | ○ | ○ | ⊙ | ⊙ | | | | |
| A431 | ○ | ⊙ | ⊙ | ⊙ | | | | |
| AGS | - | ○ | ⊙ | ⊙ | | | | |
| DU145 | - | ○ | ⊙ | ⊙ | | | | |

### [the twelfth table]

**[Table 12]**

| low attachment flat bottom plate | | | | | | | | |
|---|---|---|---|---|---|---|---|---|
| cell line | k-1:I-1 | | k-1:B-1 | | k-1:D-1 | | k-1:J-1 | |
| | 5 µM | 10 µM | 5 µM | 10 µM | 5 µM | 10 µM | 5 µM | 10 µM |
| SKOV3 | - | ○ | ⊙ | ⊙ | ⊙ | ⊙ | ⊙ | ⊙ |
| A549 | - | ○ | ⊙ | ⊙ | ⊙ | ⊙ | ⊙ | ⊙ |
| HeLa | - | - | ⊙ | ⊙ | | | | |
| A375 | - | - | ○ | ○ | | | | |
| A431 | - | ⊙ | ⊙ | ⊙ | | | | |
| AGS | ○ | ○ | ○ | ⊙ | | | | |
| DU145 | ○ | ⊙ | ⊙ | ⊙ | | | | |

As a result of this test, it was clarified that k-1:I-1, k-1:B-1, k-1:D-1, and k-1:J-1 promoted proliferation activity in a plurality of cancer cell lines under three-dimensional conditions. At this time, the cancer cell lines formed spheres using either the low attachment U-bottom plate or the low attachment flat bottom plate.

### [Experimental Example 7] Action of the compound to be used in the present invention on MDCK cells - 1

Canine kidney renal tubule epithelial cells (MDCK cells, manufactured by DS PHARMA BIO MEDICAL) were precultured in EMEM medium containing 10% FBS and 1% NEAA. Cold Matrigel (registered trade mark) Matrix GFR (manufactured by Corning) was spread on a 24-well plate by 50 µL and fixed by incubating at 37°C for 15 min. The aforementioned MDCK cells were suspended in a medium at a concentration of 20000 cells/mL, cold Matrigel (registered trade mark) Matrix GFR was added at 20 µL/mL and seeded at 1 mL/well. The compound to be used in the present invention dissolved in a medium at a final concentration of 10 µM was added, and the cells were cultured for 7 days under the conditions of 37°C, 5% CO₂ in an incubator. As a control (compound no-addition), DMSO was added at a final concentration of 0.1%. Seven days later, the cells were washed with PBS (1 mL/well), 4% paraformaldehyde/PBS (manufactured by Wako Pure Chemical Industries, Ltd.) was added (1 mL/well) and fixed at room temperature for 20 min. Thereafter, the supernatant was removed, IF buffer (0.2% Triton X-100 (manufactured by Sigma-Aldrich), 0.05% Tween 20 (manufactured by Sigma-Aldrich)-containing PBS) were added at 1 mL/well, stood for 30 min and removed. Penetration buffer (0.5% Triton X-100 (manufactured by Sigma-Aldrich)/PBS) was added at 1 mL/well and incubated at room temperature for 30 min. The supernatant was removed, washed 3 times every 5 min with IF buffer, blocking buffer (1% BSA (manufactured by Sigma-Aldrich)/IF buffer) was added at 0.5 mL/well and incubated for 30 min. The supernatant was removed, an anti-β catenin antibody (manufactured by BD Bioscience) diluted 100-fold with blocking buffer was added at 250 µL/well,and the cells were incubated overnight at 4°C in shading. The next day, the cells were washed 3 times every 5 min with IF buffer, the secondary antibody (Alexa Fluor 555, manufactured by Thermo Fisher Scientific) and Phalloidin (Alexa Fluor 488, manufactured by Thermo Fisher Scientific), each diluted 250-fold with blocking buffer, were added at 250 µL/well,and the cells were incubated at room temperature in shading for 60 min. After washing 3 times every 5 min with IF buffer, VECTASHIELD Mounting Medium with DAPI (manufactured by Vector Laboratories) was added dropwise, and the cells were observed and analyzed with a fluorescence microscope (ArrayScan, manufactured by Thermo Fisher Scientific).

As a result of this test, it was clarified that when MDCK cells form Cysts having lumens on Matrigel, the area per one Cyst increased by 20% or more in the presence of compound k-1:H-7 based on the Control target as 100. The results thereof are shown in the thirteenth table.

### [the thirteenth table]

**[Table 13]**

| | | |
|---|---|---|
| | control | k-1:H-7 |
| Cyst area (relative value) | 100 | 124 |

### [Experimental Example 8] Action of the compound to be used in the present invention on MDCK cells - 2

Canine kidney renal tubule epithelial cells (MDCK cells, manufactured by DS PHARMA BIO MEDICAL) were precultured in EMEM medium containing 10% FBS and 1% NEAA. Cold Matrigel (registered trade mark) Matrix GFR (manufactured by Corning) was spread on a 24-well plate by 50 µL and fixed by incubating at 37°C for 15 min. The aforementioned MDCK cells were suspended in a medium at 10000 cells/mL, cold Matrigel (registered trade mark) Matrix GFR was added at 20 µL/mL and seeded at 1 mL/well. The compound to be used in the present invention dissolved in a medium at a final concentration of 5 µM or 10 µM was added, and the cells were cultured for 6 days under the conditions of 37°C, 5% CO₂ in an incubator. As a control (compound no-addition), DMSO was added at a final concentration of 0.1%. Six days later, the size and number of Cysts formed were measured using Cell³iMager (manufactured by Screen Inc.). The proportion of the Cysts of 70 µm or more in the entire Cysts is shown in the fourteenth table.

### [the fourteenth table]

**[Table 14]**

| | control | k-1:I-1 | | k-1:B-1 | |
|---|---|---|---|---|---|
| Dose | - | 5 µM | 10 µM | 5 µM | 10 µM |
| proportion of Cysts of 70 µm or more | 35% | 39% | 43% | 72% | 68% |

The cells were washed with PBS (1 mL/well), 4% paraformaldehyde/PBS (manufactured by Wako Pure Chemical Industries, Ltd.) was added (1 mL/well) and fixed at room temperature for 20 min. Thereafter, the supernatant was removed, IF buffer (0.2% Triton X-100 (manufactured by Sigma-Aldrich), 0.05% Tween 20 (manufactured by Sigma-Aldrich)-containing PBS) were added at 1 mL/well, stood for 30 min and removed. Penetration buffer (0.5% Triton X-100 (manufactured by Sigma-Aldrich)/PBS) was added at 1 mL/well and incubated at room temperature for 30 min. The supernatant was removed, washed 3 times every 5 min with IF buffer, blocking buffer (1% BSA (manufactured by Sigma-Aldrich)/IF buffer) was added at 0.5 mL/well and incubated for 30 min. The supernatant was removed, an anti-β catenin antibody (manufactured by BD Bioscience) diluted 100-fold with blocking buffer was added at 250 µL/well, and the cells were incubated at room temperature for 60 min. The cells were washed 3 times every 5 min with IF buffer, the secondary antibody (Alexa Fluor 555, manufactured by Thermo Fisher Scientific) and Phalloidin (Alexa Fluor 488, manufactured by Thermo Fisher Scientific), each diluted 250-fold with blocking buffer, were added at 250 µL/well,and the cells were incubated at room temperature in shading for 60 min. After washing 3 times every 5 min with IF buffer, VECTASHIELD Mounting Medium with DAPI (manufactured by Vector Laboratories) was added dropwise, and the cells were observed with a confocal fluorescence microscope (FV1200 IX83, manufactured by Olympus Corporation).

As a result of this test, it was clarified that when MDCK cells form Cysts having lumens on Matrigel, the proportion of Cysts of 70 µm or more in the entire Cysts increased in the presence of compound k-1:I-1, k-1:B-1 (Table 13). Also, as a result of observation with a confocal fluorescence microscope, it was confirmed that a normal Cyst was formed in the presence of compound k-1:I-1, or k-1:B-1 (Fig. 1).

### [Experimental Example 9] Action of the composition of the present invention on human mesenchymal stem cells - 1

Human mesenchymal stem cells (hMSC, manufactured by TOYOBO) were precultured by a single layer culture method (2D) using an MF-medium mesenchymal stem cell proliferation medium (manufactured by TOYOBO). In three-dimensional culture method (3D), hMSC were suspended in a medium composition added with deacylated gellan gum, and seeded in a 6-well flat bottom Ultr-low Attachment surface plate (manufactured by Corning Incorporated, #3471) at 1.2×10⁵ cells/2 mL/well. In 2D, hMSC were suspended in a deacylated gellan gum-free medium composition, and seeded in a 6-well flat bottom plate (manufactured by Corning Incorporated, #3516) at 4.0×10⁴ cells/2 mL/well. Continuously, a solution of the compound to be used in the present invention dissolved in a medium at a final concentration of 10 µM was added, and the cells were cultured for 7 days in an incubator at 37°C, 5% CO₂. As a control, DMSO was added at a final concentration of 0.1%. Seven days later, in the three-dimensional culture method, cells were collected from the wells, washed with PBS, and the supernatant was removed. Then, a trypsin 0.25 w/v% trypsin-1 mmol/L EDTA (ethylenediaminetetraacetic acid) solution (manufactured by Wako Pure Chemical Industries, Ltd.) was added and the cells were incubated at 37°C for 2 - 5 min to disperse the spheres into single cells. The medium was added, and the mixture was centrifuged to remove the supernatant. Thereafter, the cells were resuspended in the same medium, a part was suspended in Trypan Blue (manufactured by Wako Pure Chemical Industries, Ltd.), and the number of viable cells was counted using automatic cell counter TC20 (manufactured by BIO-RAD).

In a single layer culture method, the cells were washed with PBS, 0.25 w/v% trypsin-1 mmol/L EDTA solution was added, and the cells were detached by incubating at 37°C for 2 - 5 min. The medium was added, the mixture was centrifuged and the supernatant was removed. Thereafter, the cells were resuspended in the same medium, a part was suspended in Trypan Blue (manufactured by Wako Pure Chemical Industries, Ltd.) and the number of viable cells was counted using TC-20 (manufactured by BIO-RAD).

CD34 antibody (APC, manufactured by BD Bioscience), CD73 (BV421, manufactured by BD Bioscience), CD29 (BB515, manufactured by BD Bioscience) were added to the above-mentioned cell suspension, and the mixture was incubated on ice for 30 min. The cells were washed twice with 2% SM buffer (2% FBS/PBS), SM containing 1 µg/mL of Propidium Iodide (PI) was added, and the mixture was analyzed with FACSAria (manufactured by Becton Dickinson). The cell number with compound non-addition (control) was taken as 1, and relative value with addition of each compound is calculated in the fifteenth table. The positive rates or negative rates of CD34, CD73, and CD29 of cells after three-dimensional culture (3D) under the conditions with no addition or addition of the compound are shown in the sixteenth table.

### [the fifteenth table]

**[Table 15]**

| cell proliferation rate (relative value) | k-1:H-7 | k-1:H-10 |
|---|---|---|
| 2D | 1.9 | 1.3 |
| 3D | 4 | 1.7 |

### [the sixteenth table]

**[Table 16]**

| differentiation marker | no addition | k-1:H-7 | k-1:H-10 |
|---|---|---|---|
| CD34+(negative rate) | 0.9% | 3% | 0.7% |
| CD29+(positive rate) | 94.6% | 94.6% | 87.6% |
| CD73+(positive rate) | 99.9% | 100% | 99.1% |

As a result of this test, k-1:H-7 and k-1:H-10 showed an effect of increasing the number of hMSC cells under two-dimensional and three-dimensional cell culture conditions. Under three-dimensional cell culture conditions, hMSC formed spheres. At that time, the negative rates of the cell surface markers CD34, CD73, and CD29 did not change by the addition of the compound (the sixteenth table). From the above results, it was clarified that k-1:H-7 and k-1:H-10 promote cell proliferation while maintaining undifferentiated state of hMSCs.

### [Experimental Example 10] Action of the compound to be used in the present invention on human mesenchymal stem cell - 2

Bone marrow-derived human mesenchymal stem cells (BM-hMSC, manufactured by PromoCell) were precultured by a single layer culture method (2D) using a mesenchymal stem cell proliferation medium (manufactured by PromoCell). In three-dimensional culture method (3D), hMSC was suspended in a medium composition added with deacylated gellan gum, and seeded in a 6-well flat bottom Ultr-low Attachment surface plate (manufactured by Corning Incorporated, #3474) at 6000 cells/90 µL/well. In the three-dimensional culture method using EZSPHERE (EZSPHERE), hMSC was suspended in a deacylated gellan gum-free medium composition, and seeded in a 96-well EZSPHERE plate (manufactured by AGC TECHNO GLASS CO., LTD., #4860-900) at 2000 cells/90 µL/well. In 2D, hMSC was suspended in a deacylated gellan gum-free medium composition, and seeded in a 96-well flat bottom plate (manufactured by Corning Incorporated, #3585) at 2000 cells/90 µL/well. Continuously, the compound to be used in the present invention dissolved in a medium at a final concentration of 1 µM, 5 µM, 10 µM or 20 µM was added at 10 µL/well, and the cells were cultured for 4 days in an incubator at 37°C, 5% CO₂. As a control, DMSO was added at a final concentration of 0.1%. Four days later, an ATP reagent (100 µL) (CellTiter-Glo (registered trade mark) Luminescent Cell Viability Assay, manufactured by Promega) was added to the culture medium, and the mixture was stirred by a plateshaker (manufactured by AS ONE Corporation, Micro plate mixer NS-P) at room temperature for 2 min, and stood at room temperature for 10 min. The luminescence intensity (RLU value) was measured by Enspire (manufactured by Perkin Elmer) and the luminescence value of the medium alone was subtracted to measure the number of viable cells. Compound non-addition RLU value (ATP measurement, luminescence intensity) was taken as 100%, and relative value with addition of each compound was calculated and the results thereof are shown in the seventeenth table.

### [the seventeenth table]

**[Table 17]**

| Compound concentration (µM) | | 0 | 1 | 5 | 10 | 20 |
|---|---|---|---|---|---|---|
| 3D | k-1:I-1 | 100% | 100% | 123% | 150% | 177% |
| | k-1:B-1 | 100% | 139% | 298% | 418% | 420% |
| | k-1:D-1 | 100% | 170% | 640% | 771% | 688% |
| | k-1:J-1 | 100% | 132% | 330% | 579% | 773% |
| EZSPHERE | k-1:I-1 | 100% | 101% | 120% | 144% | 163% |
| | k-1:B-1 | 100% | 154% | 247% | 297% | 278% |
| | k-1:D-1 | 100% | 169% | 310% | 339% | 275% |
| | k-1:J-1 | 100% | 149% | 241% | 321% | 231% |
| 2D | k-1:I-1 | 100% | 107% | 98% | 104% | 107% |
| | k-1:B-1 | 100% | 109% | 125% | 158% | 164% |
| | k-1:D-1 | 100% | 111% | 125% | 112% | 92% |
| | k-1:J-1 | 100% | 107% | 121% | 146% | 157% |

As a result of this test, it was clarified that k-1:I-1, k-1:B-1, k-1:D-1, and k-1:J-1 promoted proliferation activity of BM-hMSC under three-dimensional cell culture conditions. Under three-dimensional cell culture conditions, BM-hMSC formed spheres. In addition, it was clarified that k-1:B-1 and k-1:J-1 promoted proliferation activity of hMSC also under two-dimensional cell culture conditions.

### [Experimental Example 11] Action of the compound to be used in the present invention on fibroblast strain C3H10T1/2

Mouse embryonic fibroblast C3H10T1/2 (manufactured by DS PHARMA BIO MEDICAL) was cultured in a BME medium (manufactured by Thermo Fisher Scientific) containing 10(v/v)% FBS (manufactured by Corning) and the L-glutamine-penicillin-streptomycin stabilizing solution (manufactured by Sigma-Aldrich). The above-mentioned cells in the logarithmic growth phase were washed with PBS, a 0.25w/v% trypsin-1 mmol/L EDTA (ethylenediaminetetraacetic acid) solution (manufactured by Wako Pure Chemical Industries, Ltd.) was added, and the cells were detached by incubating at 37°C for 3 min. Each medium was added, the mixture was centrifuged, and the supernatant was removed.

The aforementioned various cells were suspended in respective deacylated gellan gum-containing or not containing media (deacylated gellan gum concentration was 0.015 w/v%), and seeded in a 96 well low attachment U-bottom plate (manufactured by Corning, #4520, deacylated gellan gum-free medium) at a cell concentration of 700 - 2000 cells/90 µL/well (3D culture). After seeding, the compound to be used in the present invention dissolved in DMSO was added to each medium at a final concentration of 1 µM or 5 µM. The amount of each compound solution to be added was 10 µL/well. As a control, a DMSO solution dissolved in a medium was added (DMSO final concentration 0.1%). After culturing in an incubator at 37°C, 5% CO₂ for 4 days, an ATP reagent (100 µL) (CellTiter-Glo (registered trade mark) Luminescent Cell Viability Assay, manufactured by Promega) was added to the culture medium. After stirring by a plate shaker (manufactured by AS ONE, Micro plate mixer NS-P) at room temperature for 15 min, the luminescence intensity (RLU value) was measured by EnSpire (manufactured by Perkin Elmer) and the luminescence value of the medium alone was subtracted to measure the number of viable cells. The RLU value (ATP measurement, luminescence intensity) of compound non-addition (control) was taken as 100%, and relative value with addition of each compound was calculated. When compared with the compound no-addition (control), one showing a value of not more than 119% as -, one showing a value of not less than 120% as ○, and one showing a value of not less than 150% as ⊙ are shown in the eighteenth table. Unperformed test is left blank.

### [the eighteenth table]

**[Table 18]**

| low attachment U-bottom plate | | | | | | | | |
|---|---|---|---|---|---|---|---|---|
| cell line | k-1:I-1 | | k-1:B-1 | | k-1:D-1 | | k-1:J-1 | |
| | 1 µM | 5 µM | 1 µM | 5 µM | 1 µM | 5 µM | 1 µM | 5µM |
| C3H10T1/2 | ○ | ○ | ⊙ | ⊙ | ○ | | ⊙ | |

As a result of this test, it was clarified that k-1:I-1, k-1:B-1, k-1:D-1, and k-1:J-1 promoted proliferation activity in fibroblast under three-dimensional conditions. At this time, the fibroblast formed a sphere when a low attachment U-bottom plate was used.

### [Experimental Example 12] Action of the compound to be used in the present invention on cells cultured by hanging drop method

Human epithelial-like cell cancer-derived cell line A431 (manufactured by ATCC) was cultured using 10% FBS and 1% MEM non-essential amino acid solution (MEM NEAA, manufactured by Wako Pure Chemical Industries, Ltd.)-containing EMEM (manufactured by Wako Pure Chemical Industries, Ltd.)). In addition, bone marrow-derived human mesenchymal stem cells (BM-hMSC, manufactured by PromoCell) were cultured using a mesenchymal stem cell proliferation medium (manufactured by PromoCell). Each of the above-mentioned cells in the logarithmic growth phase was washed with PBS, a 0.25w/v% trypsin-1 mmol/L EDTA (ethylenediaminetetraacetic acid) solution (manufactured by Wako Pure Chemical Industries, Ltd.) was added, and the cells were detached by incubating at 37°C for 3 min. Each medium was added, the mixture was centrifuged, and the supernatant was removed.

Continuously, each cell was suspended in the above-mentioned medium to 100000 cells/2 mL, and the compound to be used in the present invention dissolved in DMSO was further added to the medium at a final concentration of 5 µM. The cell suspension was seeded by 10 µL in 15 drops on the back surface of the lid of a 3.5 cm dish (manufactured by Falcon, #351008) to form droplets. At this time, as a control, a medium supplemented with DMSO (final concentration of DMSO: 0.05%) was seeded by 10 µL in 15 drops. The lid was returned to a 3.5 cm dish added with 2 mL of PBS, and cultured for 2 days in an incubator at 37°C, 5% CO₂. The cultured droplets were collected in a 1.5 mL tube, and the medium was added to a final volume of 150 µL. An ATP reagent (150 µL) (CellTiter-Glo (registered trade mark) Luminescent Cell Viability Assay, manufactured by Promega) was further added to and suspended in the medium, and the suspension was stood for 10 min at room temperature. The luminescence intensity (RLU value) was measured by Enspire (manufactured by Perkin Elmer) and the luminescence value of the medium alone was subtracted to measure the number of viable cells. Compound non-addition RLU value (ATP measurement, luminescence intensity) was taken as 100%, and relative value with addition of each compound is shown in the nineteenth table and the twentieth table.

### [the nineteenth table]

**[Table 19]**

| A431 cell | control | k-1: H-1 | k-1: H-7 | k-1: B-1 |
|---|---|---|---|---|
| cell number (relative value) | 100% | 252% | 232% | 227% |

### [the twentieth table]

**[Table 20]**

| BM-hMSC | control | k-1: H-1 | k-1: H-7 | k-1: B-1 |
|---|---|---|---|---|
| cell number (relative value) | 100% | 186% | 206% | 223% |

As a result of this test, k-1:H-1, k-1:H-7, and k-1:B-1 showed an effect of increasing the number of A431 cell line and bone marrow-derived human mesenchymal stem cells even under the condition of three-dimensional cell culture by the hanging drop method. At this time, the A431 cell line and bone marrow-derived human mesenchymal stem cells formed a sphere in the droplets by the hanging drop method.

### [Experimental Example 13] Action of the compound of the present invention on human pluripotent stem cell (hiPS cell)

hiPS cell line 253G1 (purchased from RIKEN) was cultured on a dish coated with vitronectin VTN-N (manufactured by Thermo Fisher Scientific) using mTeSR1 (registered trade mark) medium (manufactured by STEMCELL Technologies). The above-mentioned cells in the proliferation phase were washed with PBS (manufactured by Fujifilm Corporation Wako Pure Chemical Industries, Ltd.), TrypLE Select (registered trade mark) (manufactured by Thermo Fisher Scientific) was added and the mixture was incubated at 37°C for 3 min to remove the detaching solution. The medium was added and the cells were detached by pipetting. Thereafter, the supernatant was removed by centrifugation.

The aforementioned cells were suspended in a medium containing 10 µM Y-27632 (manufactured by Fujifilm Corporation Wako Pure Chemical Industries, Ltd.) and seeded in a 96-well EZSPHERE plate (manufactured by AGC TECHNO GLASS CO., LTD., #4860-900) at a cell concentration of 10000 cells/200 µL/well. After seeding, the compound to be used in the present invention dissolved in diluted DMSO was added to each medium at a final concentration of 5 µM. The amount of each compound solution to be added was 2 µL/well. As a control, a DMSO solution dissolved in a medium was added (DMSO final concentration 0.05%). The medium was exchanged by half the volume every day, and each compound solution was also added to make the compound concentration constant. After culturing in an incubator at 37°C, 5% CO₂ for 3 days, the culture supernatant (100 µL) was removed, and an ATP reagent (100 µL) (CellTiter-Glo (registered trade mark) Luminescent Cell Viability Assay, manufactured by Promega) was added to the remaining culture medium. After stirring by a plate shaker (manufactured by AS ONE, Micro plate mixer NS-P) at room temperature for 15 min, 150 µL was transferred to a 96-well plate flat bottom white/transparent (manufactured by Falcon), the luminescence intensity (RLU value) was measured by EnSpire (manufactured by Perkin Elmer) and the luminescence value of the medium alone was subtracted to measure the number of viable cells. The RLU value (ATP measurement, luminescence intensity) of DMSO addition was taken as 100%, and relative value with addition of each compound is shown in the twenty-first table.

### [the twenty-first table]

**[Table 21]**

| compound | relative value (%) |
|---|---|
| k-1:H-1 | 166% |
| k-1:H-7 | 194% |
| k-1:B-1 | 193% |
| k-1:D-1 | 207% |
| k-1:J-1 | 227% |
| k-1:I-10 | 194% |

As a result of this test, it was clarified that k-1:H-1, k-1:H-7, k-1:B-1, k-1:D-1, k-1:J-1, and k-1:I-10 promoted proliferation activity in human pluripotent stem cells under three-dimensional conditions.

### [Experimental Example 14] Action of the compound of the present invention on vascular endothelial cell

Human umbilical vein endothelial cells (manufactured by PromoCell) were precultured in Endothelial Cell proliferation Medium (manufactured by PromoCell) medium (single layer culture). The above-mentioned cells in the logarithmic growth phase were washed with PBS, DetachKit (manufactured by PromoCell) was added, and adherent cells were detached by incubating at 37°C for 3 min. The medium was added and the mixture was centrifuged and resuspended in the same medium.

The aforementioned cells were suspended in respective deacylated gellan gum-containing or not containing media (deacylated gellan gum concentration was 0.015 w/v%), and seeded in a 96 well low attachment U-bottom plate (manufactured by Corning, #4520, deacylated gellan gum-free medium), or a low attachment flat bottom plate (manufactured by Corning, #3474, deacylated gellan gum-containing medium) at a cell concentration of 700 - 2000 cells/90 µL/well (all 3D culture). Continuously, the compound to be used in the present invention dissolved in DMSO was added to each medium at a final concentration of 5 µM or 10 µM. The amount of each compound solution to be added was 10 µL/well. As a control, a DMSO solution dissolved in a medium was added (DMSO final concentration 0.1%). After culturing in an incubator at 37°C, 5% CO₂ for 4 days, an ATP reagent (100 µL) (CellTiter-Glo (registered trade mark) Luminescent Cell Viability Assay, manufactured by Promega) was added to the culture medium on day 4. After stirring by a plate shaker (manufactured by AS ONE, Micro plate mixer NS-P) at room temperature for 15 min, the luminescence intensity (RLU value) was measured by EnSpire (manufactured by Perkin Elmer) and the luminescence value of the medium alone was subtracted to measure the number of viable cells. The RLU value (ATP measurement, luminescence intensity) of compound no-addition was taken as 100%, and relative value with addition of each compound is shown in the twenty-second table.

### [the twenty-second table]

**[Table 22]**

| culture conditions | low attachment U-bottom plate | | low attachment flat bottom plate (deacylated gellan gum-containing medium) | |
|---|---|---|---|---|
| compound concentration | 5 µM | 10 µM | 5 µM | 10 µM |
| k-1:H-1 | 820% | 2031% | 2210% | 6642% |
| k-1:H-7 | 1652% | 2379% | 5213% | 9015% |
| k-1:I-1 | 327% | 304% | 209% | 268% |
| k-1:B-1 | 2199% | 3187% | 9183% | 14545% |
| k-1:D-1 | 5101% | 5728% | 22879% | 24334% |
| k-1:J-1 | 1355% | 2768% | 6465% | 13360% |

As a result of this test, it was clarified that k-1:H-1, k-1:H-7, k-1:I-1, k-1:B-1, k-1:D-1, and k-1:J-1 promoted proliferation activity in human umbilical vein endothelial cells under three-dimensional conditions. At this time, the human umbilical vein endothelial cells formed spheres using either the low attachment U-bottom plate or the low attachment flat bottom plate.

### [Experimental Example 15] Action of the compound of the present invention on animal cell lines

Various animal cell lines were precultured (single layer culture) as follows in respective media. Chinese hamster ovary-derived cell line CHO-K1 (manufactured by DS PHARMA BIO MEDICAL, 10% fetal bovine serum (FBS, manufactured by Corning)-containing Ham's F-12 medium (manufactured by Fujifilm Wako Pure Chemical Corporation)), Cercopithecus aethiops kidney epithelium-derived cell line Vero (purchased from JCRB cell bank, 5% FBS-containing Medium 199 medium (manufactured by Life Technologies)). The above-mentioned cells in the logarithmic growth phase were washed with PBS, a 0.25w/v% trypsin-1 mmol/L EDTA (ethylenediaminetetraacetic acid) solution (manufactured by Fujifilm Wako Pure Chemical Corporation) was added, and adherent cells were detached by incubating at 37°C for 3 min. Each medium was added and the mixture was centrifuged and resuspended in the same medium.

The aforementioned various animal cells were suspended in respective deacylated gellan gum-containing or not containing media (deacylated gellan gum concentration was 0.02 w/v% in Ham's F-12 medium, 0.015 w/v% in Medium 199 medium), and seeded in a 96 well low attachment U-bottom plate (manufactured by Corning, #4520, deacylated gellan gum-free medium), or a low attachment flat bottom plate (manufactured by Corning, #3474, deacylated gellan gum-containing medium) at a cell concentration of 700 - 2000 cells/90 µL/well (all 3D culture). Continuously, the compound to be used in the present invention dissolved in DMSO was added to each medium at a final concentration of 5 µM or 10 µM. The amount of each compound solution to be added was 10 µL/well. As a control, a DMSO solution dissolved in a medium was added (DMSO final concentration 0.1%). After culturing in an incubator at 37°C, 5% CO₂ for 4 days, an ATP reagent (100 µL) (CellTiter-Glo (registered trade mark) Luminescent Cell Viability Assay, manufactured by Promega) was added to the culture medium on day 4. After stirring by a plate shaker (manufactured by AS ONE, Micro plate mixer NS-P) at room temperature for 15 min, the luminescence intensity (RLU value) was measured by EnSpire (manufactured by Perkin Elmer) and the luminescence value of the medium alone was subtracted to measure the number of viable cells. The RLU value (ATP measurement, luminescence intensity) of compound non-addition (control) was taken as 100%, and relative value with addition of each compound was calculated. When compared with the compound non-addition (control), one showing a value of not more than 119% as -, one showing a value of not less than 120% as ○, and one showing a value of not less than 150% as ⊙ are shown in the twenty-third table and the twenty-fourth table.

### [the twenty-third table]

**[Table 23]**

| low attachment U-bottom plate | | | | |
|---|---|---|---|---|
| cell line | CHO-K1 | | Vero | |
| | 5 µM 10 µM | | 5 µM 10 µM | |
| k-1:H-1 | - | - | ⊙ | ⊙ |
| k-1:H-7 | - | - | ⊙ | ⊙ |
| k-1:I-1 | - | - | ○ | ⊙ |
| k-1:B-1 | - | - | ⊙ | ⊙ |
| k-1:D-1 | - | - | ⊙ | ⊙ |
| k-1:J-1 | - | - | ⊙ | ⊙ |

### [the twenty-fourth table]

**[Table 24]**

| low attachment flat bottom plate (deacylated gellan gum-containing medium) | | | | |
|---|---|---|---|---|
| cell line | CHO-K1 | | Vero | |
| | 5 µM 10 µM | | 5 µM 10 µM | |
| k-1:H-1 | ○ | - | ⊙ | ⊙ |
| k-1:H-7 | ○ | - | ⊙ | ⊙ |
| k-1:I-1 | - | - | - | - |
| k-1:B-1 | ○ | - | ⊙ | ⊙ |
| k-1:D-1 | ⊙ | ⊙ | ⊙ | ⊙ |
| k-1:J-1 | ○ | ⊙ | ○ | ⊙ |

As a result of this test, it was clarified that k-1:H-1, k-1:H-7, k-1:I-1, k-1:B-1, k-1:D-1, and k-1:J-1 promoted proliferation activity in plural animal cell lines under three-dimensional conditions. At this time, the animal cell lines formed spheres using either the low attachment U-bottom plate or the low attachment flat bottom plate.

### [Industrial Applicability]

The present invention can achieve any or any combination of promoting cell proliferation, promoting sphere formation, promoting organoid formation, and promoting Cyst formation when added to a cell medium. The cells and the like prepared by the present invention are highly useful in, for example, the field of drug discovery.

## Claims

1. A medium additive composition comprising a compound represented by the following formula (I), or a salt thereof: 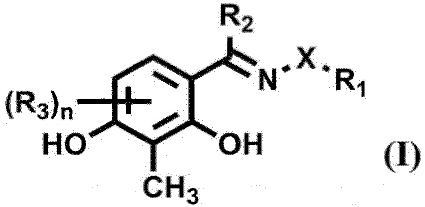 {wherein, X is a single bond, -CH₂COO-, -CONH-, or -NHCO-, R₁ is an alkyl group having 1 - 10 carbon atoms, an aryl group, or -Y-W-Z-Ar wherein Y and Z are each a single bond or an alkylene group having 1 - 6 carbon atoms, W is an oxygen atom, a sulfur atom or N(R₄), R₄ is a hydrogen atom or an alkyl group having 1 - 6 carbon atoms, Ar is an aryl group optionally having substituent(s), R₂ is an alkyl group having 1 - 6 carbon atoms, R₃ is a hydroxyl group, and n is 0, 1 or 2}, wherein when Ar has substituent(s) the substituent(s) are selected from halogeno groups and hydroxyl groups.

2. The composition according to claim 1, wherein X is -NHCO-.

3. The composition according to claim 1 or 2, wherein n is 0.

4. The composition according to claim 2, wherein R₁ is -Y-W-Z-Ar, Y is an alkylene group having 1 - 6 carbon atoms, W is N(R₄), Z is a single bond, and n=0, preferably wherein the aryl group of Ar is a phenyl group.

5. The composition according to any one of claims 1 to 3, wherein R₁ is -Y-W-Z-Ar, Y is a single bond, W is N(R₄) and R₄ is a hydrogen atom, preferably wherein the aryl group of Ar is a phenyl group, and/or Z is a methylene group.

6. The composition according to any one of claims 1 to 3, wherein R₁ is -Y-W-Z-Ar, Y is a single bond, W is an oxygen atom, and Z is a methylene group, preferably wherein the aryl group of Ar is a phenyl group.

7. The composition according to any one of claims 1 to 4, wherein the compound is a compound selected from the group consisting of the following, or a salt thereof: 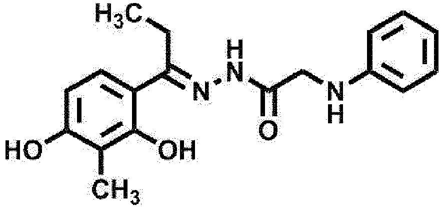 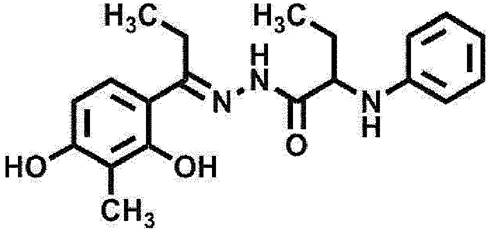 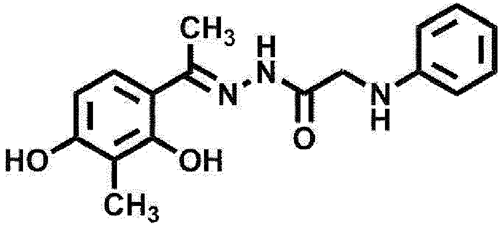 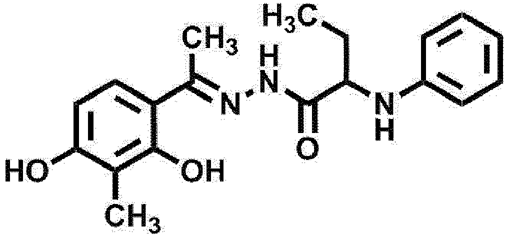 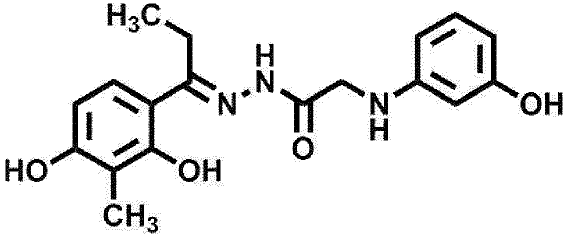 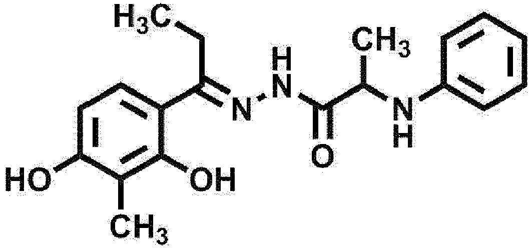 and,

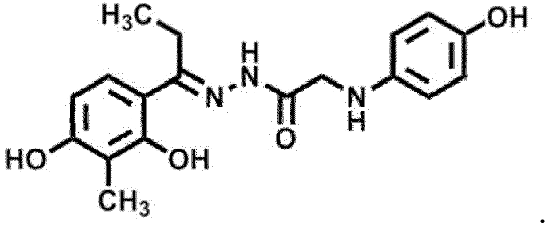

8. The composition according to any one of claims 1 to 3 and 5, wherein the compound is a compound selected from the group consisting of the following, or a salt thereof: 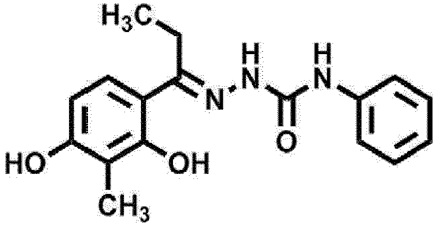 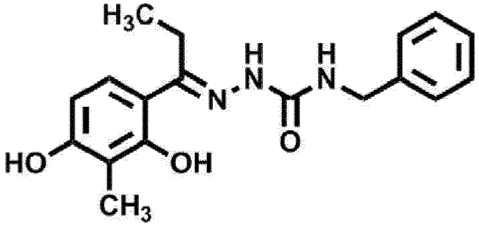 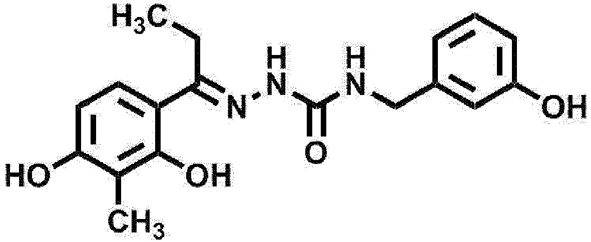 and,

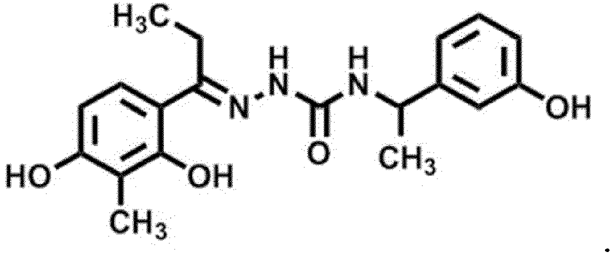

9. The composition according to any one of claims 1 to 3 and 6, wherein the compound is a compound represented by: 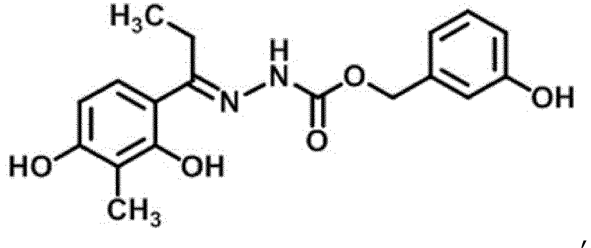 or a salt thereof.

10. A medium additive composition comprising a compound selected from the following group of compounds, or a salt thereof: 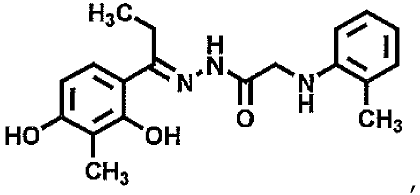 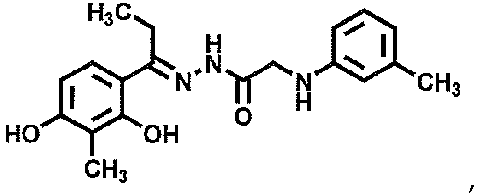 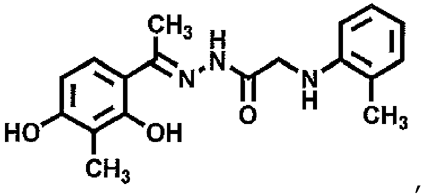 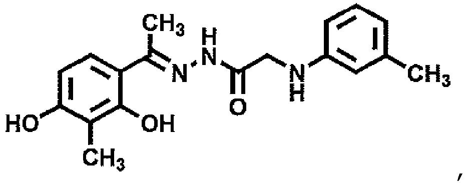 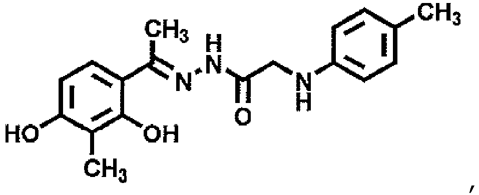 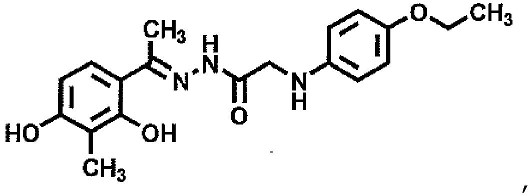 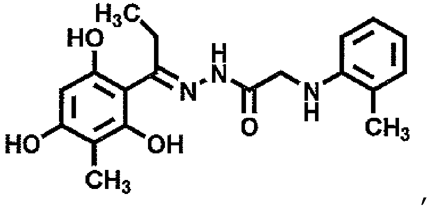 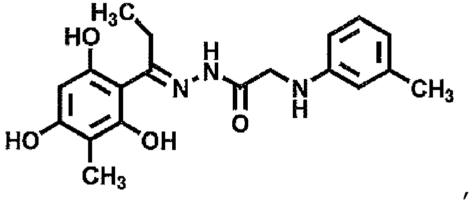 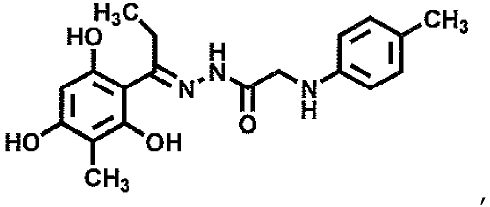 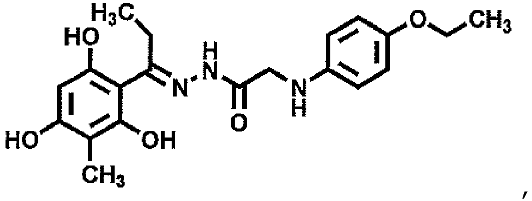 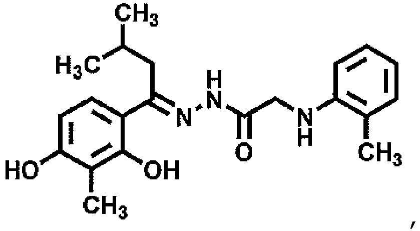 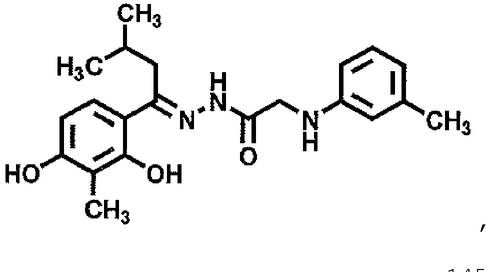 and,

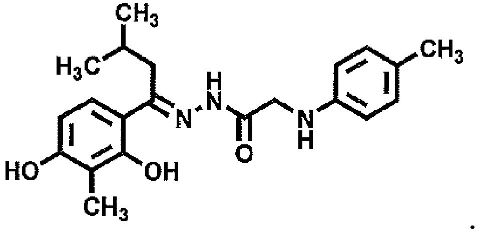

11. The composition according to any one of claims 1 to 10, wherein the composition is for promoting cell proliferation, preferably wherein the cell is selected from the group consisting of a normal cell line, a cancer cell line and a stem cell.

12. The composition according to any one of claims 1 to 10, wherein the composition is for promoting sphere formation, organoid formation, or Cyst formation.

13. A medium comprising the medium additive composition according to any one of claims 1 to 10.

14. A method for promoting cell proliferation comprising adding the medium additive composition according to any one of claims 1 to 10 to a medium, preferably wherein the cell is selected from the group consisting of a normal cell line, a cancer cell line and a stem cell.

15. A method for promoting sphere formation, organoid formation or Cyst formation, comprising adding the medium additive composition according to any one of claims 1 to 10 to a medium.

16. A compound represented by the following formula (I), or a salt thereof: 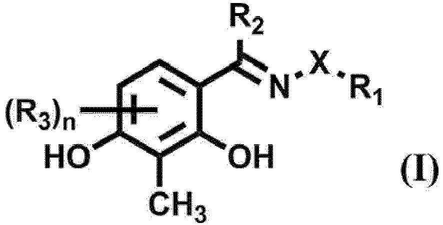 {wherein, X, R₁, R₂, R₃, and n are as defined in any one of claims 1 to 10 (provided that when X is -NHCO-, R₂ is an ethyl group, and n is 0, then R₁ is not -CH₂-NH-C₆H₅)}.

17. A compound selected from the following group of compounds, or a salt thereof: 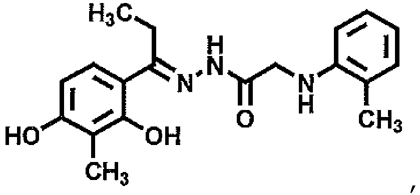 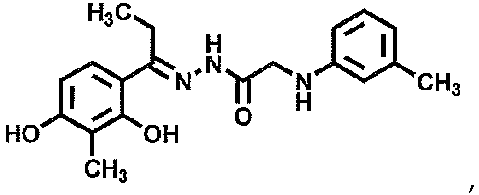 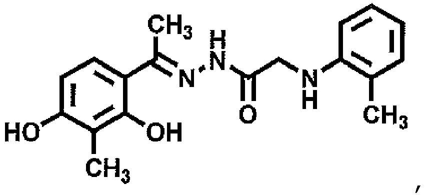 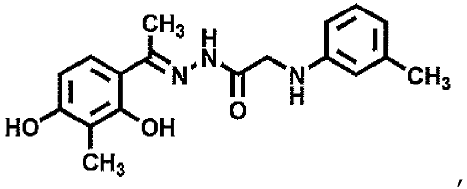 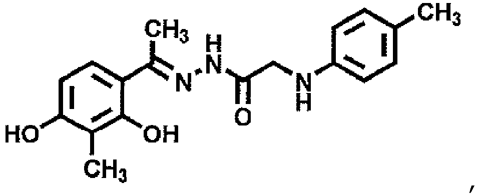 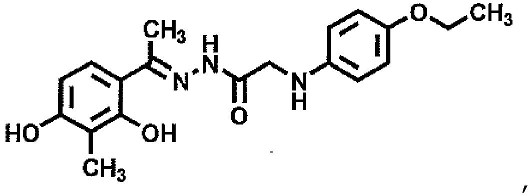 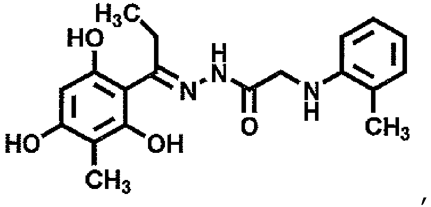 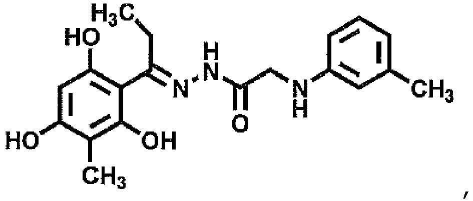 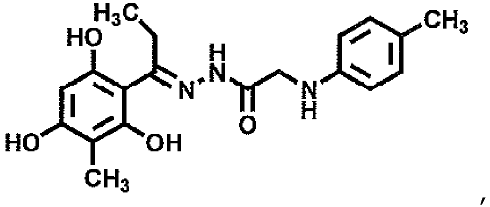 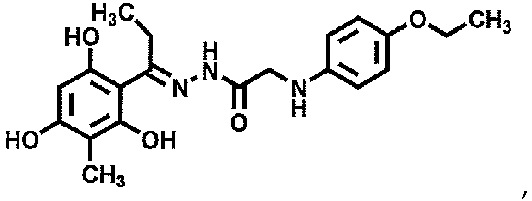 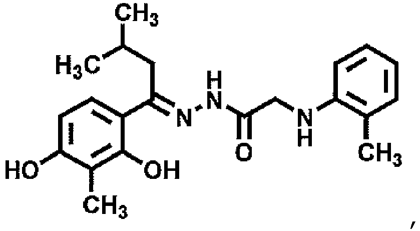 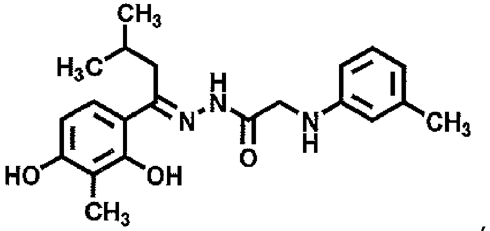 and,

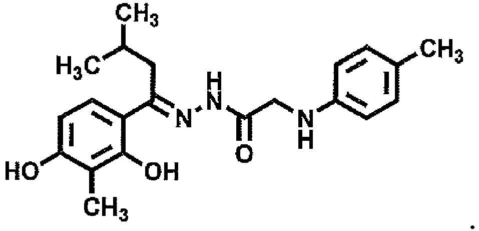

## Patentansprüche

1. Mediumadditivzusammensetzung, die eine Verbindung, die durch die folgende Formel (I) dargestellt wird, oder ein Salz davon umfasst: 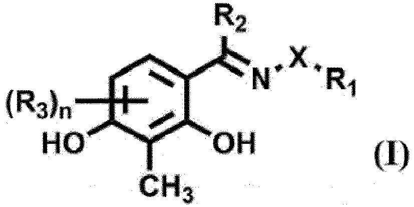 {wobei X eine Einfachbindung, -CH₂COO-, -CONH- oder -NHCO- ist, R₁ eine Alkylgruppe mit 1-10 Kohlenstoffatomen, eine Arylgruppe oder -Y-W-Z-Ar ist, wobei Y und Z jeweils eine Einfachbindung oder eine Alkylengruppe mit 1-6 Kohlenstoffatomen sind, W ein Sauerstoffatom, ein Schwefelatom oder N(R₄) ist, R₄ ein Wasserstoffatom oder eine Alkylgruppe mit 1-6 Kohlenstoffatomen ist, Ar eine Arylgruppe ist, die gegebenenfalls einen oder mehrere Substituenten aufweist, R₂ eine Alkylgruppe mit 1-6 Kohlenstoffatomen ist, R₃ eine Hydroxygruppe ist und n = 0, 1 oder 2 ist}, wobei, wenn Ar einen oder mehrere Substituenten aufweist, der eine oder die mehreren Substituenten aus Halogengruppen und Hydroxygruppen ausgewählt sind.

2. Zusammensetzung gemäß Anspruch 1, wobei X = -NHCO- ist.

3. Zusammensetzung gemäß Anspruch 1 oder 2, wobei n = 0 ist.

4. Zusammensetzung gemäß Anspruch 2, wobei R₁ = -Y-W-Z-Ar ist, Y eine Alkylengruppe mit 1-6 Kohlenstoffatomen ist, W = N(R₄) ist, Z eine Einfachbindung ist und n = 0 ist, wobei vorzugsweise die Arylgruppe von Ar eine Phenylgruppe ist.

5. Zusammensetzung gemäß einem der Ansprüche 1 bis 3, wobei R₁ = -Y-W-Z-Ar ist, Y eine Einfachbindung ist, W = N(R₄) ist und R₄ ein Wasserstoffatom ist, wobei vorzugsweise die Arylgruppe von Ar eine Phenylgruppe ist und/oder Z eine Methylengruppe ist.

6. Zusammensetzung gemäß einem der Ansprüche 1 bis 3, wobei R₁ = -Y-W-Z-Ar ist, Y eine Einfachbindung ist, W ein Sauerstoffatom ist und Z eine Methylengruppe ist, wobei vorzugsweise die Arylgruppe von Ar eine Phenylgruppe ist.

7. Zusammensetzung gemäß einem der Ansprüche 1 bis 4, wobei die Verbindung eine Verbindung, die aus der Gruppe, die aus den Folgenden besteht, ausgewählt ist, oder ein Salz davon ist: 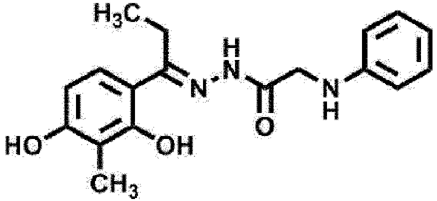 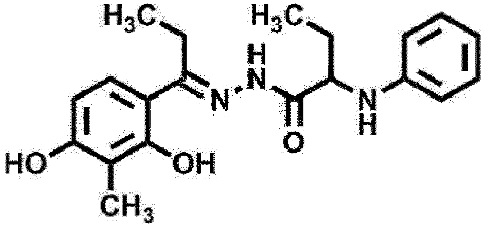 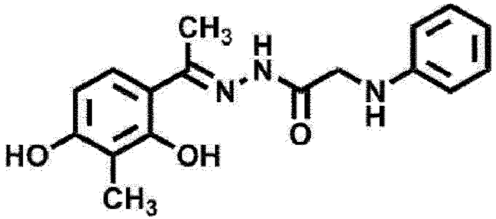 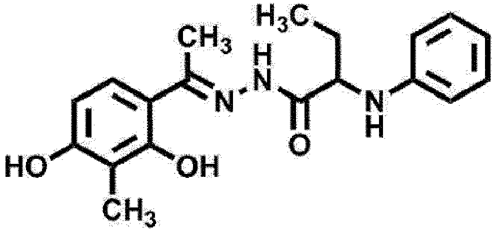 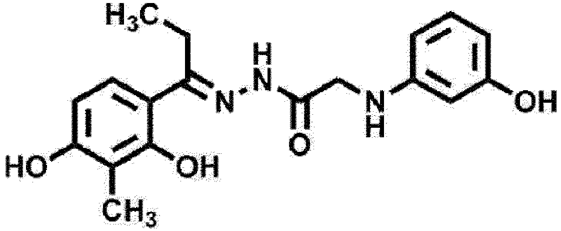 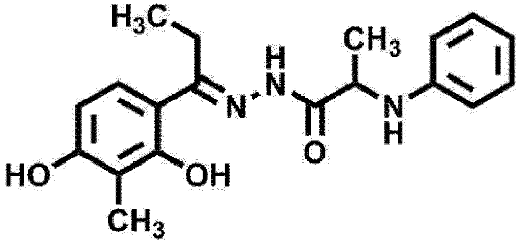 und

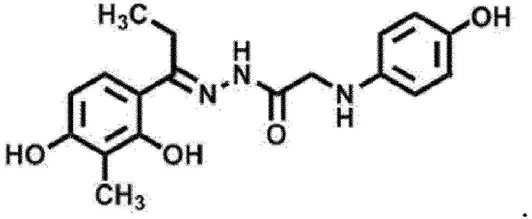

8. Zusammensetzung gemäß einem der Ansprüche 1 bis 3 und 5, wobei die Verbindung eine Verbindung, die aus der Gruppe, die aus den Folgenden besteht, ausgewählt ist, oder ein Salz davon ist: 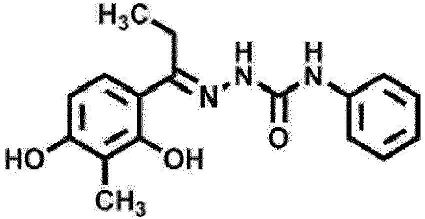 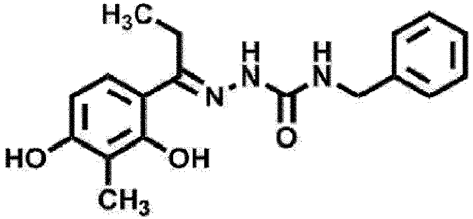 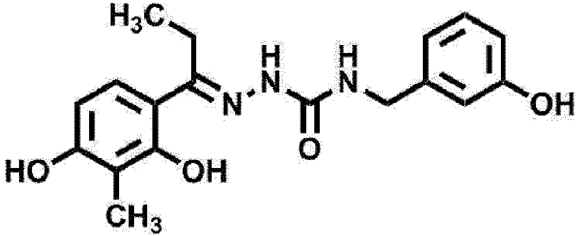 und

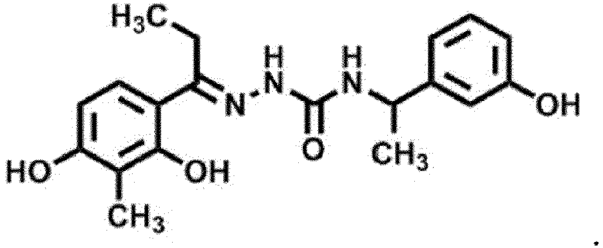

9. Zusammensetzung gemäß einem der Ansprüche 1 bis 3 und 6, wobei die Verbindung eine Verbindung, die durch 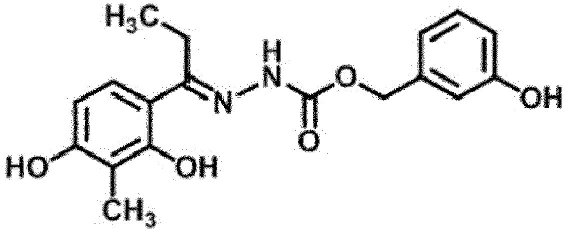 dargestellt wird, oder ein Salz davon ist.

10. Mediumadditivzusammensetzung, die eine Verbindung, welche aus der folgenden Gruppe von Verbindungen ausgewählt ist, oder ein Salz davon umfasst: 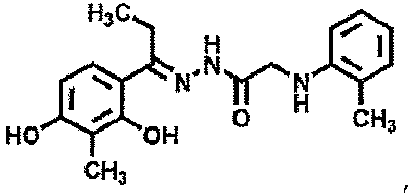 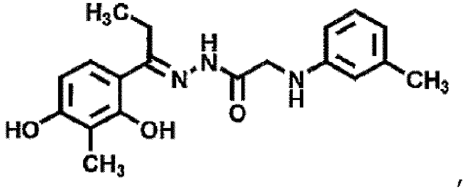 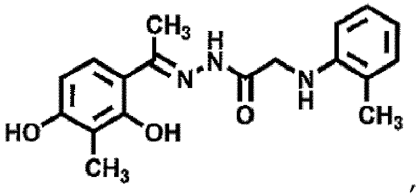 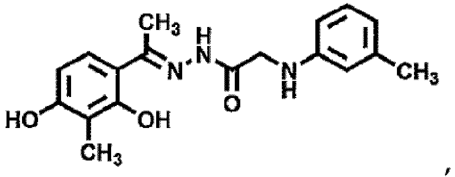 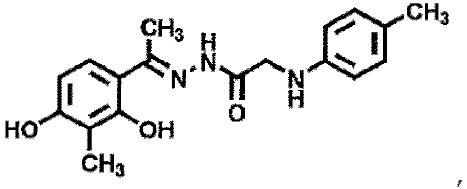 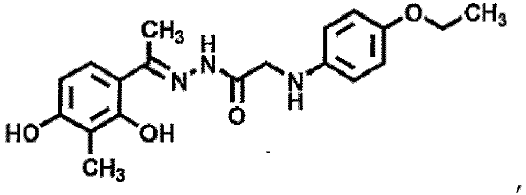 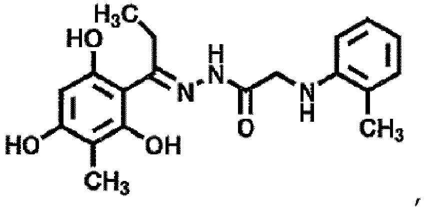 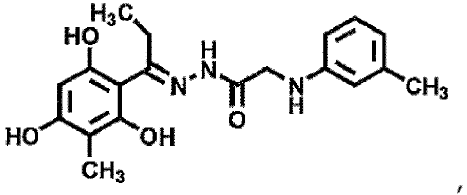 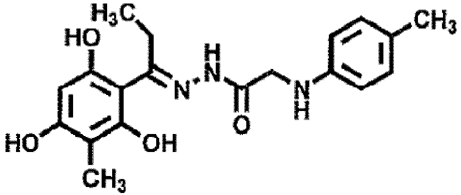 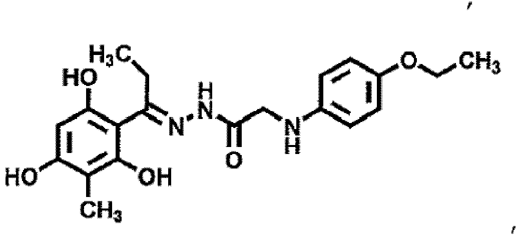 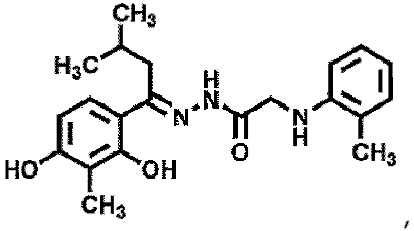 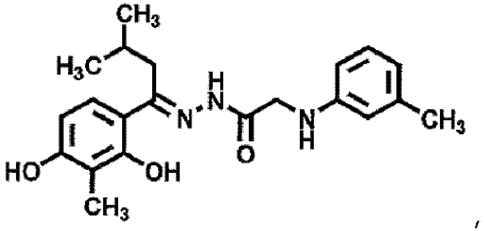 und

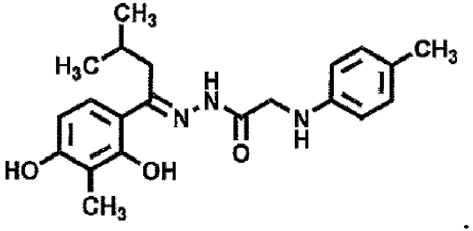

11. Zusammensetzung gemäß einem der Ansprüche 1 bis 10, wobei die Zusammensetzung zur Förderung der Zellvermehrung dient, wobei vorzugsweise die Zelle aus der Gruppe ausgewählt ist, die aus einer normalen Zelllinie, einer Krebszelllinie und einer Stammzelle besteht.

12. Zusammensetzung gemäß einem der Ansprüche 1 bis 10, wobei die Zusammensetzung zur Förderung der Sphärenbildung, der Organoidbildung oder der Zystenbildung dient.

13. Medium, das die Mediumadditivzusammensetzung gemäß einem der Ansprüche 1 bis 10 umfasst.

14. Verfahren zur Förderung der Zellvermehrung, umfassend das Hinzufügen der Mediumadditivzusammensetzung gemäß einem der Ansprüche 1 bis 10 zu einem Medium, wobei vorzugsweise die Zelle aus der Gruppe ausgewählt ist, die aus einer normalen Zelllinie, einer Krebszelllinie und einer Stammzelle besteht.

15. Verfahren zur Förderung der Sphärenbildung, der Organoidbildung oder der Zystenbildung, umfassend das Hinzufügen der Mediumadditivzusammensetzung gemäß einem der Ansprüche 1 bis 10 zu einem Medium.

16. Verbindung, die durch die folgende Formel (I) dargestellt wird, oder ein Salz davon: 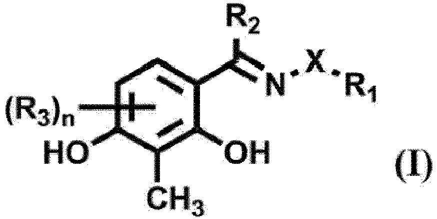 {wobei X, R₁, R₂, R₃ und n wie in einem der Ansprüche 1 bis 10 definiert sind (mit der Maßgabe, dass R₁ nicht -CH₂-NH-C₆H₅ ist, wenn X = -NHCOist, R₂ eine Ethylgruppe ist und n = 0 ist)}.

17. Verbindung, die aus der folgenden Gruppe von Verbindungen ausgewählt ist, oder Salz davon: 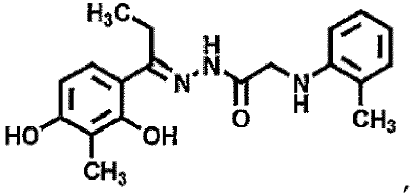 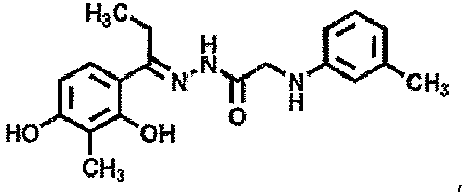 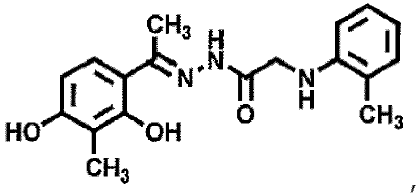 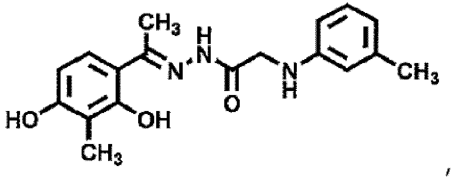 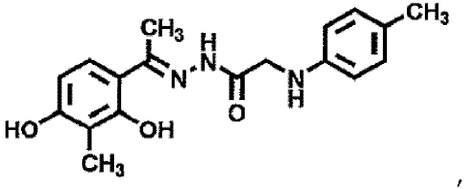 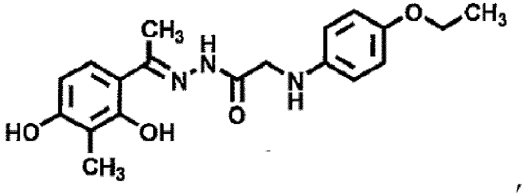 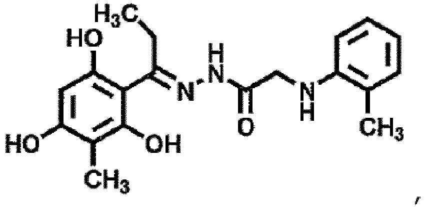 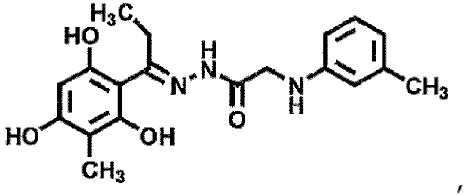 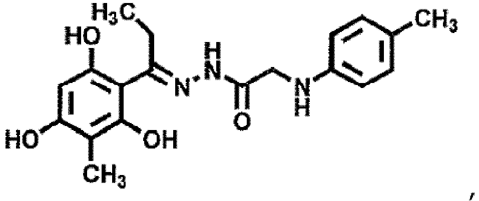 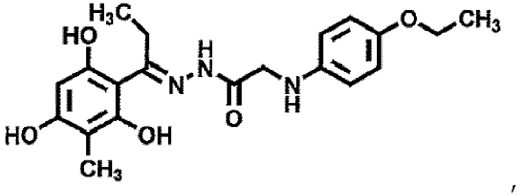 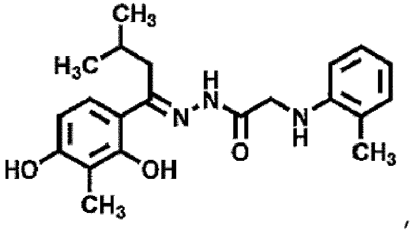 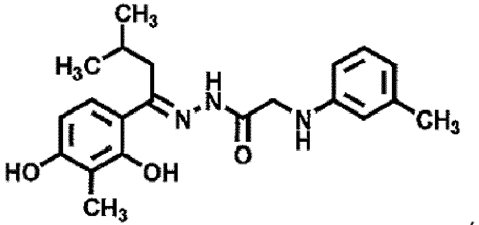 und

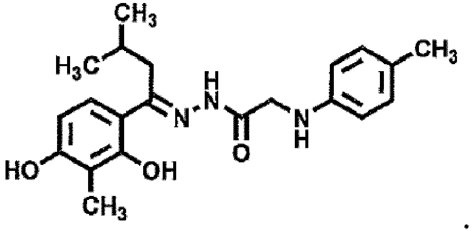

## Revendications

1. Composition d'additif de milieu comprenant un composé représenté par la formule (I) suivante, ou un sel de celui-ci : 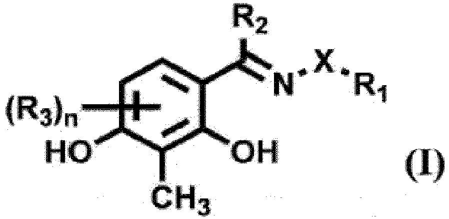 {dans laquelle, X est une liaison simple, -CH₂COO-, -CONH- ou -NHCO-, R₁ est un groupe alkyle ayant 1 à 10 atomes de carbone, un groupe aryle, ou -Y-W-Z-Ar dans lequel Y et Z sont chacun une liaison simple ou un groupe alkylène ayant 1 à 6 atomes de carbone, W est un atome d'oxygène, un atome de soufre ou N(R₄), R₄ est un atome d'hydrogène ou un groupe alkyle ayant 1 à 6 atomes de carbone, Ar est un groupe aryle ayant facultativement un ou plusieurs substituant(s), R₂ est un groupe alkyle ayant 1 à 6 atomes de carbone, R₃ est un groupe hydroxyle, et n est 0, 1 ou 2}, dans lequel lorsque Ar a un ou plusieurs substituant(s), le ou les substituant(s) sont sélectionnés parmi les groupes halogéno et les groupes hydroxyle.

2. Composition selon la revendication 1, dans laquelle X est -NHCO-.

3. Composition selon la revendication 1 ou 2, dans laquelle n est 0.

4. Composition selon la revendication 2, dans laquelle R₁ est -Y-W-Z-Ar, Y est un groupe alkylène ayant 1 à 6 atomes de carbone, W est N(R₄), Z est une liaison simple, et n = 0, de préférence dans laquelle le groupe aryle de Ar est un groupe phényle.

5. Composition selon l'une quelconque des revendications 1 à 3, dans laquelle R₁ est -Y-W-Z-Ar, Y est une liaison simple, W est N(R₄) et R₄ est un atome d'hydrogène, de préférence dans laquelle le groupe aryle de Ar est un groupe phényle, et/ou Z est un groupe méthylène.

6. Composition selon l'une quelconque des revendications 1 à 3, dans laquelle R₁ est -Y-W-Z-Ar, Y est une liaison simple, W est un atome d'oxygène, et Z est un groupe méthylène, de préférence dans laquelle le groupe aryle de Ar est un groupe phényle.

7. Composition selon l'une quelconque des revendications 1 à 4, dans laquelle le composé est un composé sélectionné dans le groupe consistant en ce qui suit, ou un sel de ceux-ci : 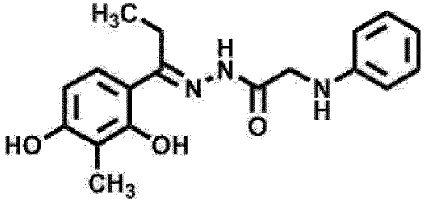 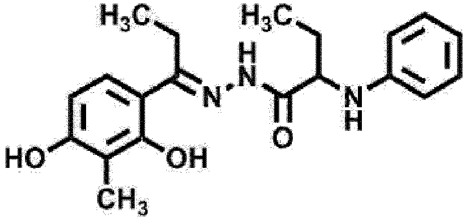 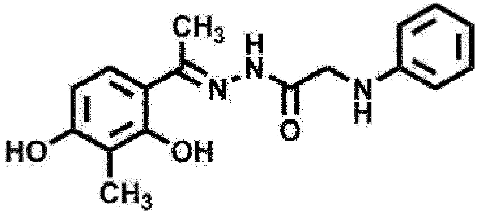 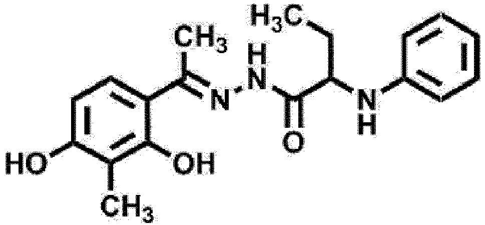 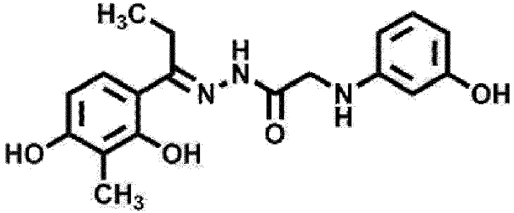 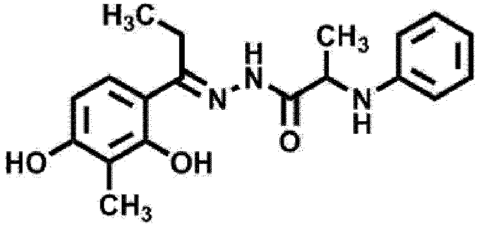 et,

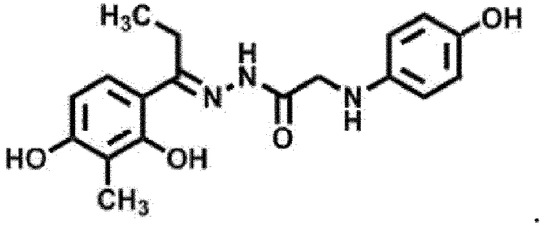

8. Composition selon l'une quelconque des revendications 1 à 3 et 5, dans laquelle le composé est un composé sélectionné dans le groupe consistant en ce qui suit, ou un sel de ceux-ci : 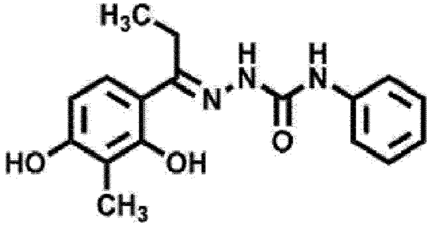 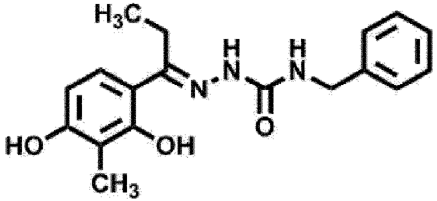 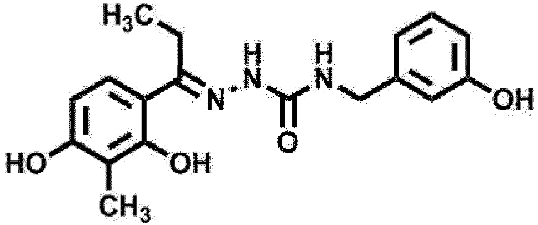 et,

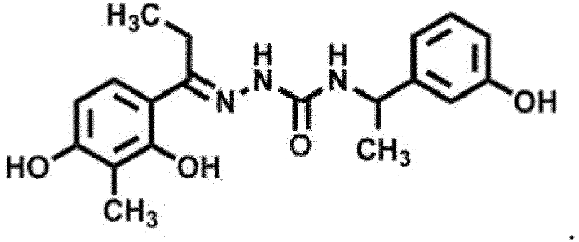

9. Composition selon l'une quelconque des revendications 1 à 3 et 6, dans laquelle le composé est un composé représenté par : 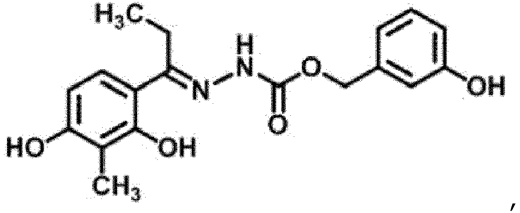 ou un sel de celui-ci.

10. Composition d'additif de milieu comprenant un composé sélectionné dans le groupe suivant de composés, ou un sel de ceux-ci : 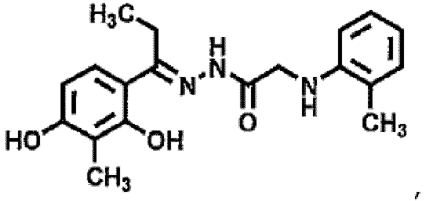 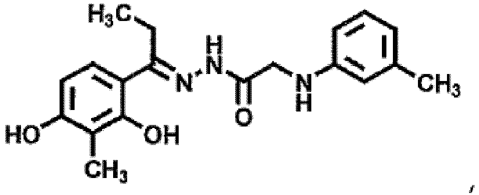 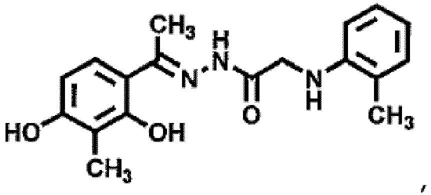 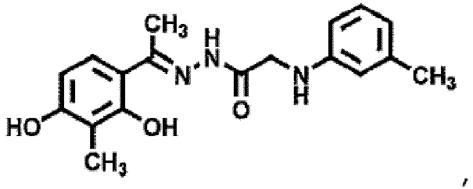 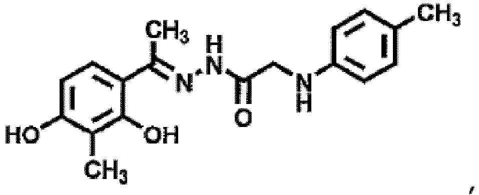 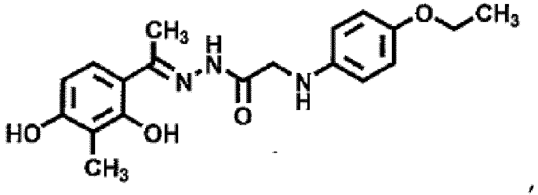 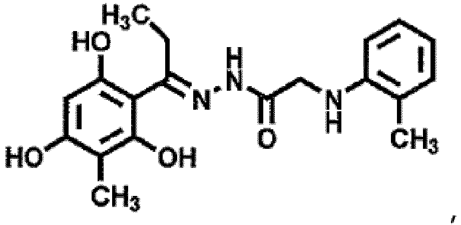 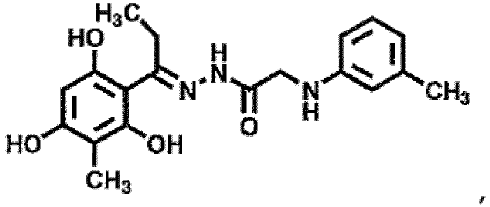 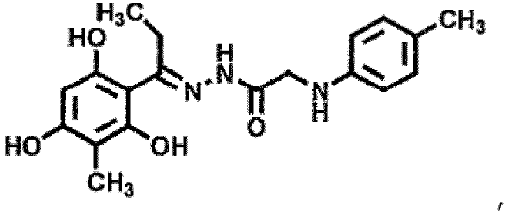 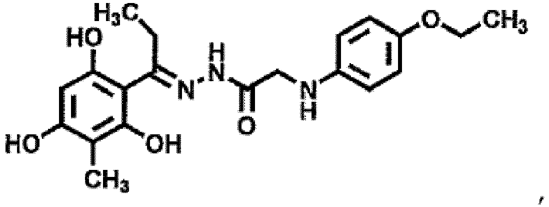 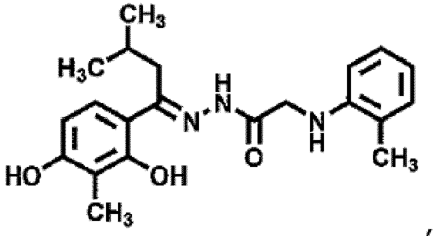 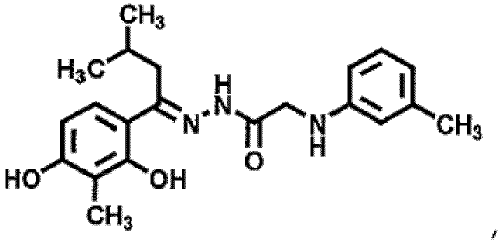 et,

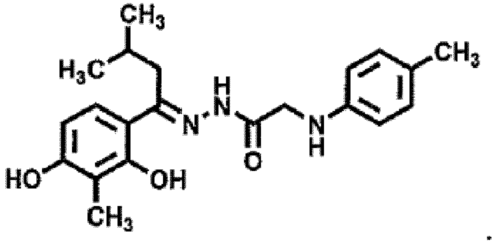

11. Composition selon l'une quelconque des revendications 1 à 10, dans laquelle la composition est destinée à favoriser la prolifération cellulaire, de préférence dans laquelle la cellule est sélectionnée dans le groupe consistant en une lignée de cellule normale, une lignée de cellule cancéreuse et une cellule souche.

12. Composition selon l'une quelconque des revendications 1 à 10, dans laquelle la composition est destinée à favoriser la formation de sphères, la formation d'organoïdes, ou la formation de kystes.

13. Milieu comprenant la composition d'additif de milieu selon l'une quelconque des revendications 1 à 10.

14. Méthode pour favoriser la prolifération cellulaire comprenant l'ajout de la composition d'additif de milieu selon l'une quelconque des revendications 1 à 10 à un milieu, de préférence dans laquelle la cellule est sélectionnée dans le groupe consistant en une lignée de cellule normale, une lignée de cellule cancéreuse et une cellule souche.

15. Méthode pour favoriser la formation de sphères, la formation d'organoïdes ou la formation de kystes, comprenant l'ajout de la composition d'additif de milieu selon l'une quelconque des revendications 1 à 10 à un milieu.

16. Composé représenté par la formule (I) suivante, ou un sel de celui-ci : 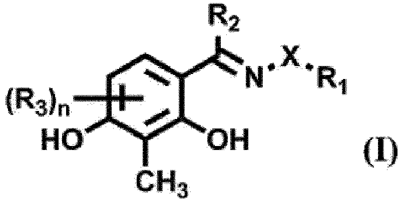 {dans laquelle X, R₁, R₂, R₃, et n sont tels que définis dans l'une quelconque des revendications 1 à 10 (à condition que lorsque X est -NHCO-, R₂ est un groupe éthyle, et n est 0, alors R₁ n'est pas -CH₂-NH-C₆H₅)}.

17. Composé sélectionné dans le groupe suivant de composés, ou un sel de ceux-ci : 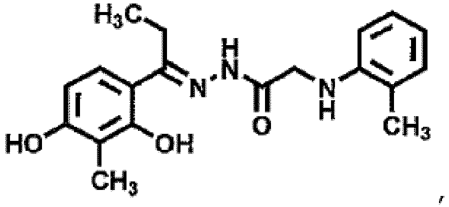 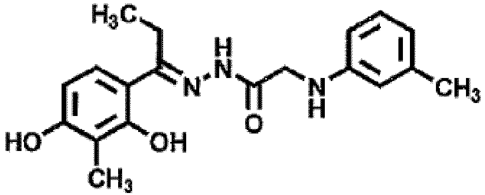 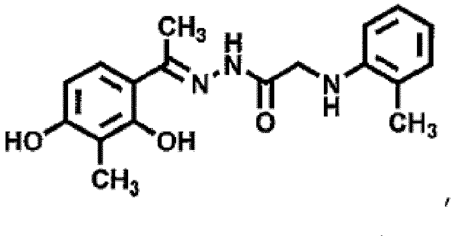 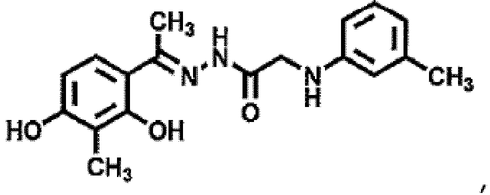 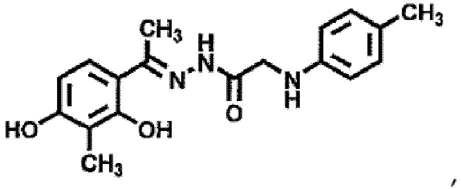 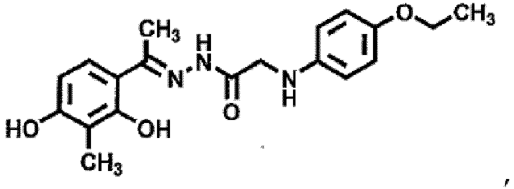 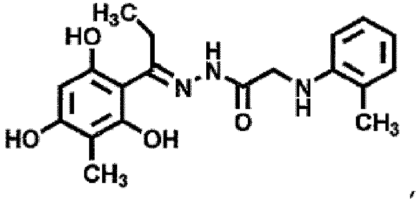 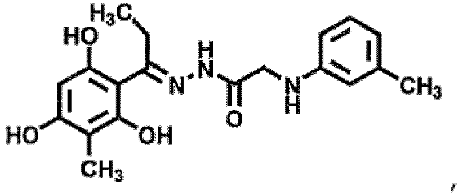 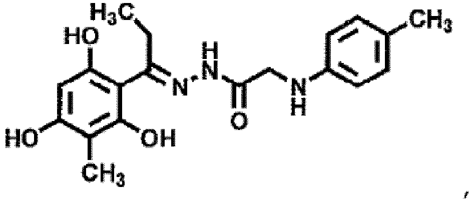 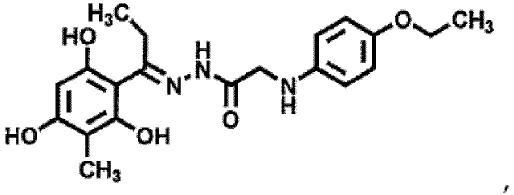 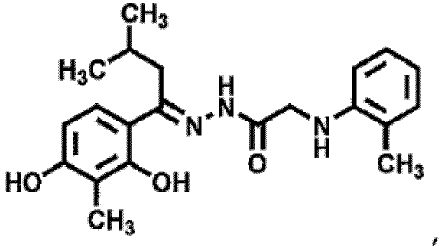 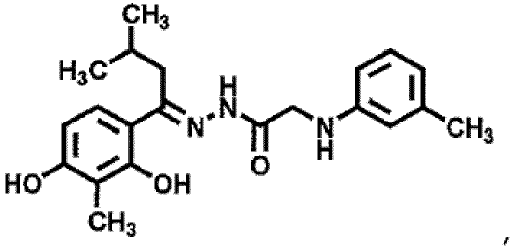 et,
